# EUROPEAN PATENT SPECIFICATION

(11) **EP 1 268 783 B1**
(45) Date of publication and mention of the grant of the patent: **28.06.2006**
(21) Application number: 01918982.8
(22) Date of filing: 28.03.2001
(51) Int. Cl.: C12N 15/12, C12N 15/62, C12N 15/86, C07K 14/78, C07K 16/18, A61K 38/39, A01K 67/027

(54) **Antiangiogene Eigenschaften von Matin und Fragmenten oder Varianten davon**
Anti-angiogenic properties of matin and of fragments or variants thereof
Propriétés anti-angiogéniques de la proteine matin et fragments et variants de celui-ci

(30) Priority: 29.03.2000 US 192875 P
(43) Date of publication of application: 02.01.2003
(73) Proprietor: Beth Israel Deaconess Medical Center, Inc., Boston, MA 02215 (US)
(72) Inventor: KALLURI, Raghuram, Boston, MA 02116 (US)
(74) Representative: White, Martin Paul
(86) International application number: PCT/US2001/009921
(87) International publication number: WO 2001/073033

(56) References cited:
- WO-A-00/56754
- WO-A-95/22979
- WO-A-98/31709
- US-A- 5 266 328
- SASAKI M ET AL: "LAMININ A MULTIDOMAIN PROTEIN THE A CHAIN HAS A UNIQUE GLOBULAR DOMAIN AND HOMOLOGY WITH THE BASEMENT MEMBRANE PROTEOGLYCAN AND THE LAMININ B CHAINS" JOURNAL OF BIOLOGICAL CHEMISTRY, vol. 263, no. 32, 1988, pages 16536-16544, XP002183074 ISSN: 0021-9258
- CAO, Y.: "Endogenous engiogenesis inhibitors: angiostatin, endostatin, and other proreolytic fragments." PROGRESS IN MOLECULAR AND SUBCELLULAR BIOLOGY, vol. 20, 1998, pages 161-176, XP001041400
- SKUBITZ A P N ET AL: "Synthetic peptides from the carboxy-terminal globular domain of the A chain of laminin: their ability to promote cell adhesion and neurite outgrowth, and interact with heparin and the beta1 integrin subunit" JOURNAL OF CELL BIOLOGY, ROCKEFELLER UNIVERSITY PRESS, NEW YORK, US, US, vol. 115, no. 4, 1 November 1991 (1991-11-01), pages 1137-1148, XP002092964 ISSN: 0021-9525
- DATABASE BIOSIS [Online] BIOSCIENCES INFORMATION SERVICE, PHILADELPHIA, PA, US; 1997 MIZUSHIMA HIROTO ET AL: "Identification of integrin-dependent and -independent cell adhesion domains in COOH-terminal globular region of laminin-5 alpha-3 chain." Database accession no. PREV199799749685 XP002183076 & CELL GROWTH & DIFFERENTIATION, vol. 8, no. 9, 1997, pages 979-987, ISSN: 1044-9523
- NICOSIA R F ET AL: "MODULATION OF ANGIOGENESIS IN VITRO BY LAMININ-ENTACTIN COMPLEX" DEVELOPMENTAL BIOLOGY, ACADEMIC PRESS, NEW YORK, NY, US, vol. 164, no. 1, July 1994 (1994-07), pages 197-206, XP000971040 ISSN: 0012-1606

### SEQUENCE LISTING

<110> Beth Israel Deaconess Medical Center Kalluri, Raghuram
<120> Anti-Angiogenic and Anti-Tumor Properties of Matin and Other Laminin Domains
<130> 1440.2004002
<140> PCT/US01/09921
   <141> 2001-03-28
<150> US 60/192,875
   <151> 2000-03-29
<160> 12
<170> FastSEQ for Windows Version 4.0
<210> 1
   <211> 9503
   <212> DNA
   <213> Mus musculus
<220>
   <221> CDS
   <222> (49)...(9300)
<400> 1
   agcacgggcg agaccttccc caggagcgca gggagcggcg gcgacaac atg cgc ggc   57
                                                        Met Arg Gly
                                                         1
   agc ggc acg gga gcc gcg ctc ctg gtg ctc ctg gcc tcg gtg ctc tgg    105
   Ser Gly Thr Gly Ala Ala Leu Leu Val Leu Leu Ala Ser Val Leu Trp
        5                  10                  15
   gtc acc gtg cgg agc cag cag aga ggc ttg ttc cct gcc att ctc aac    153
   Val Thr Val Arg Ser Gln Gln Arg Gly Leu Phe Pro Ala Ile Leu Asn
    20                  25                  30                  35
   ctg gcc acc aat gcc cac atc agc gcc aat gct acc tgt gga gag aag    201
   Leu Ala Thr Asn Ala His Ile Ser Ala Asn Ala Thr Cys Gly Glu Lys
                    40                  45                  50
   ggg cct gag atg ttc tgc aaa ctc gtg gag cac gtg ccg ggc cgg cct    249
   Gly Pro Glu Met Phe Cys Lys Leu Val Glu His Val Pro Gly Arg Pro
                55                  60                  65
   gtt cga cac gcc caa tgc cgg gtc tgt gac ggt aac agt acg aat cct    297
   Val Arg His Ala Gln Cys Arg Val Cys Asp Gly Asn Ser Thr Asn Pro
            70                  75                  80
   aga gag cgc cat ccg ata tca cac gca atc gat ggc acc aac aac tgg    345
   Arg Glu Arg His Pro Ile Ser His Ala Ile Asp Gly Thr Asn Asn Trp
        85                  90                  95
   tgg cag agc ccc agt att cag aat ggg aga gag tat cac tgg gtc act     393
   Trp Gln Ser Pro Ser Ile Gln Asn Gly Arg Glu Tyr His Trp Val Thr
   100                 105                 110                 115
   gtc acc ctg gac tta cgg cag gtc ttt caa gtt gca tac atc atc att     441
   Val Thr Leu Asp Leu Arg Gln Val Phe Gln Val Ala Tyr Ile Ile Ile
                   120                 125                 130
   aaa gct gcc aat gcc cct cgg cct gga aac tgg att ttg gag cgc tcc     489
   Lys Ala Ala Asn Ala Pro Arg Pro Gly Asn Trp Ile Leu Glu Arg Ser
               135                 140                 145
   gtg gat ggc gtc aag ttc aaa ccc tgg cag tac tat gcc gtc agc gat     537
   Val Asp Gly Val Lys Phe Lys Pro Trp Gln Tyr Tyr Ala Val Ser Asp
           150                 155                 160
   aca gag tgt ttg acc cgc tac aaa ata act cca cgg cgg gga cct ccc     585
   Thr Glu Cys Leu Thr Arg Tyr Lys Ile Thr Pro Arg Arg Gly Pro Pro
       165                 170                 175
   act tac aga gca gac aac gaa gtc atc tgc acc tcg tat tat tca aag     633
   Thr Tyr Arg Ala Asp Asn Glu Val Ile Cys Thr Ser Tyr Tyr Ser Lys
   180                 185                 190                 195
   ctg gtg cca ctt gaa cat gga gag att cac aca tca ctc atc aat ggc     681
   Leu Val Pro Leu Glu His Gly Glu Ile His Thr Ser Leu Ile Asn Gly
                   200                 205                 210
   aga ccc agc gct gac gac ccc tca ccc cag ttg ctg gaa ttc acc tca     729
   Arg Pro Ser Ala Asp Asp Pro Ser Pro Gln Leu Leu Glu Phe Thr Ser
               215                 220                 225
   gca cgg tac att cgc ctt cgt ctt cag cgc atc aga aca ctc aac gca     777
   Ala Arg Tyr Ile Arg Leu Arg Leu Gln Arg Ile Arg Thr Leu Asn Ala
           230                 235                 240
   gac ctc atg acc ctt agc cat cgg gac ctc aga gac ctt gac ccc att     825
   Asp Leu Met Thr Leu Ser His Arg Asp Leu Arg Asp Leu Asp Pro Ile
       245                 250                 255
   gtc aca aga cgt tat tac tat tcg ata aaa gac att tcc gtt gga ggc     873
   Val Thr Arg Arg Tyr Tyr Tyr Ser Ile Lys Asp Ile Ser Val Gly Gly
   260                 265                 270                 275
   atg tgc att tgc tac ggc cat gcc agc agc tgc ccg tgg gat gaa gaa     921
   Met Cys Ile Cys Tyr Gly His Ala Ser Ser Cys Pro Trp Asp Glu Glu
                   280                 285                 290
   gca aag caa cta cag tgt cag tgt gaa cac aat acg tgt ggc gag agc     969
   Ala Lys Gln Leu Gln Cys Gln Cys Glu His Asn Thr Cys Gly Glu Ser
               295                 300                 305
   tgc gac agg tgc tgt cct ggc tac cat cag cag ccc tgg agg ccc gga    1017
   Cys Asp Arg Cys Cys Pro Gly Tyr His Gln Gln Pro Trp Arg Pro Gly
           310                 315                 320
   acc att tcc tcc ggc aac gag tgt gag gaa tgc aac tgt cac aac aaa    1065
   Thr Ile Ser Ser Gly Asn Glu Cys Glu Glu Cys Asn Cys His Asn Lys
       325                 330                 335
   gcc aaa gat tgt tac tat gac agc agt gtt gca aag gag agg aga agc    1113
   Ala Lys Asp Cys Tyr Tyr Asp Ser Ser Val Ala Lys Glu Arg Arg Ser
   340                 345                 350                 355
   ctg aac act gcc ggg cag tac agt gga gga ggg gtt tgt gtc aac tgc    1161
   Leu Asn Thr Ala Gly Gln Tyr Ser Gly Gly Gly Val Cys Val Asn Cys
                   360                 365                 370
   tcg cag aat acc aca ggg atc aac tgt gaa acc tgt atc gac cag tat    1209
   Ser Gln Asn Thr Thr Gly Ile Asn Cys Glu Thr Cys Ile Asp Gln Tyr
               375                 380                 385
   tac aga cct cac aag gta tct cct tat gat gac cac cct tgc cgt ccc    1257
   Tyr Arg Pro His Lys Val Ser Pro Tyr Asp Asp His Pro Cys Arg Pro
           390                 395                 400
   tgt aac tgt gac cct gtg ggg tct ctg agt tct gtc tgt atc aag gat    1305
   Cys Asn Cys Asp Pro Val Gly Ser Leu Ser Ser Val Cys Ile Lys Asp
       405                 410                 415
   gac cgc cat gcc gat tta gcc aat gga aag tgg cca ggt cag tgt cca    1353
   Asp Arg His Ala Asp Leu Ala Asn Gly Lys Trp Pro Gly Gln Cys Pro
   420                 425                 430                 435
   tgt agg aaa ggt tat gct gga gat aaa tgt gac cgc tgc cag ttt ggc    1401
   Cys Arg Lys Gly Tyr Ala Gly Asp Lys Cys Asp Arg Cys Gln Phe Gly
                   440                 445                 450
   tac cgg ggt ttc cca aat tgc atc ccc tgt gac tgc agg act gtc ggc    1449
   Tyr Arg Gly Phe Pro Asn Cys Ile Pro Cys Asp Cys Arg Thr Val Gly
               455                 460                 465
   agc ctg aat gag gat cca tgc ata gag ccg tgt ctt tgt aag aaa aat    1497
   Ser Leu Asn Glu Asp Pro Cys Ile Glu Pro Cys Leu Cys Lys Lys Asn
           470                 475                 480
   gtt gag ggt aag aac tgt gat cgc tgc aag cca gga ttc tac aac ttg    1545
   Val Glu Gly Lys Asn Cys Asp Arg Cys Lys Pro Gly Phe Tyr Asn Leu
       485                 490                 495
   aag gaa cga aac ccc gag ggc tgc tcc gag tgc ttc tgc ttc ggt gtc    1593
   Lys Glu Arg Asn Pro Glu Gly Cys Ser Glu Cys Phe Cys Phe Gly Val
   500                 505                 510                 515
   tct ggt gtc tgt gac agc ctc acg tgg tcc att agt cag gtg acc aat    1641
   Ser Gly Val Cys Asp Ser Leu Thr Trp Ser Ile Ser Gln Val Thr Asn
                   520                 525                 530
   atg tca ggg tgg ctg gtc act gac ttg atg agc aca aat aag atc cgg    1689
   Met Ser Gly Trp Leu Val Thr Asp Leu Met Ser Thr Asn Lys Ile Arg
               535                 540                 545
   tcc cag caa gat gtc ctg ggt ggg cac cgt cag atc agc atc aac aac    1737
   Ser Gln Gln Asp Val Leu Gly Gly His Arg Gln Ile Ser Ile Asn Asn
           550                 555                 560
   acg gcg gtc atg cag agg ctg act tcc act tac tac tgg gca gct cct    1785
   Thr Ala Val Met Gln Arg Leu Thr Ser Thr Tyr Tyr Trp Ala Ala Pro
       565                 570                 575
   gag gcc tac ctc gga aac aag ctg aca gca ttt ggc ggt ttc ctg aaa    1833
   Glu Ala Tyr Leu Gly Asn Lys Leu Thr Ala Phe Gly Gly Phe Leu Lys
   580                 585                 590                 595
   tac act gtg tct tac gac att cca gtg gag acg gtg gac agt gac ctc    1881
   Tyr Thr Val Ser Tyr Asp Ile Pro Val Glu Thr Val Asp Ser Asp Leu
                   600                 605                 610
   atg tct cat gct gac atc atc atc aag ggg aat ggg ctc acc ata agc    1929
   Met Ser His Ala Asp Ile Ile Ile Lys Gly Asn Gly Leu Thr Ile Ser
               615                 620                 625
   aca aga gct gag ggc ctg tcc ttg caa ccc tat gag gaa tac ttc aac    1977
   Thr Arg Ala Glu Gly Leu Ser Leu Gln Pro Tyr Glu Glu Tyr Phe Asn
           630                 635                 640
   gtg gtt aga ctt gtg cct gag aac ttc cgg gac ttt aat acc aga agg    2025
   Val Val Arg Leu Val Pro Glu Asn Phe Arg Asp Phe Asn Thr Arg Arg
       645                 650                 655
   gag att gac cgt gac cag ctg atg act gtc ctg gcc aat gtg aca cat    2073
   Glu Ile Asp Arg Asp Gln Leu Met Thr Val Leu Ala Asn Val Thr His
   660                 665                 670                 675
   ctc ttg atc aga gcc aat tat aat tct gct aaa atg gcg ctc tat agg    2121
   Leu Leu Ile Arg Ala Asn Tyr Asn Ser Ala Lys Met Ala Leu Tyr Arg
                   680                 685                 690
   ctg gat tct gtc tct ctg gac ata gca agc cct aat gct ata gac ttg    2169
   Leu Asp Ser Val Ser Leu Asp Ile Ala Ser Pro Asn Ala Ile Asp Leu
               695                 700                 705
   gca gtg gct gct gat gtg gaa cac tgt gaa tgt ccc caa ggc tac acg    2217
   Ala Val Ala Ala Asp Val Glu His Cys Glu Cys Pro Gln Gly Tyr Thr
           710                 715                 720
   ggg acc tcc tgt gag gcc tgc ctc cct ggc tat tat cga gtg gac ggg    2265
   Gly Thr Ser Cys Glu Ala Cys Leu Pro Gly Tyr Tyr Arg Val Asp Gly
       725                 730                 735
   ata ctc ttt gga gga atc tgt cag ccc tgc gag tgc cac ggg cat gca    2313
   Ile Leu Phe Gly Gly Ile Cys Gln Pro Cys Glu Cys His Gly His Ala
   740                 745                 750                 755
   tcc gag tgt gac att cat gga att tgc tct gtg tgt aca cac aac acc    2361
   Ser Glu Cys Asp Ile His Gly Ile Cys Ser Val Cys Thr His Asn Thr
                   760                 765                 770
   acg ggg gat cac tgt gag cag tgc ctg cct ggc ttc tat ggg aca cct    2409
   Thr Gly Asp His Cys Glu Gln Cys Leu Pro Gly Phe Tyr Gly Thr Pro
               775                 780                 785
   tca cgt ggg acc cca gga gac tgc cag cct tgt gcc tgc cct ctc tcc    2457
   Ser Arg Gly Thr Pro Gly Asp Cys Gln Pro Cys Ala Cys Pro Leu Ser
           790                 795                 800
   att gac tct aac aat ttc agc cct acc tgc cac ctc act gat gga gag    2505
   Ile Asp Ser Asn Asn Phe Ser Pro Thr Cys His Leu Thr Asp Gly Glu
       805                 810                 815
   gaa gtg gtt tgt gac caa tgt gcc ccg ggt tac tca gga tcc tgg tgt    2553
   Glu Val Val Cys Asp Gln Cys Ala Pro Gly Tyr Ser Gly Ser Trp Cys
   820                 825                 830                 835
   gag aga tgt gca gat ggt tac tat gga aac cca acc gtg cca ggg gga    2601
   Glu Arg Cys Ala Asp Gly Tyr Tyr Gly Asn Pro Thr Val Pro Gly Gly
                   840                 845                 850
   acc tgt gta cca tgc aac tgc agt ggc aat gtt gat ccc ttg gag gct    2649
   Thr Cys Val Pro Cys Asn Cys Ser Gly Asn Val Asp Pro Leu Glu Ala
               855                 860                 865
   ggc cac tgt gac tct gtc acg ggg gaa tgc ctg aag tgc tta tgg aac    2697
   Gly His Cys Asp Ser Val Thr Gly Glu Cys Leu Lys Cys Leu Trp Asn
           870                 875                 880
   aca gac ggt gcc cat tgt gag agg tgt gca gat ggc ttc tat gga gat    2745
   Thr Asp Gly Ala His Cys Glu Arg Cys Ala Asp Gly Phe Tyr Gly Asp
       885                 890                 895
   gcc gtg act gcc aaa aac tgc cga gcc tgt gac tgc cac gag aat ggc    2793
   Ala Val Thr Ala Lys Asn Cys Arg Ala Cys Asp Cys His Glu Asn Gly
   900                 905                 910                 915
   tcc ctt tct ggc gtc tgc cat ctg gag act gga ctg tgt gac tgc aaa    2841
   Ser Leu Ser Gly Val Cys His Leu Glu Thr Gly Leu Cys Asp Cys Lys
                   920                 925                 930
   cct cac gtg aca gga cag cag tgt gac cag tgc ctg tct ggc tac tac    2889
   Pro His Val Thr Gly Gln Gln Cys Asp Gln Cys Leu Ser Gly Tyr Tyr
               935                 940                 945
   ggg ttg gac acg ggg ctt ggc tgt gtg ccc tgt aac tgc agt gtg gaa    2937
   Gly Leu Asp Thr Gly Leu Gly Cys Val Pro Cys Asn Cys Ser Val Glu
           950                 955                 960
   ggc tct gta tct gac aac tgc acg gag gaa gga cag tgt cac tgt gga    2985
   Gly Ser Val Ser Asp Asn Cys Thr Glu Glu Gly Gln Cys His Cys Gly
       965                 970                 975
   cca ggt gtc tct ggg aaa cag tgt gac agg tgt tca cat ggt ttc tat    3033
   Pro Gly Val Ser Gly Lys Gln Cys Asp Arg Cys Ser His Gly Phe Tyr
   980                 985                 990                 995
   gca ttc cag gat ggc ggc tgc aca ccc tgt gac tgt gct cat acc cag    3081
   Ala Phe Gln Asp Gly Gly Cys Thr Pro Cys Asp Cys Ala His Thr Gln
                   1000                1005                1010
   aat aac tgt gac ccc gcc tct gga gag tgt ctc tgc ccg cct cac acg    3129
   Asn Asn Cys Asp Pro Ala Ser Gly Glu Cys Leu Cys Pro Pro His Thr
               1015                1020                1025
   cag ggg ctg aag tgt gag gag tgt gaa gag gca tac tgg ggt ctg gac    3177
   Gln Gly Leu Lys Cys Glu Glu Cys Glu Glu Ala Tyr Trp Gly Leu Asp
           1030                1035                1040
   ccg gag cag ggg tgc cag gct tgc aat tgc agt gct gtg ggc tcc acg    3225
   Pro Glu Gln Gly Cys Gln Ala Cys Asn Cys Ser Ala Val Gly Ser Thr
       1045                1050                1055
   agt gcc cag tgt gat gtt ctc tct ggc cac tgc ccc tgc aaa aaa ggg    3273
   Ser Ala Gln Cys Asp Val Leu Ser Gly His Cys Pro Cys Lys Lys Gly
   1060                1065                1070                1075
   ttt ggt ggg cag agc tgc cat cag tgt tcc tta ggc tac aga agt ttt    3321
   Phe Gly Gly Gln Ser Cys His Gln Cys Ser Leu Gly Tyr Arg Ser Phe
                   1080                1085                1090
   cct gac tgt gtc ccc tgt ggc tgt gac ctg agg ggg aca ctg cct gac    3369
   Pro Asp Cys Val Pro Cys Gly Cys Asp Leu Arg Gly Thr Leu Pro Asp
               1095                1100                1105
   acc tgt gac ctg gaa cag ggt ctc tgc agc tgc tca gag gac agt ggt    3417
   Thr Cys Asp Leu Glu Gln Gly Leu Cys Ser Cys Ser Glu Asp Ser Gly
           1110                1115                1120
   acc tgc tcc tgc aag gag aat gtc gtg ggc ccc cag tgc agt aag tgc    3465
   Thr Cys Ser Cys Lys Glu Asn Val Val Gly Pro Gln Cys Ser Lys Cys
       1125                1130                1135
   caa gcc ggc acc ttt gcc ttg cga ggg gac aac cct caa ggc tgc agc    3513
   Gln Ala Gly Thr Phe Ala Leu Arg Gly Asp Asn Pro Gln Gly Cys Ser
   1140                1145                1150                1155
   ccc tgc ttc tgc ttc ggt ctg tcg cag ctc tgc tca gag ttg gag ggt    3561
   Pro Cys Phe Cys Phe Gly Leu Ser Gln Leu Cys Ser Glu Leu Glu Gly
                   1160                1165                1170
   tac gtg agg act ctg ata act cta gcc tcc gat cag ccc ctc ctg cat    3609
   Tyr Val Arg Thr Leu Ile Thr Leu Ala Ser Asp Gln Pro Leu Leu His
               1175                1180                1185
   gtg gtt tca cag agc aac ctc aag ggc aca atc gaa ggc gtg cat ttc    3657
   Val Val Ser Gln Ser Asn Leu Lys Gly Thr Ile Glu Gly Val His Phe
           1190                1195                1200
   cag cct cct gac acc ttg ctg gac gca gag gct gtc cgc cag cat atc    3705
   Gln Pro Pro Asp Thr Leu Leu Asp Ala Glu Ala Val Arg Gln His Ile
       1205                1210                1215
   tat gca gag cca ttt tac tgg cgg cta cca aag cag ttc cag gga gac    3753
   Tyr Ala Glu Pro Phe Tyr Trp Arg Leu Pro Lys Gln Phe Gln Gly Asp
   1220                1225                1230                1235
   cag ctc ttg gcc tat ggt ggg aaa ctc cag tac agt gtg gct ttc tac    3801
   Gln Leu Leu Ala Tyr Gly Gly Lys Leu Gln Tyr Ser Val Ala Phe Tyr
                   1240                1245                1250
   tct aca ctt ggc acc gga aca tcc aat tat gag cct caa gtc ctc atc    3849
   Ser Thr Leu Gly Thr Gly Thr Ser Asn Tyr Glu Pro Gln Val Leu Ile
               1255                1260                1265
   aaa gga ggt cgg gcc agg aag cac gtc att tat atg gat gcc cca gcg    3897
   Lys Gly Gly Arg Ala Arg Lys His Val Ile Tyr Met Asp Ala Pro Ala
           1270                1275                1280
   cct gag aat gga gtg aga cag gat tac gaa gtg cag atg aaa gag gaa    3945
   Pro Glu Asn Gly Val Arg Gln Asp Tyr Glu Val Gln Met Lys Glu Glu
       1285                1290                1295
   ttc tgg aaa tat ttt aac tcc gtg tct gag aaa cac gtc aca cac tct    3993
   Phe Trp Lys Tyr Phe Asn Ser Val Ser Glu Lys His Val Thr His Ser
   1300                1305                1310                1315
   gat ttt atg tct gtt ctc agc aat att gac tac atc ctc atc aaa gca    4041
   Asp Phe Met Ser Val Leu Ser Asn Ile Asp Tyr Ile Leu Ile Lys Ala
                   1320                1325                1330
   tca tac ggc cag gga ctg cag cag agc aga att gcc aac att tcc atg    4089
   Ser Tyr Gly Gln Gly Leu Gln Gln Ser Arg Ile Ala Asn Ile Ser Met
               1335                1340                1345
   gag gtt ggc cgg aaa gct gtc gag ctg ccc gct gag ggc gag gcg gca    4137
   Glu Val Gly Arg Lys Ala Val Glu Leu Pro Ala Glu Gly Glu Ala Ala
           1350                1355                1360
   ttg ctg ttg gag ctc tgt gtc tgt cct cct ggc acc gca gga cac tcc    4185
   Leu Leu Leu Glu Leu Cys Val Cys Pro Pro Gly Thr Ala Gly His Ser
       1365                1370                1375
   tgt cag gac tgt gct cct ggg tac tac aga gaa aag ctc cca gaa agt    4233
   Cys Gln Asp Cys Ala Pro Gly Tyr Tyr Arg Glu Lys Leu Pro Glu Ser
   1380                1385                1390                1395
   ggt ggc agg gga ccc cgc cct ctg ctg gct cct tgt gtg ccc tgc aat    4281
   Gly Gly Arg Gly Pro Arg Pro Leu Leu Ala Pro Cys Val Pro Cys Asn
                   1400                1405                1410
   tgc aac aac cac agt gat gtc tgt gac ccc gaa act gga aag tgc ctg    4329
   Cys Asn Asn His Ser Asp Val Cys Asp Pro Glu Thr Gly Lys Cys Leu
               1415                1420                1425
   agc tgc agg gac cac aca tcc ggg gac cac tgt gag ctg tgt gct tct    4377
   Ser Cys Arg Asp His Thr Ser Gly Asp His Cys Glu Leu Cys Ala Ser
           1430                1435                1440
   ggc tac tat ggg aag gtg act gga ctg cct gga gac tgt acc ccg tgc    4425
   Gly Tyr Tyr Gly Lys Val Thr Gly Leu Pro Gly Asp Cys Thr Pro Cys
       1445                1450                1455
   acc tgt cct cat cac cct cct ttc agt ttc agc ccc act tgt gtc gtg    4473
   Thr Cys Pro His His Pro Pro Phe Ser Phe Ser Pro Thr Cys Val Val
   1460                1465                1470                1475
   gaa ggt gac agt gat ttc cgg tgc aat gcc tgc ctc ccc ggc tat gaa    4521
   Glu Gly Asp Ser Asp Phe Arg Cys Asn Ala Cys Leu Pro Gly Tyr Glu
                   1480                1485                1490
   gga cag tac tgt gaa agg tgc tct gca ggc tat cac ggc aac cct cga    4569
   Gly Gln Tyr Cys Glu Arg Cys Ser Ala Gly Tyr His Gly Asn Pro Arg
               1495                1500                1505
   gca gca ggt ggt agc tgc caa acg tgt gat tgc aac ccc caa ggc tct    4617
   Ala Ala Gly Gly Ser Cys Gln Thr Cys Asp Cys Asn Pro Gln Gly Ser
           1510                1515                1520
   gtc cac agt gac tgt gac cgt gca tcc ggg cag tgt gtc tgc aag cca    4665
   Val His Ser Asp Cys Asp Arg Ala Ser Gly Gln Cys Val Cys Lys Pro
       1525                1530                1535
   gga gct aca ggg ctc cac tgt gag aaa tgc ctg ccg aga cac atc ctg    4713
   Gly Ala Thr Gly Leu His Cys Glu Lys Cys Leu Pro Arg His Ile Leu
   1540                1545                1550                1555
   atg gag agc gac tgt gtt tcc tgt gat gat gac tgt gtg ggt cct ttg    4761
   Met Glu Ser Asp Cys Val Ser Cys Asp Asp Asp Cys Val Gly Pro Leu
                   1560                1565                1570
   ctg aac gac ctg gat tct gtt ggt gat gcc gtt ctg tct ctg aac ctc    4809
   Leu Asn Asp Leu Asp Ser Val Gly Asp Ala Val Leu Ser Leu Asn Leu
               1575                1580                1585
   acg ggc gtt tcc cct gct ccc tat gga atc ctg gaa aat ctg gaa aat    4857
   Thr Gly Val Ser Pro Ala Pro Tyr Gly Ile Leu Glu Asn Leu Glu Asn
           1590                1595                1600
   aca act aaa tat ttc cag agg tat tta ata aag gaa aat gcc aag aag    4905
   Thr Thr Lys Tyr Phe Gln Arg Tyr Leu Ile Lys Glu Asn Ala Lys Lys
       1605                1610                1615
   att cga gca gag atc cag ctc gaa ggg att gca gag caa aca gaa aat    4953
   Ile Arg Ala Glu Ile Gln Leu Glu Gly Ile Ala Glu Gln Thr Glu Asn
   1620                1625                1630                1635
   ctg caa aag gag ctc acc aga gtg tta gca cgc cat cag aag gtg aac    5001
   Leu Gln Lys Glu Leu Thr Arg Val Leu Ala Arg His Gln Lys Val Asn
                   1640                1645                1650
   gct gaa atg gaa aga act tcc aat ggg act caa gcc ctg gcc acg ttc    5049
   Ala Glu Met Glu Arg Thr Ser Asn Gly Thr Gln Ala Leu Ala Thr Phe
               1655                1660                1665
   att gag cag cta cat gca aac atc aaa gaa atc aca gaa aag gtg gca    5097
   Ile Glu Gln Leu His Ala Asn Ile Lys Glu Ile Thr Glu Lys Val Ala
           1670                1675                1680
   acg ttg aac cag acg gcg cgt aaa gat ttc cag cca ccc gtg tct gcc    5145
   Thr Leu Asn Gln Thr Ala Arg Lys Asp Phe Gln Pro Pro Val Ser Ala
       1685                1690                1695
   ctt cag agc atg cac cag aac att tcg tct ctg ctg gga ctc atc aag    5193
   Leu Gln Ser Met His Gln Asn Ile Ser Ser Leu Leu Gly Leu Ile Lys
   1700                1705                1710                1715
   gaa agg aat ttc aca gag atg cag cag aat gct acc ctt gag ctc aag    5241
   Glu Arg Asn Phe Thr Glu Met Gln Gln Asn Ala Thr Leu Glu Leu Lys
                   1720                1725                1730
   gct gct aaa gac tta ttg tca cgg att cag aaa agg ttt cag aag cct    5289
   Ala Ala Lys Asp Leu Leu Ser Arg Ile Gln Lys Arg Phe Gln Lys Pro
               1735                1740                1745
   cag gaa aag ttg aag gca ttg aag gag gcc aac agc ctc ctt tcc aac    5337
   Gln Glu Lys Leu Lys Ala Leu Lys Glu Ala Asn Ser Leu Leu Ser Asn
           1750                1755                1760
   cac agt gaa aaa ctg cag gct gct gag gag ctc ctt aag gaa gct gga    5385
   His Ser Glu Lys Leu Gln Ala Ala Glu Glu Leu Leu Lys Glu Ala Gly
       1765                1770                1775
   agc aag acc cag gag agc aac ctc ctg ctg ctc ctt gtc aag gcc aac    5433
   Ser Lys Thr Gln Glu Ser Asn Leu Leu Leu Leu Leu Val Lys Ala Asn
   1780                1785                1790                1795
   ctg aaa gag gaa ttc cag gag aaa aag ctg cgt gtt caa gaa gaa caa    5481
   Leu Lys Glu Glu Phe Gln Glu Lys Lys Leu Arg Val Gln Glu Glu Gln
                   1800                1805                1810
   aat gtg acc tca gag ctc att gcc aag ggt aga gaa tgg gtg gat gct    5529
   Asn Val Thr Ser Glu Leu Ile Ala Lys Gly Arg Glu Trp Val Asp Ala
               1815                1820                1825
   gcc ggg act cac aca gct gct gca caa gac acc cta aca cag ctg gag    5577
   Ala Gly Thr His Thr Ala Ala Ala Gln Asp Thr Leu Thr Gln Leu Glu
           1830                1835                1840
   cat cac cga gat gaa ctc ctt ctg tgg gcc aga aaa atc agg agc cac    5625
   His His Arg Asp Glu Leu Leu Leu Trp Ala Arg Lys Ile Arg Ser His
       1845                1850                1855
   gta gat gac ctc gtc atg cag atg tcc aaa cga aga gcc cgt gac ctg    5673
   Val Asp Asp Leu Val Met Gln Met Ser Lys Arg Arg Ala Arg Asp Leu
   1860                1865                1870                1875
   gtc cac agg gca gag cag cat gcc tct gag ctg cag agc agg gca gga    5721
   Val His Arg Ala Glu Gln His Ala Ser Glu Leu Gln Ser Arg Ala Gly
                   1880                1885                1890
   gct ttg gac aga gac ctt gaa aat gtt aga aac gtg tct ttg aat gcc    5769
   Ala Leu Asp Arg Asp Leu Glu Asn Val Arg Asn Val Ser Leu Asn Ala
               1895                1900                1905
   acc agt gcg gca cac gtc cac agc aac atc cag aca ctg aca gag gaa    5817
   Thr Ser Ala Ala His Val His Ser Asn Ile Gln Thr Leu Thr Glu Glu
           1910                1915                1920
   gct gag atg ctg gct gct gat gct cac aag acg gcg aat aag aca gac    5865
   Ala Glu Met Leu Ala Ala Asp Ala His Lys Thr Ala Asn Lys Thr Asp
       1925                1930                1935
   ttg atc tcc gaa tcc ctg gct tct cgg ggg aaa gca gtc ctt cag cgc    5913
   Leu Ile Ser Glu Ser Leu Ala Ser Arg Gly Lys Ala Val Leu Gln Arg
   1940                1945                1950                1955
   tcg tcc cgg ttt cta aag gaa agt gtc ggt acc agg agg aag cag caa    5961
   Ser Ser Arg Phe Leu Lys Glu Ser Val Gly Thr Arg Arg Lys Gln Gln
                   1960                1965                1970
   ggc att acg atg aag ctg gat gag ttg aaa aac tta acg agt caa ttt    6009
   Gly Ile Thr Met Lys Leu Asp Glu Leu Lys Asn Leu Thr Ser Gln Phe
               1975                1980                1985
   cag gag agc gtg gat aac att acg aag cag gcc aac gac tcc ctt gcg    6057
   Gln Glu Ser Val Asp Asn Ile Thr Lys Gln Ala Asn Asp Ser Leu Ala
           1990                1995                2000
   atg ctt aga gaa agc cct gga ggt atg aga gag aag ggc agg aaa gcc    6105
   Met Leu Arg Glu Ser Pro Gly Gly Met Arg Glu Lys Gly Arg Lys Ala
       2005                2010                2015
   aga gag ctg gcg gca gca gcc aac gag agt gcg gtg aag aca ctg gag     6153
   Arg Glu Leu Ala Ala Ala Ala Asn Glu Ser Ala Val Lys Thr Leu Glu
   2020                2025                2030                2035
   gat gtg ctg gct ttg agc ctg agg gtc ttc aat aca tca gag gac ctg     6201
   Asp Val Leu Ala Leu Ser Leu Arg Val Phe Asn Thr Ser Glu Asp Leu
                   2040                2045                2050
   tcc aga gtg aat gcc aca gtc cag gag aca aac gac ctt ctg cat aac     6249
   Ser Arg Val Asn Ala Thr Val Gln Glu Thr Asn Asp Leu Leu His Asn
               2055                2060                2065
   tcc acg atg acc act ctg ttg gct gga aga aaa atg aaa gac atg gaa     6297
   Ser Thr Met Thr Thr Leu Leu Ala Gly Arg Lys Met Lys Asp Met Glu
           2070                2075                2080
   atg caa gcc aac ctt tta ttg gat cga ttg aaa cct ttg aaa acc ctg     6345
   Met Gln Ala Asn Leu Leu Leu Asp Arg Leu Lys Pro Leu Lys Thr Leu
       2085                2090                2095
   gag gag aac ctg agc aga aac ctg tcg gag atc aag ctg ctg atc agc     6393
   Glu Glu Asn Leu Ser Arg Asn Leu Ser Glu Ile Lys Leu Leu Ile Ser
   2100                2105                2110                2115
   cgg gcc cgg aaa caa gcg gcg tcg atc aaa gtc gcc gtg tct gca gac     6441
   Arg Ala Arg Lys Gln Ala Ala Ser Ile Lys Val Ala Val Ser Ala Asp
                   2120                2125                2130
   aga gac tgc atc cgc gcc tat cag cct cag act tca tct acc aat tac     6489
   Arg Asp Cys Ile Arg Ala Tyr Gln Pro Gln Thr Ser Ser Thr Asn Tyr
               2135                2140                2145
   aac acc ttg atc ctg aac gtg aag acg cag gag ccc gac aac ctc ctc     6537
   Asn Thr Leu Ile Leu Asn Val Lys Thr Gln Glu Pro Asp Asn Leu Leu
           2150                2155                2160
   ttc tac ctc ggc agc agc agc agt tct gac ttt ctc gca gtg gag atg     6585
   Phe Tyr Leu Gly Ser Ser Ser Ser Ser Asp Phe Leu Ala Val Glu Met
       2165                2170                2175
   cgg cgg ggg aag gtc gcc ttt ctc tgg gac ctg ggc tcc ggg tcc aca     6633
   Arg Arg Gly Lys Val Ala Phe Leu Trp Asp Leu Gly Ser Gly Ser Thr
   2180                2185                2190                2195
   agg ttg gaa ttc cca gag gtc tcc atc aat aac aac aga tgg cac agc     6681
   Arg Leu Glu Phe Pro Glu Val Ser Ile Asn Asn Asn Arg Trp His Ser
                   2200                2205                2210
   atc tac ata acc agg ttt gga aac atg ggg tcc ctg agt gta aag gaa     6729
   Ile Tyr Ile Thr Arg Phe Gly Asn Met Gly Ser Leu Ser Val Lys Glu
               2215                2220                2225
   gca agc gct gcc gag aac cca ccg gtc agg aca agc aaa tct cct gga     6777
   Ala Ser Ala Ala Glu Asn Pro Pro Val Arg Thr Ser Lys Ser Pro Gly
           2230                2235                2240
   ccg tcg aag gtt ctg gac ata aac aat tca acg ctg atg ttt gtt gga     6825
   Pro Ser Lys Val Leu Asp Ile Asn Asn Ser Thr Leu Met Phe Val Gly
       2245                2250                2255
   ggg ctc gga ggt cag atc aag aaa tcc ccg gct gtg aag gtt act cat    6873
   Gly Leu Gly Gly Gln Ile Lys Lys Ser Pro Ala Val Lys Val Thr His
   2260                2265                2270                2275
   ttt aag ggc tgc atg gga gag gcc ttc ttg aat ggc aaa tcg att ggc    6921
   Phe Lys Gly Cys Met Gly Glu Ala Phe Leu Asn Gly Lys Ser Ile Gly
                   2280                2285                2290
   ctg tgg aat tac atc gag aga gag ggg aag tgc aat ggc tgc ttt gga    6969
   Leu Trp Asn Tyr Ile Glu Arg Glu Gly Lys Cys Asn Gly Cys Phe Gly
               2295                2300                2305
   agc tcc cag aac gaa gat tcc tcc ttc cat ttc gat gga agc ggg tac    7017
   Ser Ser Gln Asn Glu Asp Ser Ser Phe His Phe Asp Gly Ser Gly Tyr
           2310                2315                2320
   gcc atg gtg gag aag acg ctc cgg ccc acc gtg acg cag ata gta att    7065
   Ala Met Val Glu Lys Thr Leu Arg Pro Thr Val Thr Gln Ile Val Ile
       2325                2330                2335
   ctc ttc agc acc ttc tcc ccg aat ggc ctt ctt ttc tac ctg gct tca    7113
   Leu Phe Ser Thr Phe Ser Pro Asn Gly Leu Leu Phe Tyr Leu Ala Ser
   2340                2345                2350                2355
   aac ggc acc aag gac ttc cta tcc atc gag ctg gtc cgt ggc agg gtc    7161
   Asn Gly Thr Lys Asp Phe Leu Ser Ile Glu Leu Val Arg Gly Arg Val
                   2360                2365                2370
   aaa gtg atg gtg gac cta ggg tca gga ccc ctc act ctt atg aca gac    7209
   Lys Val Met Val Asp Leu Gly Ser Gly Pro Leu Thr Leu Met Thr Asp
               2375                2380                2385
   agg cgg tat aac aac gga acc tgg tat aaa atc gcc ttc cag cgg aac    7257
   Arg Arg Tyr Asn Asn Gly Thr Trp Tyr Lys Ile Ala Phe Gln Arg Asn
           2390                2395                2400
   cgg aag caa gga ctg cta gct gtc ttc gat gca tat gac acc agt gac    7305
   Arg Lys Gln Gly Leu Leu Ala Val Phe Asp Ala Tyr Asp Thr Ser Asp
       2405                2410                2415
   aag gag aca aag caa gga gag act cca gga gcc gct tcc gac ctc aat    7353
   Lys Glu Thr Lys Gln Gly Glu Thr Pro Gly Ala Ala Ser Asp Leu Asn
   2420                2425                2430                2435
   cgg ctg gag aaa gac ctg att tac gtg ggt gga ttg cct cat tct aag    7401
   Arg Leu Glu Lys Asp Leu Ile Tyr Val Gly Gly Leu Pro His Ser Lys
                   2440                2445                2450
   gct gtg agg aaa ggg gtc agc agc aga agc tat gtg ggc tgt atc aag    7449
   Ala Val Arg Lys Gly Val Ser Ser Arg Ser Tyr Val Gly Cys Ile Lys
               2455                2460                2465
   aac cta gag ata tcc agg tcc acc ttt gat ttg ctg aga aat tcc tac    7497
   Asn Leu Glu Ile Ser Arg Ser Thr Phe Asp Leu Leu Arg Asn Ser Tyr
           2470                2475                2480
   gga gtg aga aaa ggc tgc gca ctg gag cct atc cag agt gtg agt ttc    7545
   Gly Val Arg Lys Gly Cys Ala Leu Glu Pro Ile Gln Ser Val Ser Phe
       2485                2490                2495
   ctg aga ggc ggc tat gtg gag atg cca ccc aag tct ctc tca cct gag 7593
   Leu Arg Gly Gly Tyr Val Glu Met Pro Pro Lys Ser Leu Ser Pro Glu
   2500 2505 2510 2515
   tca tcc ctg ctg gcc aca ttc gcc acc aag aac agc agc gga atc ctc 7641
   Ser Ser Leu Leu Ala Thr Phe Ala Thr Lys Asn Ser Ser Gly Ile Leu
   2520 2525 2530
   ctg gtt gcc ctg ggc aag gat gcg gag gag gct ggt ggg gct cag gca 7689
   Leu Val Ala Leu Gly Lys Asp Ala Glu Glu Ala Gly Gly Ala Gln Ala
   2535 2540 2545
   cat gtg ccc ttc ttt tcc atc atg ctg ctt gag gga cga att gaa gtg 7737
   His Val Pro Phe Phe Ser Ile Met Leu Leu Glu Gly Arg Ile Glu Val
   2550 2555 2560
   cat gtc aac tct ggg gac ggg acc agt ctg agg aag gcc ctc ctg cat 7785
   His Val Asn Ser Gly Asp Gly Thr Ser Leu Arg Lys Ala Leu Leu His
   2565 2570 2575
   gcc ccc acc ggc tcc tac agt gat gga cag gaa cac tcc atc tcc ctg 7833
   Ala Pro Thr Gly Ser Tyr Ser Asp Gly Gln Glu His Ser Ile Ser Leu
   2580 2585 2590 2595
   gtt agg aat cgg aga gtt atc acc ata caa gtg gat gag aac agt ccc 7881
   Val Arg Asn Arg Arg Val Ile Thr Ile Gln Val Asp Glu Asn Ser Pro
   2600 2605 2610
   gta gaa atg aag ttg ggt cca tta aca gaa gga aag acg atc gac ata 7929
   Val Glu Met Lys Leu Gly Pro Leu Thr Glu Gly Lys Thr Ile Asp Ile
   2615 2620 2625
   tcc aac ctg tac ata ggg gga ctt ccg gag gac aag gcg acc ccg atg 7977
   Ser Asn Leu Tyr Ile Gly Gly Leu Pro Glu Asp Lys Ala Thr Pro Met
   2630 2635 2640
   ctc aag atg cgg act tcg ttc cat ggg tgt att aaa aac gtg gtc ctt 8025
   Leu Lys Met Arg Thr Ser Phe His Gly Cys Ile Lys Asn Val Val Leu
   2645 2650 2655
   gac gct caa ctt ttg gac ttc acc cat gcg act ggc tct gag caa gta 8073
   Asp Ala Gln Leu Leu Asp Phe Thr His Ala Thr Gly Ser Glu Gln Val
   2660 2665 2670 2675
   gag ctg gac aca tgc ttg ctg gca gaa gag ccc atg cag agt ctg cac 8121
   Glu Leu Asp Thr Cys Leu Leu Ala Glu Glu Pro Met Gln Ser Leu His
   2680 2685 2690
   aga gaa cac ggg gaa ctc cct ccg gag ccc cca act cta cca cag cct 8169
   Arg Glu His Gly Glu Leu Pro Pro Glu Pro Pro Thr Leu Pro Gln Pro
   2695 2700 2705
   gaa ctg tgc gca gta gac acg gct ccg ggg tat gtg gca ggt gct cac 8217
   Glu Leu Cys Ala Val Asp Thr Ala Pro Gly Tyr Val Ala Gly Ala His
   2710 2715 2720
   cag ttt ggc ctc tcg cag aac agc cac ttg gtg ctc cct ctg aat cag 8265
   Gln Phe Gly Leu Ser Gln Asn Ser His Leu Val Leu Pro Leu Asn Gln
   2725 2730 2735
   tct gat gtc cgg aag agg ctc cag gtg cag ctg agc att cgg aca ttt    8313
   Ser Asp Val Arg Lys Arg Leu Gln Val Gln Leu Ser Ile Arg Thr Phe
   2740                2745                2750                2755
   gcc tcc agt ggc ctc att tac tat gtg gct cat cag aac caa atg gac    8361
   Ala Ser Ser Gly Leu Ile Tyr Tyr Val Ala His Gln Asn Gln Met Asp
                   2760                2765                2770
   tac gcc acg ctc cag ctc caa gag ggc cgc ctg cac ttc atg ttt gat    8409
   Tyr Ala Thr Leu Gln Leu Gln Glu Gly Arg Leu His Phe Met Phe Asp
               2775                2780                2785
   ctc ggc aag ggc cgg acc aag gtc tcc cac cct gcc ctg ctc agt gat    8457
   Leu Gly Lys Gly Arg Thr Lys Val Ser His Pro Ala Leu Leu Ser Asp
           2790                2795                2800
   ggc aag tgg cac aca gtc aag aca gaa tac att aaa agg aag gcg ttc    8505
   Gly Lys Trp His Thr Val Lys Thr Glu Tyr Ile Lys Arg Lys Ala Phe
       2805                2810                2815
   atg act gtt gac ggc caa gag tcc ccc agt gtg act gtg gtg ggc aat    8553
   Met Thr Val Asp Gly Gln Glu Ser Pro Ser Val Thr Val Val Gly Asn
   2820                2825                2830                2835
   gca acc acg ctg gat gtg gaa agg aaa ctg tac ctc gga ggc ctt ccc    8601
   Ala Thr Thr Leu Asp Val Glu Arg Lys Leu Tyr Leu Gly Gly Leu Pro
                   2840                2845                2850
   agc cac tac agg gcc agg aac atc ggg act atc acc cac agc atc cct    8649
   Ser His Tyr Arg Ala Arg Asn Ile Gly Thr Ile Thr His Ser Ile Pro
               2855                2860                2865
   gcc tgc att ggg gaa atc atg gtt aat ggc caa cag ctg gat aaa gac    8697
   Ala Cys Ile Gly Glu Ile Met Val Asn Gly Gln Gln Leu Asp Lys Asp
           2870                2875                2880
   agg ccc ttg tct gcc tct gct gtg gac agg tgc tat gtc gtg gct cag    8745
   Arg Pro Leu Ser Ala Ser Ala Val Asp Arg Cys Tyr Val Val Ala Gln
       2885                2890                2895
   gaa gga act ttc ttt gaa gga agc gga tat gca gct ctt gtc aag gaa    8793
   Glu Gly Thr Phe Phe Glu Gly Ser Gly Tyr Ala Ala Leu Val Lys Glu
   2900                2905                2910                2915
   ggt tac aaa gtt cga ttg gat tta aat atc aca ctg gag ttc cgt act    8841
   Gly Tyr Lys Val Arg Leu Asp Leu Asn Ile Thr Leu Glu Phe Arg Thr
                   2920                2925                2930
   acc tct aag aat ggc gtc ctc ctg gga atc agc agt gcc aaa gtg gat    8889
   Thr Ser Lys Asn Gly Val Leu Leu Gly Ile Ser Ser Ala Lys Val Asp
               2935                2940                2945
   gcc att ggc cta gag att gta gat ggc aag gtc tta ttt cac gtc aac    8937
   Ala Ile Gly Leu Glu Ile Val Asp Gly Lys Val Leu Phe His Val Asn
           2950                2955                2960
   aac ggt gcc gga agg ata aca gcc acc tac cag ccc aga gcc gcc aga    8985
   Asn Gly Ala Gly Arg Ile Thr Ala Thr Tyr Gln Pro Arg Ala Ala Arg
       2965                2970                2975
   gct ctc tgt gat ggc aag tgg cac aca ctc caa gcc cac aaa agc aag    9033
   Ala Leu Cys Asp Gly Lys Trp His Thr Leu Gln Ala His Lys Ser Lys
   2980                2985                2990                2995
   cac cgc atc gtc ctg act gtg gac ggg aat tcc gtt agg gct gaa agc    9081
   His Arg Ile Val Leu Thr Val Asp Gly Asn Ser Val Arg Ala Glu Ser
                   3000                3005                3010
   ccc cac acc cat tcc acc tcg gca gac acc aac gat ccc att tat gtg    9129
   Pro His Thr His Ser Thr Ser Ala Asp Thr Asn Asp Pro Ile Tyr Val
               3015                3020                3025
   ggt ggc tat cct gcc cac atc aaa cag aac tgc ctg agc agc cgg gcc    9177
   Gly Gly Tyr Pro Ala His Ile Lys Gln Asn Cys Leu Ser Ser Arg Ala
           3030                3035                3040
   tca ttc cgg ggc tgt gtg agg aac ctc agg ctg agc agg ggc tca caa    9225
   Ser Phe Arg Gly Cys Val Arg Asn Leu Arg Leu Ser Arg Gly Ser Gln
       3045                3050                3055
   gtg cag tcc ttg gac ctg agc cga gcc ttt gac cta caa gga gtc ttc    9273
   Val Gln Ser Leu Asp Leu Ser Arg Ala Phe Asp Leu Gln Gly Val Phe
   3060                3065                3070                3075
   cct cat tcc tgc ccc ggg cct gag ccc taaactgtcg ccagcctctg          9320
   Pro His Ser Cys Pro Gly Pro Glu Pro
                   3080
   cccttggaat catcgccaac gcatggaaga gagcagtttg tgaactcaag cagctcagct 9380
   cccattccca tcccattgcc atctcaggtt atgtttccag aggaaaatgc tgtatttatg 9440
   ttgaactaaa gccacacgga caacagatac ctctattaaa tggtttaaaa cgtcagtgga 9500
   att                                                               9503
<210> 2
   <211> 3084
   <212> PRT
   <213> Mus musculus
<400> 2
   Met Arg Gly Ser Gly Thr Gly Ala Ala Leu Leu Val Leu Leu Ala Ser
    1               5                  10                  15
   Val Leu Trp Val Thr Val Arg Ser Gln Gln Arg Gly Leu Phe Pro Ala
               20                  25                  30
   Ile Leu Asn Leu Ala Thr Asn Ala His Ile Ser Ala Asn Ala Thr Cys
           35                  40                  45
   Gly Glu Lys Gly Pro Glu Met Phe Cys Lys Leu Val Glu His Val Pro
       50                  55                  60
   Gly Arg Pro Val Arg His Ala Gln Cys Arg Val Cys Asp Gly Asn Ser
   65                  70                  75                  80
   Thr Asn Pro Arg Glu Arg His Pro Ile Ser His Ala Ile Asp Gly Thr
                   85                  90                  95
   Asn Asn Trp Trp Gln Ser Pro Ser Ile Gln Asn Gly Arg Glu Tyr His
               100                 105                 110
   Trp Val Thr Val Thr Leu Asp Leu Arg Gln Val Phe Gln Val Ala Tyr
           115                 120                 125
   Ile Ile Ile Lys Ala Ala Asn Ala Pro Arg Pro Gly Asn Trp Ile Leu
       130                 135                 140
   Glu Arg Ser Val Asp Gly Val Lys Phe Lys Pro Trp Gln Tyr Tyr Ala
   145                 150                 155                 160
   Val Ser Asp Thr Glu Cys Leu Thr Arg Tyr Lys Ile Thr Pro Arg Arg
                   165                 170                 175
   Gly Pro Pro Thr Tyr Arg Ala Asp Asn Glu Val Ile Cys Thr Ser Tyr
               180                 185                 190
   Tyr Ser Lys Leu Val Pro Leu Glu His Gly Glu Ile His Thr Ser Leu
           195                 200                 205
   Ile Asn Gly Arg Pro Ser Ala Asp Asp Pro Ser Pro Gln Leu Leu Glu
       210                 215                 220
   Phe Thr Ser Ala Arg Tyr Ile Arg Leu Arg Leu Gln Arg Ile Arg Thr
   225                 230                 235                 240
   Leu Asn Ala Asp Leu Met Thr Leu Ser His Arg Asp Leu Arg Asp Leu
                   245                 250                 255
   Asp Pro Ile Val Thr Arg Arg Tyr Tyr Tyr Ser Ile Lys Asp Ile Ser
               260                 265                 270
   Val Gly Gly Met Cys Ile Cys Tyr Gly His Ala Ser Ser Cys Pro Trp
           275                 280                 285
   Asp Glu Glu Ala Lys Gln Leu Gln Cys Gln Cys Glu His Asn Thr Cys
       290                 295                 300
   Gly Glu Ser Cys Asp Arg Cys Cys Pro Gly Tyr His Gln Gln Pro Trp
   305                 310                 315                 320
   Arg Pro Gly Thr Ile Ser Ser Gly Asn Glu Cys Glu Glu Cys Asn Cys
                   325                 330                 335
   His Asn Lys Ala Lys Asp Cys Tyr Tyr Asp Ser Ser Val Ala Lys Glu
               340                 345                 350
   Arg Arg Ser Leu Asn Thr Ala Gly Gln Tyr Ser Gly Gly Gly Val Cys
           355                 360                 365
   Val Asn Cys Ser Gln Asn Thr Thr Gly Ile Asn Cys Glu Thr Cys Ile
       370                 375                 380
   Asp Gln Tyr Tyr Arg Pro His Lys Val Ser Pro Tyr Asp Asp His Pro
   385                 390                 395                 400
   Cys Arg Pro Cys Asn Cys Asp Pro Val Gly Ser Leu Ser Ser Val Cys
                   405                 410                 415
   Ile Lys Asp Asp Arg His Ala Asp Leu Ala Asn Gly Lys Trp Pro Gly
               420                 425                 430
   Gln Cys Pro Cys Arg Lys Gly Tyr Ala Gly Asp Lys Cys Asp Arg Cys
           435                 440                 445
   Gln Phe Gly Tyr Arg Gly Phe Pro Asn Cys Ile Pro Cys Asp Cys Arg
       450                 455                 460
   Thr Val Gly Ser Leu Asn Glu Asp Pro Cys Ile Glu Pro Cys Leu Cys
   465                 470                 475                 480
   Lys Lys Asn Val Glu Gly Lys Asn Cys Asp Arg Cys Lys Pro Gly Phe
                   485                 490                 495
   Tyr Asn Leu Lys Glu Arg Asn Pro Glu Gly Cys Ser Glu Cys Phe Cys
               500                 505                 510
   Phe Gly Val Ser Gly Val Cys Asp Ser Leu Thr Trp Ser Ile Ser Gln
           515                 520                 525
   Val Thr Asn Met Ser Gly Trp Leu Val Thr Asp Leu Met Ser Thr Asn
       530                 535                 540
   Lys Ile Arg Ser Gln Gln Asp Val Leu Gly Gly His Arg Gln Ile Ser
   545                 550                 555                 560
   Ile Asn Asn Thr Ala Val Met Gln Arg Leu Thr Ser Thr Tyr Tyr Trp
                   565                 570                 575
   Ala Ala Pro Glu Ala Tyr Leu Gly Asn Lys Leu Thr Ala Phe Gly Gly
               580                 585                 590
   Phe Leu Lys Tyr Thr Val Ser Tyr Asp Ile Pro Val Glu Thr Val Asp
           595                 600                 605
   Ser Asp Leu Met Ser His Ala Asp Ile Ile Ile Lys Gly Asn Gly Leu
       610                 615                 620
   Thr Ile Ser Thr Arg Ala Glu Gly Leu Ser Leu Gln Pro Tyr Glu Glu
   625                 630                 635                 640
   Tyr Phe Asn Val Val Arg Leu Val Pro Glu Asn Phe Arg Asp Phe Asn
                   645                 650                 655
   Thr Arg Arg Glu Ile Asp Arg Asp Gln Leu Met Thr Val Leu Ala Asn
               660                 665                 670
   Val Thr His Leu Leu Ile Arg Ala Asn Tyr Asn Ser Ala Lys Met Ala
           675                 680                 685
   Leu Tyr Arg Leu Asp Ser Val Ser Leu Asp Ile Ala Ser Pro Asn Ala
       690                 695                 700
   Ile Asp Leu Ala Val Ala Ala Asp Val Glu His Cys Glu Cys Pro Gln
   705                 710                 715                 720
   Gly Tyr Thr Gly Thr Ser Cys Glu Ala Cys Leu Pro Gly Tyr Tyr Arg
                   725                 730                 735
   Val Asp Gly Ile Leu Phe Gly Gly Ile Cys Gln Pro Cys Glu Cys His
               740                 745                 750
   Gly His Ala Ser Glu Cys Asp Ile His Gly Ile Cys Ser Val Cys Thr
           755                 760                 765
   His Asn Thr Thr Gly Asp His Cys Glu Gln Cys Leu Pro Gly Phe Tyr
       770                 775                 780
   Gly Thr Pro Ser Arg Gly Thr Pro Gly Asp Cys Gln Pro Cys Ala Cys
   785                 790                 795                 800
   Pro Leu Ser Ile Asp Ser Asn Asn Phe Ser Pro Thr Cys His Leu Thr
                   805                 810                 815
   Asp Gly Glu Glu Val Val Cys Asp Gln Cys Ala Pro Gly Tyr Ser Gly
               820                 825                 830
   Ser Trp Cys Glu Arg Cys Ala Asp Gly Tyr Tyr Gly Asn Pro Thr Val
           835                 840                 845
   Pro Gly Gly Thr Cys Val Pro Cys Asn Cys Ser Gly Asn Val Asp Pro
       850                 855                 860
   Leu Glu Ala Gly His Cys Asp Ser Val Thr Gly Glu Cys Leu Lys Cys
   865                 870                 875                 880
   Leu Trp Asn Thr Asp Gly Ala His Cys Glu Arg Cys Ala Asp Gly Phe
                   885                 890                 895
   Tyr Gly Asp Ala Val Thr Ala Lys Asn Cys Arg Ala Cys Asp Cys His
               900                 905                 910
   Glu Asn Gly Ser Leu Ser Gly Val Cys His Leu Glu Thr Gly Leu Cys
           915                 920                 925
   Asp Cys Lys Pro His Val Thr Gly Gln Gln Cys Asp Gln Cys Leu Ser
       930                 935                 940
   Gly Tyr Tyr Gly Leu Asp Thr Gly Leu Gly Cys Val Pro Cys Asn Cys
   945                 950                 955                 960
   Ser Val Glu Gly Ser Val Ser Asp Asn Cys Thr Glu Glu Gly Gln Cys
                   965                 970                 975
   His Cys Gly Pro Gly Val Ser Gly Lys Gln Cys Asp Arg Cys Ser His
               980                 985                 990
   Gly Phe Tyr Ala Phe Gln Asp Gly Gly Cys Thr Pro Cys Asp Cys Ala
           995                 1000                1005
   His Thr Gln Asn Asn Cys Asp Pro Ala Ser Gly Glu Cys Leu Cys Pro
       1010                1015                1020
   Pro His Thr Gln Gly Leu Lys Cys Glu Glu Cys Glu Glu Ala Tyr Trp
   1025                1030                1035                1040
   Gly Leu Asp Pro Glu Gln Gly Cys Gln Ala Cys Asn Cys Ser Ala Val
                   1045                1050                1055
   Gly Ser Thr Ser Ala Gln Cys Asp Val Leu Ser Gly His Cys Pro Cys
               1060                1065                1070
   Lys Lys Gly Phe Gly Gly Gln Ser Cys His Gln Cys Ser Leu Gly Tyr
           1075                1080                1085
   Arg Ser Phe Pro Asp Cys Val Pro Cys Gly Cys Asp Leu Arg Gly Thr
       1090                1095                1100
   Leu Pro Asp Thr Cys Asp Leu Glu Gln Gly Leu Cys Ser Cys Ser Glu
   1105                1110                1115                1120
   Asp Ser Gly Thr Cys Ser Cys Lys Glu Asn Val Val Gly Pro Gln Cys
                   1125                1130                1135
   Ser Lys Cys Gln Ala Gly Thr Phe Ala Leu Arg Gly Asp Asn Pro Gln
               1140                1145                1150
   Gly Cys Ser Pro Cys Phe Cys Phe Gly Leu Ser Gln Leu Cys Ser Glu
           1155                1160                1165
   Leu Glu Gly Tyr Val Arg Thr Leu Ile Thr Leu Ala Ser Asp Gln Pro
       1170                1175                1180
   Leu Leu His Val Val Ser Gln Ser Asn Leu Lys Gly Thr Ile Glu Gly
   1185                1190                1195                1200
   Val His Phe Gln Pro Pro Asp Thr Leu Leu Asp Ala Glu Ala Val Arg
                   1205                1210                1215
   Gln His Ile Tyr Ala Glu Pro Phe Tyr Trp Arg Leu Pro Lys Gln Phe
               1220                1225                1230
   Gln Gly Asp Gln Leu Leu Ala Tyr Gly Gly Lys Leu Gln Tyr Ser Val
           1235                1240                1245
   Ala Phe Tyr Ser Thr Leu Gly Thr Gly Thr Ser Asn Tyr Glu Pro Gln
       1250                1255                1260
   Val Leu Ile Lys Gly Gly Arg Ala Arg Lys His Val Ile Tyr Met Asp
   1265                1270                1275                1280
   Ala Pro Ala Pro Glu Asn Gly Val Arg Gln Asp Tyr Glu Val Gln Met
                   1285                1290                1295
   Lys Glu Glu Phe Trp Lys Tyr Phe Asn Ser Val Ser Glu Lys His Val
               1300                1305                1310
   Thr His Ser Asp Phe Met Ser Val Leu Ser Asn Ile Asp Tyr Ile Leu
           1315                1320                1325
   Ile Lys Ala Ser Tyr Gly Gln Gly Leu Gln Gln Ser Arg Ile Ala Asn
       1330                1335                1340
   Ile Ser Met Glu Val Gly Arg Lys Ala Val Glu Leu Pro Ala Glu Gly
   1345                1350                1355                1360
   Glu Ala Ala Leu Leu Leu Glu Leu Cys Val Cys Pro Pro Gly Thr Ala
                   1365                1370                1375
   Gly His Ser Cys Gln Asp Cys Ala Pro Gly Tyr Tyr Arg Glu Lys Leu
               1380                1385                1390
   Pro Glu Ser Gly Gly Arg Gly Pro Arg Pro Leu Leu Ala Pro Cys Val
           1395                1400                1405
   Pro Cys Asn Cys Asn Asn His Ser Asp Val Cys Asp Pro Glu Thr Gly
       1410                1415                1420
   Lys Cys Leu Ser Cys Arg Asp His Thr Ser Gly Asp His Cys Glu Leu
   1425                1430                1435                1440
   Cys Ala Ser Gly Tyr Tyr Gly Lys Val Thr Gly Leu Pro Gly Asp Cys
                   1445                1450                1455
   Thr Pro Cys Thr Cys Pro His His Pro Pro Phe Ser Phe Ser Pro Thr
               1460                1465                1470
   Cys Val Val Glu Gly Asp Ser Asp Phe Arg Cys Asn Ala Cys Leu Pro
           1475                1480                1485
   Gly Tyr Glu Gly Gln Tyr Cys Glu Arg Cys Ser Ala Gly Tyr His Gly
       1490                1495                1500
   Asn Pro Arg Ala Ala Gly Gly Ser Cys Gln Thr Cys Asp Cys Asn Pro
   1505                1510                1515                1520
   Gln Gly Ser Val His Ser Asp Cys Asp Arg Ala Ser Gly Gln Cys Val
                   1525                1530                1535
   Cys Lys Pro Gly Ala Thr Gly Leu His Cys Glu Lys Cys Leu Pro Arg
               1540                1545                1550
   His Ile Leu Met Glu Ser Asp Cys Val Ser Cys Asp Asp Asp Cys Val
           1555                1560                1565
   Gly Pro Leu Leu Asn Asp Leu Asp Ser Val Gly Asp Ala Val Leu Ser
       1570                1575                1580
   Leu Asn Leu Thr Gly Val Ser Pro Ala Pro Tyr Gly Ile Leu Glu Asn
   1585                1590                1595                1600
   Leu Glu Asn Thr Thr Lys Tyr Phe Gln Arg Tyr Leu Ile Lys Glu Asn
                   1605                1610                1615
   Ala Lys Lys Ile Arg Ala Glu Ile Gln Leu Glu Gly Ile Ala Glu Gln
               1620                1625                1630
   Thr Glu Asn Leu Gln Lys Glu Leu Thr Arg Val Leu Ala Arg His Gln
           1635                1640                1645
   Lys Val Asn Ala Glu Met Glu Arg Thr Ser Asn Gly Thr Gln Ala Leu
       1650                1655                1660
   Ala Thr Phe Ile Glu Gln Leu His Ala Asn Ile Lys Glu Ile Thr Glu
   1665 1670 1675 1680
   Lys Val Ala Thr Leu Asn Gln Thr Ala Arg Lys Asp Phe Gln Pro Pro
   1685 1690 1695
   Val Ser Ala Leu Gln Ser Met His Gln Asn Ile Ser Ser Leu Leu Gly
   1700 1705 1710
   Leu Ile Lys Glu Arg Asn Phe Thr Glu Met Gln Gln Asn Ala Thr Leu
   1715 1720 1725
   Glu Leu Lys Ala Ala Lys Asp Leu Leu Ser Arg Ile Gln Lys Arg Phe
   1730 1735 1740
   Gln Lys Pro Gln Glu Lys Leu Lys Ala Leu Lys Glu Ala Asn Ser Leu
   1745 1750 1755 1760
   Leu Ser Asn His Ser Glu Lys Leu Gln Ala Ala Glu Glu Leu Leu Lys
   1765 1770 1775
   Glu Ala Gly Ser Lys Thr Gln Glu Ser Asn Leu Leu Leu Leu Leu Val
   1780 1785 1790
   Lys Ala Asn Leu Lys Glu Glu Phe Gln Glu Lys Lys Leu Arg Val Gln
   1795 1800 1805
   Glu Glu Gln Asn Val Thr Ser Glu Leu Ile Ala Lys Gly Arg Glu Trp
   1810 1815 1820
   Val Asp Ala Ala Gly Thr His Thr Ala Ala Ala Gln Asp Thr Leu Thr
   1825 1830 1835 1840
   Gln Leu Glu His His Arg Asp Glu Leu Leu Leu Trp Ala Arg Lys Ile
   1845 1850 1855
   Arg Ser His Val Asp Asp Leu Val Met Gln Met Ser Lys Arg Arg Ala
   1860 1865 1870
   Arg Asp Leu Val His Arg Ala Glu Gln His Ala Ser Glu Leu Gln Ser
   1875 1880 1885
   Arg Ala Gly Ala Leu Asp Arg Asp Leu Glu Asn Val Arg Asn Val Ser
   1890 1895 1900
   Leu Asn Ala Thr Ser Ala Ala His Val His Ser Asn Ile Gln Thr Leu
   1905 1910 1915 1920
   Thr Glu Glu Ala Glu Met Leu Ala Ala Asp Ala His Lys Thr Ala Asn
   1925 1930 1935
   Lys Thr Asp Leu Ile Ser Glu Ser Leu Ala Ser Arg Gly Lys Ala Val
   1940 1945 1950
   Leu Gln Arg Ser Ser Arg Phe Leu Lys Glu Ser Val Gly Thr Arg Arg
   1955 1960 1965
   Lys Gln Gln Gly Ile Thr Met Lys Leu Asp Glu Leu Lys Asn Leu Thr
   1970 1975 1980
   Ser Gln Phe Gln Glu Ser Val Asp Asn Ile Thr Lys Gln Ala Asn Asp
   1985 1990 1995 2000
   Ser Leu Ala Met Leu Arg Glu Ser Pro Gly Gly Met Arg Glu Lys Gly
   2005 2010 2015
   Arg Lys Ala Arg Glu Leu Ala Ala Ala Ala Asn Glu Ser Ala Val Lys
   2020 2025 2030
   Thr Leu Glu Asp Val Leu Ala Leu Ser Leu Arg Val Phe Asn Thr Ser
   2035 2040 2045
   Glu Asp Leu Ser Arg Val Asn Ala Thr Val Gln Glu Thr Asn Asp Leu
   2050 2055 2060
   Leu His Asn Ser Thr Met Thr Thr Leu Leu Ala Gly Arg Lys Met Lys
   2065 2070 2075 2080
   Asp Met Glu Met Gln Ala Asn Leu Leu Leu Asp Arg Leu Lys Pro Leu
   2085 2090 2095
   Lys Thr Leu Glu Glu Asn Leu Ser Arg Asn Leu Ser Glu Ile Lys Leu
   2100 2105 2110
   Leu Ile Ser Arg Ala Arg Lys Gln Ala Ala Ser Ile Lys Val Ala Val
   2115 2120 2125
   Ser Ala Asp Arg Asp Cys Ile Arg Ala Tyr Gln Pro Gln Thr Ser Ser
   2130 2135 2140
   Thr Asn Tyr Asn Thr Leu Ile Leu Asn Val Lys Thr Gln Glu Pro Asp
   2145 2150 2155 2160
   Asn Leu Leu Phe Tyr Leu Gly Ser Ser Ser Ser Ser Asp Phe Leu Ala
   2165 2170 2175
   Val Glu Met Arg Arg Gly Lys Val Ala Phe Leu Trp Asp Leu Gly Ser
   2180 2185 2190
   Gly Ser Thr Arg Leu Glu Phe Pro Glu Val Ser Ile Asn Asn Asn Arg
   2195 2200 2205
   Trp His Ser Ile Tyr Ile Thr Arg Phe Gly Asn Met Gly Ser Leu Ser
   2210 2215 2220
   Val Lys Glu Ala Ser Ala Ala Glu Asn Pro Pro Val Arg Thr Ser Lys
   2225 2230 2235 2240
   Ser Pro Gly Pro Ser Lys Val Leu Asp Ile Asn Asn Ser Thr Leu Met
   2245 2250 2255
   Phe Val Gly Gly Leu Gly Gly Gln Ile Lys Lys Ser Pro Ala Val Lys
   2260 2265 2270
   Val Thr His Phe Lys Gly Cys Met Gly Glu Ala Phe Leu Asn Gly Lys
   2275 2280 2285
   Ser Ile Gly Leu Trp Asn Tyr Ile Glu Arg Glu Gly Lys Cys Asn Gly
   2290 2295 2300
   Cys Phe Gly Ser Ser Gln Asn Glu Asp Ser Ser Phe His Phe Asp Gly
   2305 2310 2315 2320
   Ser Gly Tyr Ala Met Val Glu Lys Thr Leu Arg Pro Thr Val Thr Gln
   2325 2330 2335
   Ile Val Ile Leu Phe Ser Thr Phe Ser Pro Asn Gly Leu Leu Phe Tyr
   2340 2345 2350
   Leu Ala Ser Asn Gly Thr Lys Asp Phe Leu Ser Ile Glu Leu Val Arg
   2355 2360 2365
   Gly Arg Val Lys Val Met Val Asp Leu Gly Ser Gly Pro Leu Thr Leu
   2370 2375 2380
   Met Thr Asp Arg Arg Tyr Asn Asn Gly Thr Trp Tyr Lys Ile Ala Phe
   2385 2390 2395 2400
   Gln Arg Asn Arg Lys Gln Gly Leu Leu Ala Val Phe Asp Ala Tyr Asp
   2405 2410 2415
   Thr Ser Asp Lys Glu Thr Lys Gln Gly Glu Thr Pro Gly Ala Ala Ser
   2420 2425 2430
   Asp Leu Asn Arg Leu Glu Lys Asp Leu Ile Tyr Val Gly Gly Leu Pro
   2435 2440 2445
   His Ser Lys Ala Val Arg Lys Gly Val Ser Ser Arg Ser Tyr Val Gly
   2450 2455 2460
   Cys Ile Lys Asn Leu Glu Ile Ser Arg Ser Thr Phe Asp Leu Leu Arg
   2465 2470 2475 2480
   Asn Ser Tyr Gly Val Arg Lys Gly Cys Ala Leu Glu Pro Ile Gln Ser
   2485 2490 2495
   Val Ser Phe Leu Arg Gly Gly Tyr Val Glu Met Pro Pro Lys Ser Leu
   2500 2505 2510
   Ser Pro Glu Ser Ser Leu Leu Ala Thr Phe Ala Thr Lys Asn Ser Ser
   2515 2520 2525
   Gly Ile Leu Leu Val Ala Leu Gly Lys Asp Ala Glu Glu Ala Gly Gly
   2530 2535 2540
   Ala Gln Ala His Val Pro Phe Phe Ser Ile Met Leu Leu Glu Gly Arg
   2545 2550 2555 2560
   Ile Glu Val His Val Asn Ser Gly Asp Gly Thr Ser Leu Arg Lys Ala
   2565 2570 2575
   Leu Leu His Ala Pro Thr Gly Ser Tyr Ser Asp Gly Gln Glu His Ser
   2580 2585 2590
   Ile Ser Leu Val Arg Asn Arg Arg Val Ile Thr Ile Gln Val Asp Glu
   2595 2600 2605
   Asn Ser Pro Val Glu Met Lys Leu Gly Pro Leu Thr Glu Gly Lys Thr
   2610 2615 2620
   Ile Asp Ile Ser Asn Leu Tyr Ile Gly Gly Leu Pro Glu Asp Lys Ala
   2625 2630 2635 2640
   Thr Pro Met Leu Lys Met Arg Thr Ser Phe His Gly Cys Ile Lys Asn
   2645 2650 2655
   Val Val Leu Asp Ala Gln Leu Leu Asp Phe Thr His Ala Thr Gly Ser
               2660                2665                2670
   Glu Gln Val Glu Leu Asp Thr Cys Leu Leu Ala Glu Glu Pro Met Gln
           2675                2680                2685
   Ser Leu His Arg Glu His Gly Glu Leu Pro Pro Glu Pro Pro Thr Leu
       2690                2695                2700
   Pro Gln Pro Glu Leu Cys Ala Val Asp Thr Ala Pro Gly Tyr Val Ala
   2705                2710                2715                2720
   Gly Ala His Gln Phe Gly Leu Ser Gln Asn Ser His Leu Val Leu Pro
                   2725                2730                2735
   Leu Asn Gln Ser Asp Val Arg Lys Arg Leu Gln Val Gln Leu Ser Ile
               2740                2745                2750
   Arg Thr Phe Ala Ser Ser Gly Leu Ile Tyr Tyr Val Ala His Gln Asn
           2755                2760                2765
   Gln Met Asp Tyr Ala Thr Leu Gln Leu Gln Glu Gly Arg Leu His Phe
       2770                2775                2780
   Met Phe Asp Leu Gly Lys Gly Arg Thr Lys Val Ser His Pro Ala Leu
   2785                2790                2795                2800
   Leu Ser Asp Gly Lys Trp His Thr Val Lys Thr Glu Tyr Ile Lys Arg
                   2805                2810                2815
   Lys Ala Phe Met Thr Val Asp Gly Gln Glu Ser Pro Ser Val Thr Val
               2820                2825                2830
   Val Gly Asn Ala Thr Thr Leu Asp Val Glu Arg Lys Leu Tyr Leu Gly
           2835                2840                2845
   Gly Leu Pro Ser His Tyr Arg Ala Arg Asn Ile Gly Thr Ile Thr His
       2850                2855                2860
   Ser Ile Pro Ala Cys Ile Gly Glu Ile Met Val Asn Gly Gln Gln Leu
   2865                2870                2875                2880
   Asp Lys Asp Arg Pro Leu Ser Ala Ser Ala Val Asp Arg Cys Tyr Val
                   2885                2890                2895
   Val Ala Gln Glu Gly Thr Phe Phe Glu Gly Ser Gly Tyr Ala Ala Leu
               2900                2905                2910
   Val Lys Glu Gly Tyr Lys Val Arg Leu Asp Leu Asn Ile Thr Leu Glu
           2915                2920                2925
   Phe Arg Thr Thr Ser Lys Asn Gly Val Leu Leu Gly Ile Ser Ser Ala
       2930                2935                2940
   Lys Val Asp Ala Ile Gly Leu Glu Ile Val Asp Gly Lys Val Leu Phe
   2945                2950                2955                2960
   His Val Asn Asn Gly Ala Gly Arg Ile Thr Ala Thr Tyr Gln Pro Arg
                   2965                2970                2975
   Ala Ala Arg Ala Leu Cys Asp Gly Lys Trp His Thr Leu Gln Ala His
               2980                2985                2990
   Lys Ser Lys His Arg Ile Val Leu Thr Val Asp Gly Asn Ser Val Arg
           2995                3000                3005
   Ala Glu Ser Pro His Thr His Ser Thr Ser Ala Asp Thr Asn Asp Pro
       3010                3015                3020
   Ile Tyr Val Gly Gly Tyr Pro Ala His Ile Lys Gln Asn Cys Leu Ser
   3025                3030                3035                3040
   Ser Arg Ala Ser Phe Arg Gly Cys Val Arg Asn Leu Arg Leu Ser Arg
                   3045                3050                3055
   Gly Ser Gln Val Gln Ser Leu Asp Leu Ser Arg Ala Phe Asp Leu Gln
               3060                3065                3070
   Gly Val Phe Pro His Ser Cys Pro Gly Pro Glu Pro
           3075                3080
<210> 3
   <211> 30
   <212> DNA
   <213> Artificial Sequence
<220>
   <223> Olgionucleotide Primer
<400> 3
   cgggatccta gagactgcat ccgcgcctat 30
<210> 4
   <211> 30
   <212> DNA
   <213> Artificial Sequence
<220>
   <223> Olgionucleotide Primer
<400> 4
   cccaagcttt actatctgcg tcacggtggg 30
<210> 5
   <211> 33
   <212> DNA
   <213> Artificial Sequence
<220>
   <223> Olgionucleotide Primer
<400> 5
   cgggatcctc agatagtaat tctcttcagc acc 33
<210> 6
   <211> 30
   <212> DNA
   <213> Artificial Sequence
<220>
   <223> Olgionucleotide Primer
<400> 6
   cccaagcttg gatgactcag gtgagagaga 30
<210> 7
   <211> 28
   <212> DNA
   <213> Artificial Sequence
<220>
   <223> Olgionucleotide Primer
<400> 7
   cgggatcctc tgctggccac attcgcca 28
<210> 8
   <211> 28
   <212> DNA
   <213> Artificial Sequence
<220>
   <223> Olgionucleotide Primer
<400> 8
   cccaagcttc ctcttccgga catcagac 28
<210> 9
   <211> 30
   <212> DNA
   <213> Artificial Sequence
<220>
   <223> Olgionucleotide Primer
<400> 9
   cgggatcctc tccaggtgca gctgagcatt 30
<210> 10
   <211> 30
   <212> DNA
   <213> Artificial Sequence
<220>
   <223> Olgionucleotide Primer
<400> 10
   cccaagcttc tgttggccat taaccatgat 30
<210> 11
   <211> 30
   <212> DNA
   <213> Artificial Sequence
<220>
   <223> Olgionucleotide Primer
<400> 11
   cgggatcctc tggataaaga caggcccttg 30
<210> 12
   <211> 32
   <212> DNA
   <213> Artificial Sequence
<220>
   <223> Olgionucleotide Primer
<400> 12
   cccaagcttg ggctcaggcc cggggcagga at 32

## Description

### BACKGROUND OF THE INVENTION

Basement membranes are thin layers of specialized extracellular matrix that provide supporting structure on which epithelial and endothelial cells grow, and that surround muscle or fat (Paulsson, M., 1992, *Crit*. Rev. *Biochem. Mol. Biol.* 27:93-127). Basement membranes are always associated with cells, and it has been well documented that basement membranes not only provide mechanical support, but also influence cellular behavior such as differentiation and proliferation. Vascular basement membranes are composed of macromolecules such as collagen, laminin, heparan sulfate proteoglycans, fibronectin and nidogen (also called entactin) (Timpl, R, 1996, *Curr. Opin. Cell. Biol*. 8:618-624).

Angiogenesis is the process of formation of new blood vessels from pre-existing ones (Madri, J.A. *et al*., 1986, *J. Histochem. Cytochem*. 34:85-91; Folkman, J., 1972, *Ann. Surg*. 175:409-416). Angiogenesis is a complex process, and requires sprouting and migration of endothelial cells, proliferation of those cells, and their differentiation into tube-like structures and the production of a basement membrane matrix around the developing blood vessel. Additionally angiogenesis is a process critical for normal physiological events such as wound repair and endometrium remodeling (Folkman, J. *et al*., 1995, *J*. *Biol. Chem*. 267:10931-10934). It is now well documented that angiogenesis is required for metastasis and growth of solid tumors beyond a few mm³ in size (Folkman, J., 1972, *Ann. Surg*. 175:409-416; Follonan, J., 1995, *Nat. Med.* 1:27-31). Expansion of tumor mass occurs not only by perfusion of blood through the tumor, but also by paracrine stimulation of tumor cells by several growth factors and matrix proteins produced by the new capillary endothelium (Folkman, J., 1995, *Nat. Med*. 1:27-31). Recently, a number of inhibitors have been identified, namely angiostatin (O'Reilly, M.S. *et al.,* 1994, *Cell* 79:315-28), endostatin (O'Reilly, MS. *et al*., 1997, *Cell* 88:277-85), restin (Ramchandran, R. *et al*., 1999, *Biochem. Biophys. Res. Commun.* 255:735-9), Arrestin (Colorado, P.C. *et al*, 2000, *Cancer Res.* 60:2520-6), Canstatin (Kamphaus, G.D. *et al*., 2000, *J. Biol Chem*. 275:1209-15) and Tumstatin (Maeshima, *Y. et al*., 2000, *J Biol. Chem.* 275:21340-8; Maeshinia, Y. *et al*., 2000, *J Biol. Chem.* 275:23745-50) and pigment epithelium-derived factor (PEDF) (Dawson, D.W. *et al*., 1999, *Science* 285:245-8).

### SUMMARY OF THE INVENTION

The present invention relates to a protein that consists of the amino acids 2132 to 2338 or of amino acids 2132 to 3084 of SEQ ID No:2, or a fragment thereof comprising at least 25 continuous amino acids of said protein, or a variant of said protein having at least 70% sequence identity therewith; wherein said protein, variant or fragment has anti-angiogenic activity; with the proviso that the variant does not have the sequence shown as SEQ ID number 113 of WO 00/56754.

The present invention includes a protein (designated herein as "Matin") that consists of the G1 domain of the α1 chain of laminin. The laminin is preferably mammalian.

The present invention includes proteins, fragments and variants from the other G domains of laminin that are within the scope of Claim 1.

Preferred variants of the present invention have at least 80% or at least 90% sequence identity with amino acids 2132 to 2338 or amino acids 2132 to 3084 of SEQ ID No:2.

The protein, fragment, or variant may be in monomeric or in multimeric form (e.g. dimeric or trimeric).

Also within the scope of the present invention is a chimeric protein (also known as a fusion protein) comprising the protein, fragment or variant thereof; with the proviso that it is not a fusion protein of glutathione-S-transferase and the G1, G2, G3, G4 or G5 domain of the human laminin α3 chain.

The chimeric protein preferably comprises at least one protein molecule selected from the group consisting of: Vascostatin or fragments thereof, arrestin or fragments thereof, canstatin or fragments thereof, tumstatin or fragments thereof, endostatin or fragments thereof, angiostatin or fragments thereof, restin or fragments thereof, apomigren or fragments thereof, or other anti-angiogenic proteins or fragments thereof.

(Vascostatin comprises the C-terminal domain of nidogen, and has anti-angiogenic properties, Vacostatin is described in international patent application number PCT/US01/40382, entitled "Anti-angiogenic and Anti-tumor Properties of Vascostatin and Other Nidogen Domains", by Raghuram Kalluri, filed on March 28, 2001.)

The invention also features a polynucleotide encoding the protein, fragment, variant or chimeric protein of the present invention.

The polynucleotide may be operably linked to an expression control sequence. The polynucleotide may be isolated.

A polynucleotide of the present invention can be produced by a process comprising
(a) preparing one or more polynucleotide probes that hybridize under conditions of moderate stringency or high stringency to a polynucleotide of the prsent invention;
(b) hybridizing said probe(s) to mammalian DNA; and
(c) isolating the DNA polynucleotide detected with the probe(s); wherein the nucleotide sequence of the isolated polynucleotide corresponds to the nucleotide sequence of the polynucleotide of the present invention.

The polynucleotide may be a subsequence of SEQ ID No 1. For example, it may correspond to the sequence that extends from about nucleotide 6442 to about nucleotide 7062 thereof; from about nucleotide 7054 to about nucleotide 7599 thereof, from about nucleotide 7600 to about nucleotide 8283 thereof; from about nucleotide 8284 to about nucleotide 8685 thereof; or from about nucleotide 8686 to about nucleotide 9300 thereof.

A polynucleotide of the present invention can be used (with or without operable linkage to an expression control sequence) to transform a host cell. The host cell may be selected from the group comprising bacterial, yeast, mammalian, insect or plant cells; with the proviso that the host cell is not part of the human embryo. The present invention therefore includes a host cell, such as bacterial, yeast, mammalian, insect or plant cell, transformed with a polynucleotide of the present invention; with the proviso that the host cell is not part of a human embryo.

The polynucleotide or host cell can be used in medicine.

The present invention includes the use of the polynucleotide in the preparation of a therapeutic mammalian cell for treating a mammal having a disorder as previously described; wherein the preparation involves treating a mammalian cell *in vitro* to insert therein the polynucleotide; with the proviso that said cell is not part of a human embryo.

The mammalian cell may be chosen from the group consisting of: blood cells, TIL cells, bone marrow cells, vascular cells, tumor cells, liver cells, muscle cells, fibroblast cells. The polynucleotide may be inserted into the cells by a viral vector.

The cell may allow transient expression.

Mammalian host cells may be used in a process for providing a mammal with an anti-angiogenic protein, where the mammalian cells express *in vivo* within the mammal a therapeutically effective amount of the anti-angiogenic protein in an amount sufficient to inhibit angiogenic activity in the mammal.

The present invention also includes a pharmaceutical or contraceptive composition comprising a protein, fragment, variant, monomer, multimer, or chimeric protein of the present invention.

The composition may comprise a pharmaceutically compatible carrier.

It may comprise at least one protein molecule selected from the group consisting of: vascostatin or fragments thereof, arrestin or fragments thereof, canstatin or fragments thereof, tumstatin or fragments thereof, endostatin or fragments thereof, angiostatin or fragments thereof, restin or fragments thereof, apomigren or fragments thereof, or other anti-angiogenic proteins, or fragments thereof.

The present invention further includes the fragment, variant, monomer, multimer, chimeric protein, and composition of the present invention; for use in medicine.

The fragment, variant, monomer, multimer, chimeric protein, and composition can be used to contact mammalian tissue and can therefore be used in the treatment of mammals.

The protein, fragment, variant, monomer, multimer, chimeric protein, or composition can be used in the preparation of a medicament for treating a disorder involving angiogenesis.

The disorder may be cancer. It may involve tumor growth.

The present invention includes the use of a protein, fragment, variant, monomer, multimer, chimeric protein or composition of the present invention in the preparation of a medicament for treating a disorder involving endothelial cell proliferation.

The present invention further includes the use of the protein, fragment, variant, monomer, multimer, chimeric protein or composition in the preparation of a medicament for treating a disorder selected from the group comprising angiogenesis-dependent cancers, benign tumors, rheumatoid arthritis, diabetic retinopathy, fibrosis, psoriasis, ocular angiogenesis diseases, Osler-Webber Syndrome, myocardial angiogenesis, plaque neovascularization, telangiectasia, hemopheliac joints, angiofibroma, wound granulation, intestinal adhesions, atherosclerosis, scleroderma, hypertrophic scars, cat scratch disease, *Helicobacter pylori* ulcers, dialysis graft vascular access stenosis and obesity.

The protein, fragment, variant, monomer, multimer, chimeric protein or composition may be combined with a radiotherapeutic, chemotherapeutic or immunotherapeutic agent; for simultaneous, sequential or separate use in anti-cancer therapy.

In addition to the various aspects discussed above, the present invention also includes the protein, fragment, variant, monomer, multimer, chimeric protein, polynucleotide or host cell in isolated form.

A host cell of the present invention can be used to produce a protein, fragment, variant, monomer, multimer, or chimeric protein of the present invention by the steps of
a) growing the host cell in culture and
b) purifying a protein, fragment, variant, monomer, multimer, or chimeric protein encoded by the polynucleotide within the host cell from the culture.

For example, the method may comprise: (a) growing a culture of a host cell transformed with the polynucleotide of nucleotide 6442 to nucleotide 7062 of SEQ ID NO:1, where the host cell is selected from the group comprising bacterial, yeast, mammalian, insect or plant cells; and (b) purifying the protein from the culture; so that an anti-angiogenic polypeptide is produced. Alternatively, for example, the polynucleotide can also correspond to about nucleotide 7054 to about nucleotide 7599, nucleotide 7600 to about nucleotide 78283, nucleotide 8284 to about nucleotide 8685, or nucleotide 8686 to about nucleotide 9300.

### BRIEF DESCRIPTION OF THE DRAWINGS

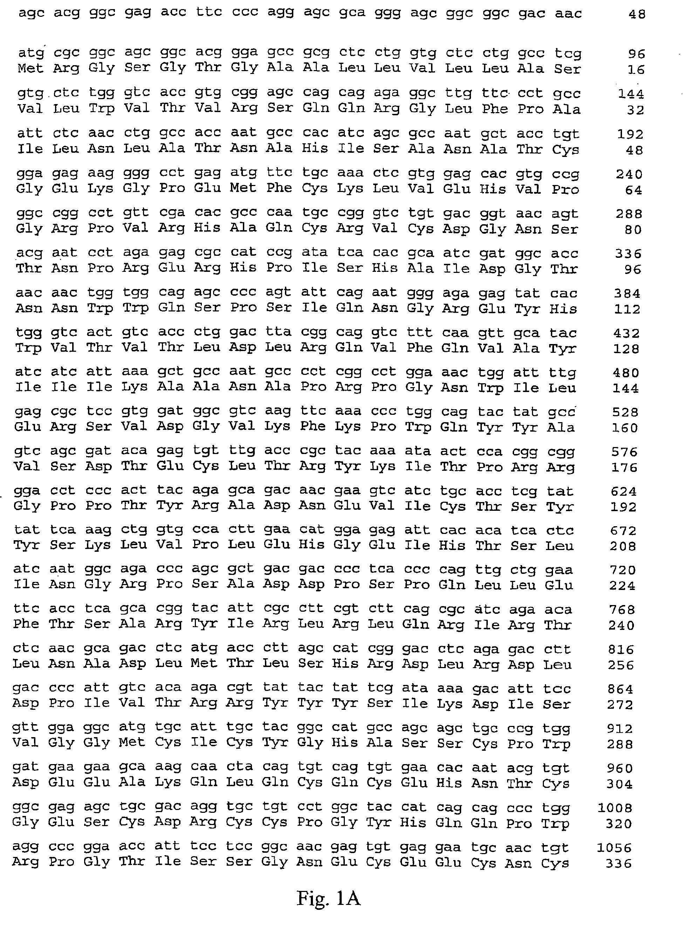

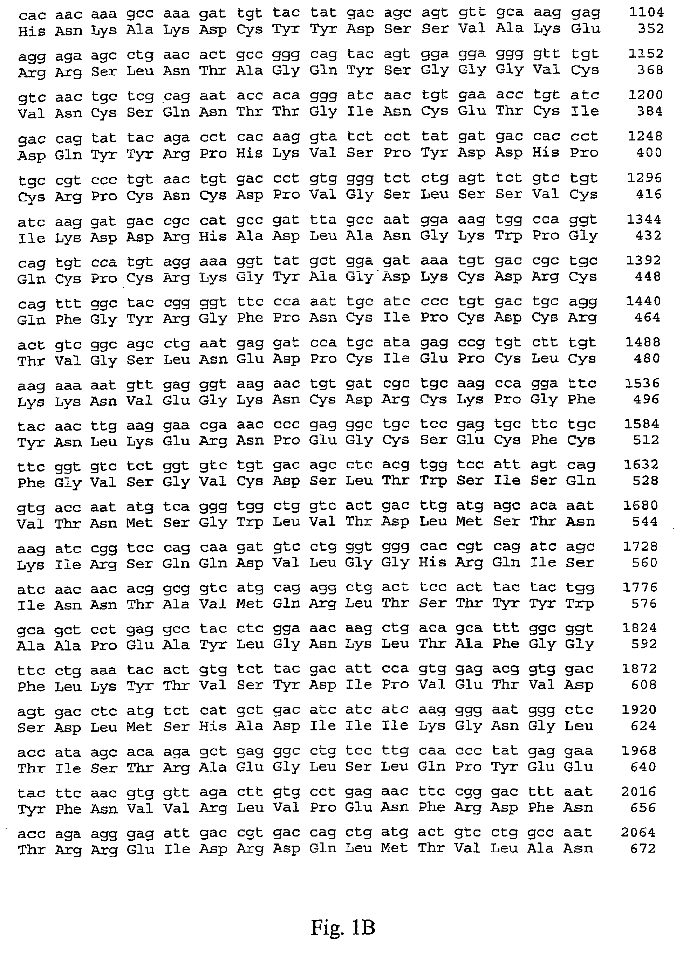

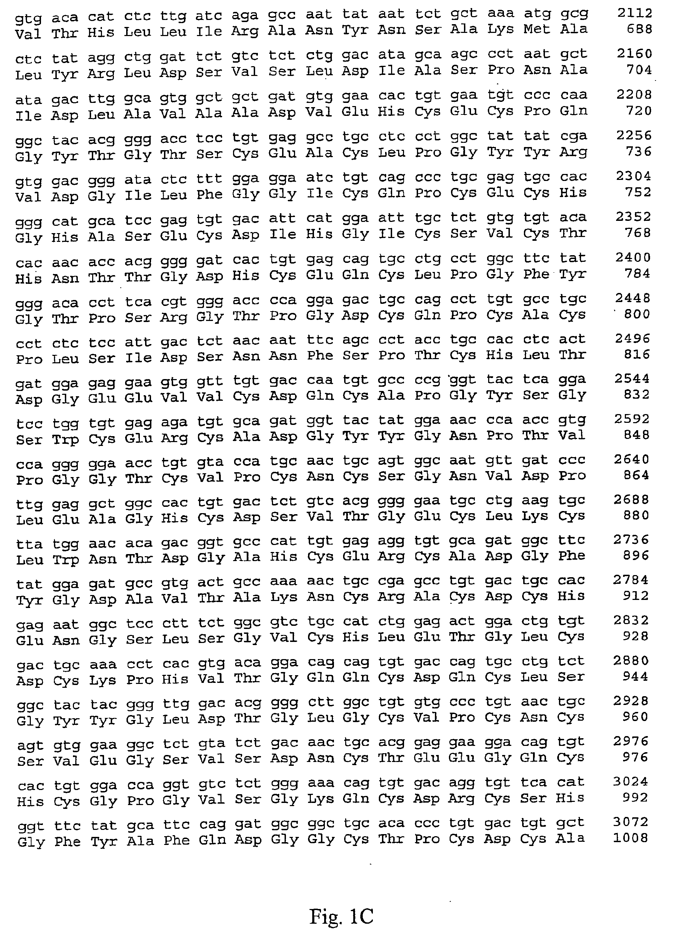

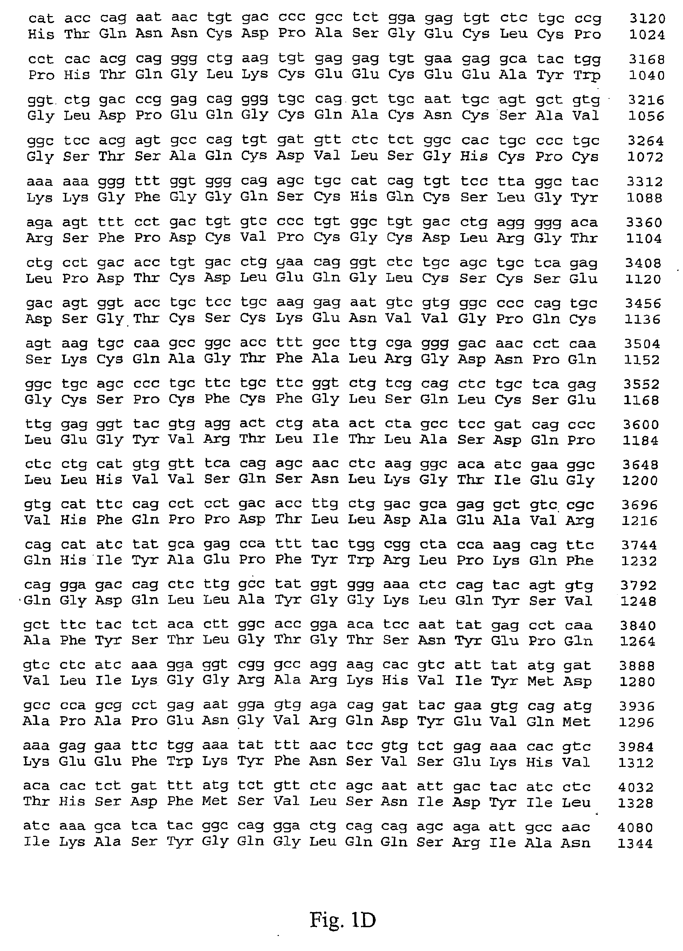

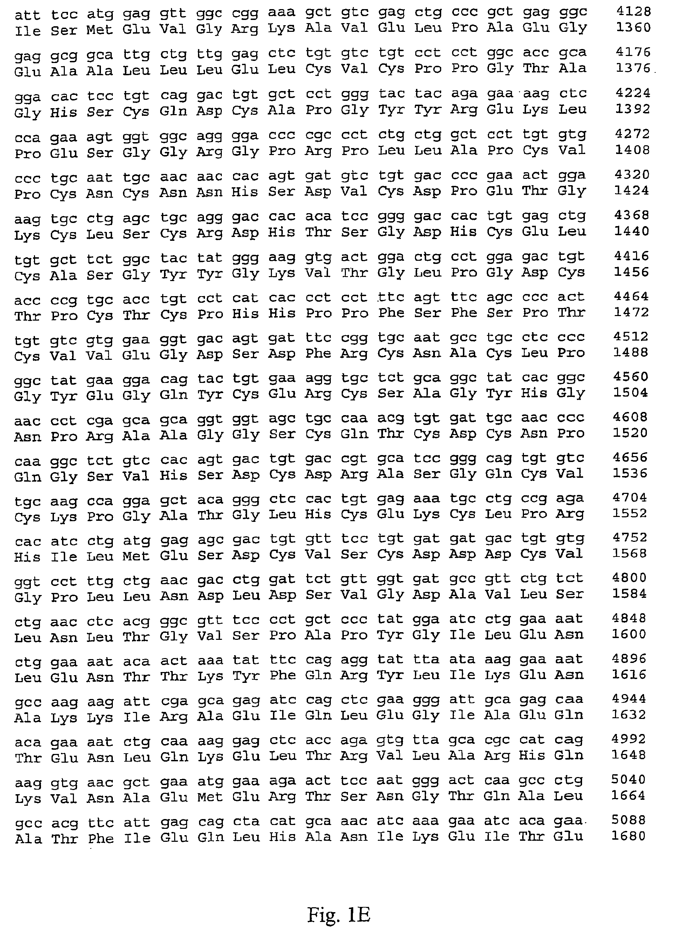

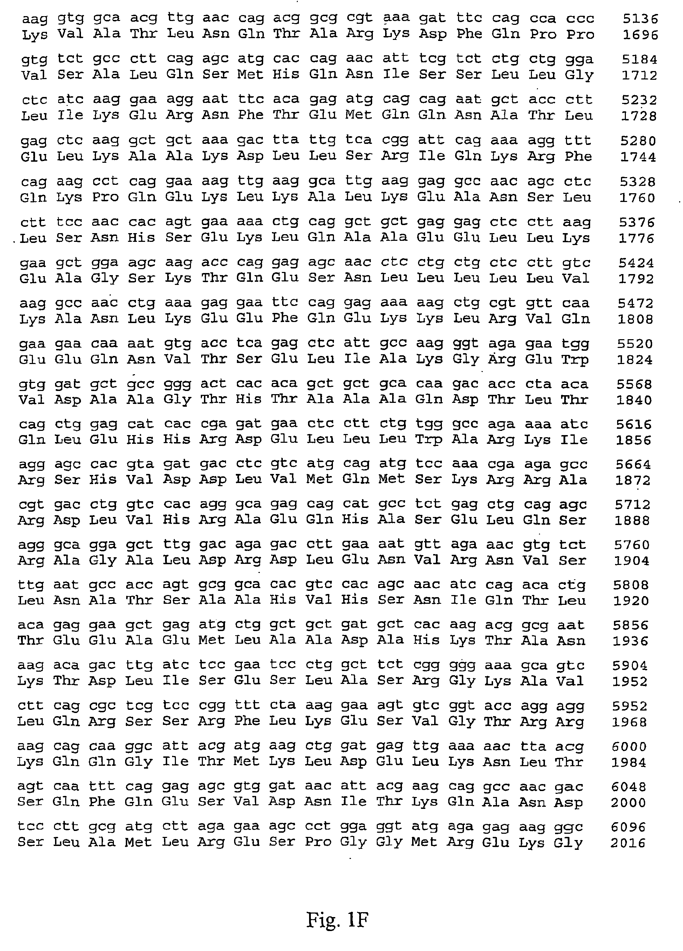

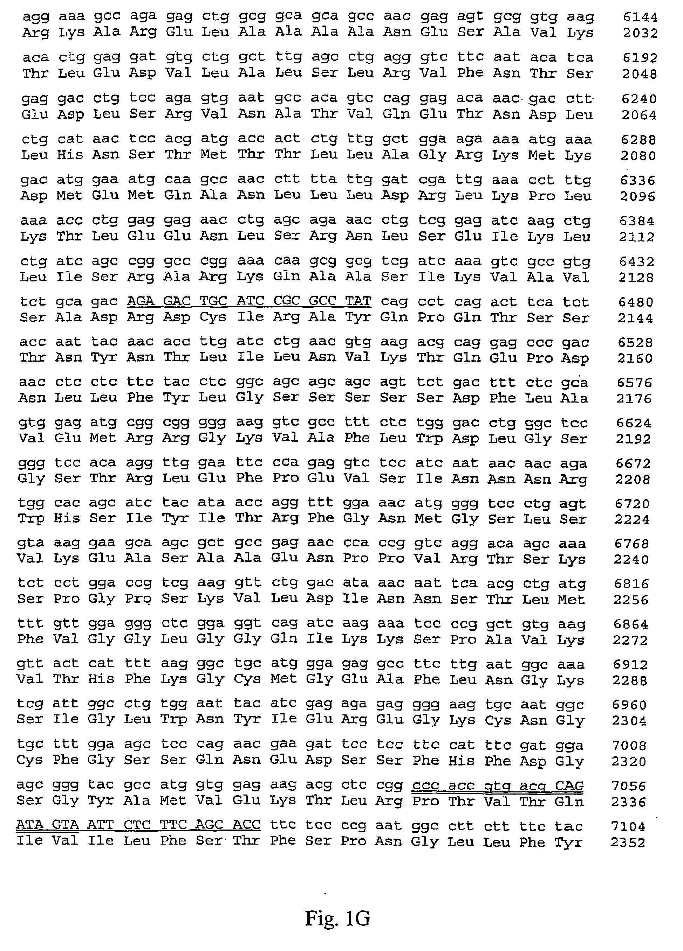

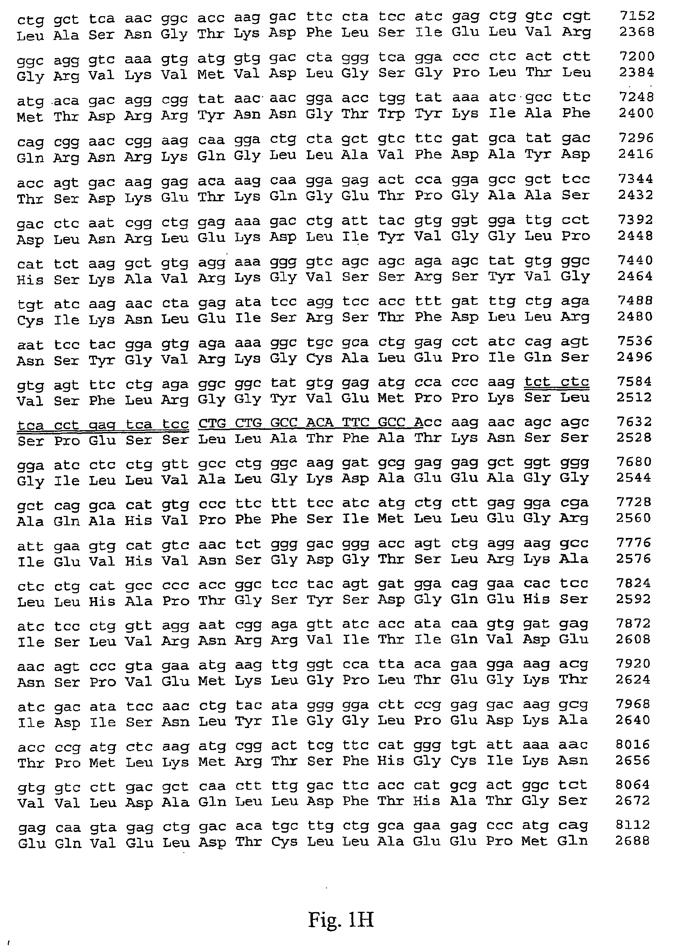

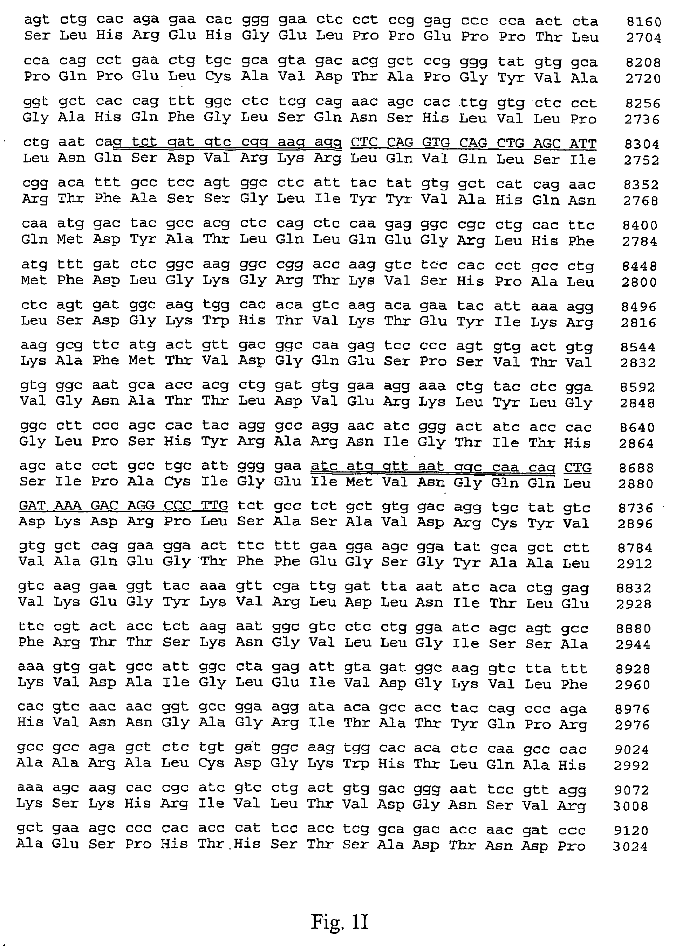

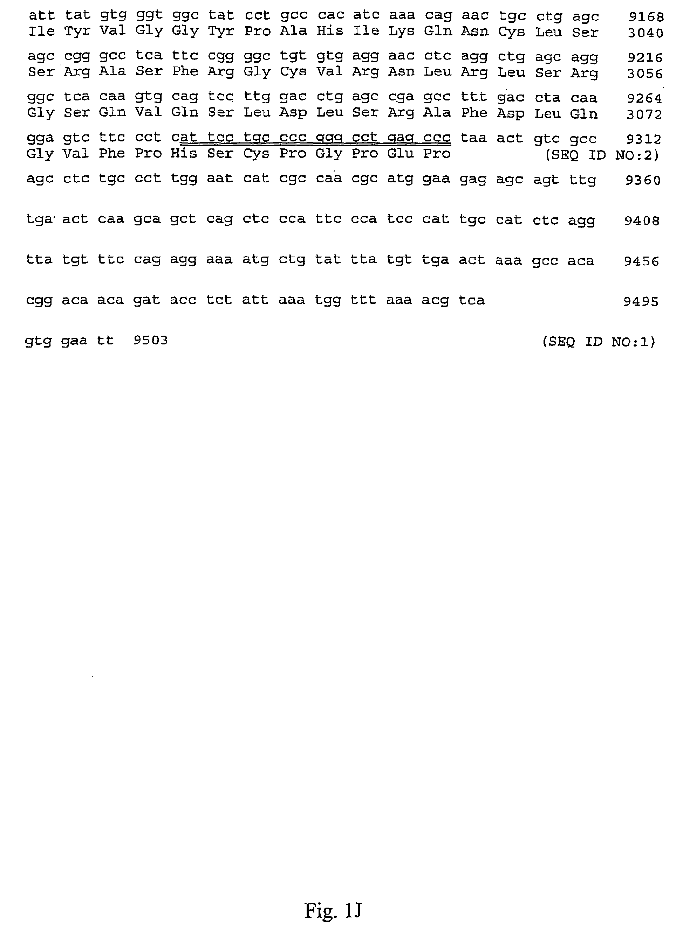

Figs. 1A, 1B, 1C, 1D, 1E, 1F, 1G, 1H, 1I and 1J are a diagram depicting the nucleotide (SEQ ID NO:1) and amino acid (SEQ ID NO:2) sequences of the five globular domains (G1, G2, G3, G4 and G5) of the α1 chain of laminin-1 (GenBank Acc.No. NM_008480). Forward primers are depicted in bold face type with single underlining, and reverse primers are shown with double underlining. Globular domain 1 (G1) extends from about nucleotide 6442 to about nucleotide 7062, and about amino acid 2132 to about amino acid 2338. The forward primer used for the G1 domain was 5'-CGG-GAT-CCT-AGA-GAC-TGC-ATC-CGC-GCC-TAT-3' (SEQ ID NO:3), and the reverse primer was 5'-CCC-AAG-CTT-TAC-TAT-CTG-CGT-CAC-GGT-GGG-3' (SEQ ID NO:4). Globular domain 2 (G2) extends from about nucleotide 7054 to about nucleotide 7599, and about amino acid 2336 to about amino acid 2517. The forward primer used for the G2 domain was 5'-CGG-GAT-CCT-CAG-ATA-GTA-ATT-CTC-TTC-AGC-ACC-3' (SEQ ID NO:5), and the reverse primer was 5'-CCC-AAG-CTT-GGA-TGA-CTC-AGG-TGA-GAG-AGA-3' (SEQ ID NO:6). Globular domain 3 (G3) extends from about nucleotide 7600 to about nucleotide 8283, and about amino acid 2518 to about amino acid 2745. The forward primer used for the G3 domain was 5'-CGG-GAT-CCT-CTG-CTG-GCC-ACA-TTC-GCC-A-3' (SEQ ID NO:7), and the reverse primer was 5'-CCC-AAG-CTT-CCT-CTT-CCG-GAC-ATC-AGA-C-3' (SEQ ID NO:8). Globular domain 4 (G4) extends from about nucleotide 8284 to about nucleotide 8685, and about amino acid 2746 to about amino acid 2879. The forward primer used for the G4 domain was 5'-CGG-GAT-CCT-CTC-CAG-GTG-CAG-CTG-AGC-ATT-3' (SEQ ID NO:9), and the reverse primer was 5'-CCC-AAG-CTT-CTG-TTG-GCC-ATT-AAC-CAT-GAT-3' (SEQ ID NO:10). Globular domain 5 (G5) extends from about nucleotide 8686 to about nucleotide 9300, and about amino acid 288.0 to about amino acid 3084. The forward primer used for the G5 domain was 5'-CGG-GAT-CCT-CTG GAT-AAA-GAC-AGG-CCC-TTG-3' (SEQ ID NO:11), and the reverse primer was 5'-CCC-AAG-CTT-GGG-CTC-AGG-CCC-GGG-GCA-GGA-AT-3' (SEQ ID NO:12). Underlined portions of the above primers correspond to the laminin sequence.
Fig. 2 is a schematic diagram representing the Matin cloning vector pET22b(+). Forward (SEQ ID NO:3) and reverse (SEQ ID NO:4) primers and site into which Matin was cloned are indicated.
Figs. 3A and 3B are histograms showing the effect of varying concentrations of Matin (x-axis) on proliferation of endothelial (C-PAE) cells (Fig. 3A) and non-endothelial (PC-3) cells (Fig. 3B). Proliferation was measured as a function of methylene blue staining.
Fig. 4 is a plot showing annexin V fluorescence for cells treated with Matin as compared to controls.
Figs. 5A and 5B are a pair of bar charts showing caspase-3 activity in Matin-treated CPAE cells (Fig. 5A) as compared to PC-3 cells (Fig. 5B).
Figs. 6A and 6B are a pair of histograms showing cell viability at increasing concentrations of Matin (x-axis) as a function of OD₅₉₀ (y-axis) in an MTT apoptosis assay for CPAE cells (Fig. 6A) as compared to PC-3 cells (Fig. 6B). Each point represents the mean +/- the standard error of the mean for triplicate wells.
Fig. 7 is a line graph showing the effect on tumor size (mm³, y-axis) against days of treatment (x-axis) with 20 mg/ml Matin (■) versus controls (20 mg/ml nephrin (○) and PBS (□).

### DETAILED DESCRIPTION OF THE INVENTION

A wide variety of diseases are the result of undesirable angiogenesis. Put another way, many diseases and undesirable conditions could be prevented or alleviated if it were possible to stop the growth and extension of capillary blood vessels under some conditions, at certain times, or in particular tissues.

The formation of new capillaries from pre-existing vessels, angiogenesis, is essential for the process of tumor growth and metastasis (Folkman, J. *et al*., 1992, *J. Biol. Chem*. 267:10931-10934; Folkman, J., 1995, *Nat. Med*. 1:27-31; Hanahan, D. *et al*., 1996, *Cell* 86:353-364). Human and animal tumors are not vascularized at the beginning, however for a tumor to grow beyond few mm³, it might vascularize (Folkman, J., 1995, *Nat. Med.* 1:27-31; Hanahan, D. *et al*., 1996, *Cell* 86:353-364). The switch to an angiogenic phenotype requires both upregulation of angiogenic stimulators and downregulation of angiogenesis inhibitors (Folkman, J., 1995, *Nat. Med*. 1:27-31). Vascular endothelial growth factor (VEGF) and basic fibroblast growth factor (bFGF) are the most commonly expressed angiogenic factors in tumors. Vascularized tumors may overexpress one or more of these angiogenic factors which can synergistically promote tumor growth. Inhibition of a single angiogenic factor such as VEGF with a receptor antagonist may not be enough to arrest tumor growth. A number of angiogenesis inhibitors have been recently identified, and certain factors such as IFN-α, platelet-factor-4 (Maione, T.E. *et al*., 1990, *Science* 247:77-79) and PEX (Brooks, P.C. *et al*., 1998, *Cell* 92:391-400) are not endogenously associated with tumor cells, whereas angiostatin (O'Reilly, M.S. *et al*., 1994, *Cell* 79:315-328) and endostatin (O'Reilly, M.S. *et al*., 1997, *Cell* 88:277-285) are tumor associated angiogenesis inhibitors generated by tumor tissue itself. Although treatment of tumor growth and metastasis with these endogenous angiogenesis inhibitors is very effective and an attractive idea, some potential problems associated with anti-angiogenic therapies must be considered. Delayed toxicity induced by chronic anti-angiogenic therapy as well as the possibility of impaired wound healing and reproductive angiogenesis occurring during treatment are to be considered seriously.

In the present invention, a protein, and fragments, analogs, derivatives, homologs and mutants thereof with anti-angiogenic properties are described, along with methods of use of this protein, analogs, derivatives, homologs and mutants to inhibit angiogenesis-mediated proliferative diseases. The protein comprises the G1 domain of the α1 chain of laminin, and is called "Matin." This protein is about 30 kDa, and inhibits endothelial cell proliferation.

Laminin is the most abundant noncollagenous protein found in basement membranes. It was initially purified from mouse EHS (Engelbreth-Holm-Swarm) tumors and the mouse embryonal carcinoma cell line M1536-B3. These oncogenic sources produce large amounts of easily extractable basement membrane-like substance, and most early research into components of the basement membrane used these tumor lines as sources, rather than naturally-occurring basement membranes. The patterns of gene expression are known to be different, however, between oncogenic and naturally-occurring tissues.

Laminin is a multidomain protein (Paulsson, M., 1992, *Grit. Rev. Biochem. Mol. Biol*. 27:93-127), with three distinct polypeptide chains, α, β1 and β2, connected into a cross shape by disulfide bonds. The N-terminal portion of each chain, containing domains III through VI, each forms one arm of the cross, and the C-terminal portions (containing domains I and II) of all three chains are joined together by disulfide bonds into the fourth arm. Put another way, the N-terminal half of the α chain makes up the vertical arms of the cross, while the N-terminal half of the β1 and β2 chains make up the left and right arms. The C-terminal halves of all three chains join together to form the lower vertical arm of the cross. The G domain only exists at the C-terminal end of the α chain, not on either of the β chains. The G domain is subdivided into five subdomains, G1 through G5. Merosin, an isoform of laminin, was found to share some amino acid identity with the C-terminus of the α chain of mouse laminin, and the general domain structure is conserved between the two.

Matin can be obtained from a variety of sources. Such sources include, but are not limited to, P19137 (LAMININ ALPHA-1 CHAIN PRECURSOR (LAMININ A CHAIN)), MMMSA (laminin alpha-1 chain precursor - mouse) and AAA39410 (laminin A chain [Mus musculus]). Human laminin has a slightly lower sequence identity with SEQ ID NO:2, *e.g*., around 83%. Such sequences are also useful for obtaining Matin, and include, but are not limited to, P25391 (LAMININ ALPHA-1 CHAIN PRECURSOR (LAMININ A CHAIN)), S14458 (laminin alpha-1 chain precursor - human) and CAA41418 (laminin A chain [Homo sapiens]). Other sequences have a lower identity with SEQ ID NO:2, but may still be useful sources of anti-angiogenic Matin. These may include, but are not necessarily limited to, PX0082 (laminin, M chain - human (fragment)), MMHUMH (laminin alpha-2 chain - human (fragment)), AAB33989 (laminin M chain, merosin=basement membrane protein {G-domain} [human, placenta, Peptide Partial, 1751 aa] [Homo sapiens]), AAA63215 (merosin [Homo sapiens]), AAB18388 (laminin alpha 2 chain [Homo sapiens]), NP_000417 (laminin alpha 2 subunit precursor; Laminin, alpha-2 (merosin) [Homo sapiens]), P24043 (LAMININ ALPHA-2 CHAIN PRECURSOR (LAMININ M CHAIN) (MEROSIN HEAVY CHAIN)), CAA81394 (laminin M chain (merosin) [Homo sapiens]), XP_011387 (laminin alpha 2 subunit precursor [Homo sapiens]), Q60675 (LAMININ ALPHA-2 CHAIN PRECURSOR (LAMININ M CHAIN) (MEROSIN HEAVY CHAIN)), S53868 (laminin alpha-2 chain precursor - mouse), AAC52165 (laminin-2 alpha2 chain precursor [Mus musculus]).

Interestingly, the various globular domains themselves possess varying levels of sequence identity with each other. This is shown in Table 1, below.

**Table 1. Percent sequence identity of the globular domains of the mouse laminin α chain.**

| | G1 | G2 | G3 | G4 | G5 |
|---|---|---|---|---|---|
| G1 | | | | | |
| G2 | 31 | | | | |
| G3 | - | 27 | | | |
| G4 | 24 | 25 | - | | |
| G5 | 23 | 31 | 27 | 25 | |

Polynucleotides encoding Matin can also be obtained from a variety of sources. For instance, other mouse laminin α chain globular domains (*e.g.,*) generally possess greater than 90% sequence identity with SEQ ID NO:1, and include, but are not limited to, J04064 (Mus musculus laminin A chain mRNA, complete cds) and X58531 (Human LAMA mRNA for laminin A chain, partial cds).

As disclosed herein, Matin can be can be produced in *E*. *coli* using a bacterial expression plasmid, such as pET22b, which is capable of periplasmic transport, thus resulting in soluble protein. Matin can also be produced in other cells, for instance, it can be produced as a secreted soluble protein in 293 kidney cells using the pcDNA 3.1 eukaryotic vector.

*E. coli*-produced Matin inhibits endothelial cell proliferation of endothelial cells in a dose-dependent manner.

Specific inhibition of endothelial cell proliferation and migration by Matin demonstrates its anti-angiogenic activity, and that it may function via a cell surface protein/receptor. Integrins are potential candidate molecules based on their extracellular matrix binding capacity and ability to modulate cell behavior such as migration and proliferation. In particular, aᵥb₃ integrin is a possible receptor, due to its induction during angiogenesis, and its promiscuous binding capacity. Angiogenesis also depends on specific endothelial cell adhesive events mediated by integrin aᵥb₃ (Brooks, P.C. *et al.,* 1994, *Cell* 79:1157-1164). Matin may disrupt the interaction of proliferating endothelial cells to the matrix component, and thus drive endothelial cells to undergo apoptosis (Re, F. *et al*., 1994, *J. Cell. Biol.* 127:537-546). Matrix metalloproteinases (MMP's) have been implicated as key enzymes that regulate the formation of new blood vessels in tumors (Ray, J.M. *et al*., 1994, *Eur. Respir. J.* 7:2062-2072). Recently, it was demonstrated that an inhibitor of MMP-2 (PEX) can suppress tumor growth by inhibiting angiogenesis (Brooks, P.C. *et al*., 1998, *Cell* 92:391-400). Matin may function through inhibiting the activity of MMPs.

As used herein, the term "angiogenesis" means the generation of new blood vessels into a tissue or organ, and involves endothelial cell proliferation. Under normal physiological conditions, humans or animals undergo angiogenesis only in very specific restricted situations. For example, angiogenesis is normally observed in wound healing, fetal and embryonal development, and formation of the corpus luteum, endometrium and placenta. The term "endothelium" means a thin layer of flat epithelial cells that lines serous cavities, lymph vessels, and blood vessels. "Anti-angiogenic activity" therefore refers to the capability of a composition to inhibit the growth of blood vessels. The growth of blood vessels is a complex series of events, and includes localized breakdown of the basement membrane lying under the individual endothelial cells, proliferation of those cells, migration of the cells to the location of the future blood vessel, reorganization of the cells to form a new vessel membrane, cessation of endothelial cell proliferation, and, incorporation of pericytes and other cells that support the new blood vessel wall. "Anti-angiogenic activity" as used herein therefore includes interruption of any or all of these stages, with the end result that formation of new blood vessels is inhibited.

Anti-angiogenic activity may include endothelial inhibiting activity, which refers to the capability of a composition to inhibit angiogenesis in general and, for example, to inhibit the growth or migration of bovine capillary endothelial cells in culture in the presence of fibroblast growth factor, angiogenesis-associated factors, or other known growth factors. A "growth factor" is a composition that stimulates the growth, reproduction, or synthetic activity of cells. An "angiogenesis-associated factor" is a factor which either inhibits or promotes angiogenesis. An example of an angiogenesis-associated factor is an angiogenic growth factor, such as basic fibroblastic growth factor (bFGF), which is an angiogenesis promoter. Another example of an angiogenesis-associated factor is an angiogenesis inhibiting factor such as *e.g*., angiostatin (see, *e.g.,* U.S. Pat. No. 5,801,012, U.S. Pat. No. 5,837,682, U.S. Pat. No. 5,733,876, U.S. Pat. No. 5,776,704, U.S. Pat. No. 5,639,725, U.S. Pat. No. 5,792,845, WO 96/35774, WO 95/29242, WO 96/41194, WO 97/23500) or endostatin (see, *e.g*., U.S. Pat. No. 5,854,205; U.S. Pat. No. 6,174,861; WO 97/15666).

By "substantially the same biological activity" or "substantially the same or superior biological activity" is meant that a composition has anti-angiogenic activity, and behaves similarly as does Matin, as determined in standard assays. "Standard assays" include, but are not limited to, those protocols used in the molecular biological arts to assess anti-angiogenic activity, cell cycle arrest, and apoptosis. Such assays include, but are not limited to, assays of endothelial cell proliferation, endothelial cell migration, cell cycle analysis, and endothelial cell tube formation, detection of apoptosis, *e*.*g*., by apoptotic cell morphology or Annexin V-FITC assay, chorioallantoic membrane (CAM) assay, and inhibition of renal cancer tumor growth in nude mice. Such assays are provided in the Examples below, and also in U.S.S.N. 09/335,224,"Anti-Angiogenic Proteins and Methods of Use thereof," filed June 17, 1999, by Raghuram Kalluri, and in U.S.S.N. 09/479,118, "Anti-Angiogenic Proteins and Receptors and Methods of Use thereof," by Raghuram Kalluri, filed January 7, 2000.

"Matin" is intended to include fragments, mutants, homologs, analogs, and allelic variants of the amino acid sequence of the Matin sequence, as well as Matin from other globular domains, globular domains from other α chains, other laminins, laminins from other mammals, and fragments, mutants, homologs, analogs and allelic variants of the Matin amino acid sequence.

It is to be understood that the present invention is contemplated to include any derivatives of Matin within the scope of the claims that have endothelial inhibitory activity (*e*.*g*., the capability of a composition to inhibit angiogenesis in general and, for example, to inhibit the growth or migration of bovine capillary endothelial cells in culture in the presence of fibroblast growth factor, angiogenesis-associated factors, or other known growth factors). The present invention includes the entire Matin protein, derivatives of this protein and biologically-active fragments of this protein. This includes proteins with Matin activity that have amino acid substitutions or have sugars or other molecules attached to amino acid functional groups.

The invention also includes fragments, mutants, homologs and analogs of Matin that are within the score of the claims. A "fragment" of a protein is defined herein as any amino acid sequence shorter than that protein, comprising at least 25 consecutive amino acids of the full polypeptide. Such a fragment may alternatively comprise 26,27,28,29,30, 31, 32, 33, 34, 35, 36, 37, 38, 39, 40, 41, 42, 43, 44, 45, 46, 47, 48, 49 or 50 consecutive amino acids of the full polypeptide. The fragment may comprise 51, 52, 53, 54, 55, 56, 57, 58, 59, 60, 61, 62, 63, 64, 65, 66, 67, 68, 69, 70, 71, 72, 73, 74 or 75 consecutive amino acids of the full polypeptide. Such molecules may or may not also comprise additional amino acids derived from the process of cloning, *e*.*g*., amino acid residues or amino acid sequences corresponding to full or partial linker sequences. To be,encompassed by the present invention, such molecules, with or without such additional amino acid residues, must have substantially the same biological activity as the reference polypeptide.

Where the full-length molecule possesses more than one activity, *e.g*., it may be possible to "split" the activities by splitting the full-length protein into several fragments, *e.g*., the full-length protein can be split into two fragments, one of which may possess one activity, while the other possesses another activity. The two fragments may or may not overlap, and the two activities may or may not be apparent in the full-length molecule. For instance, the full-length molecule may possess activity "A", and two fragments whereof may possess activities "A₁" and "A₂", respectively, or they may possess activities "B" and "C". Therefore, when it is stated that a fragment or mutant "must have substantially the same biological activity as the reference polypeptide", it is intended that in situations where one or more biological activities are split, the "reference polypeptide" is that subsequence of the overall molecule that corresponds to the fragment or mutant. That is, the fragment or mutant must have the substantially the same biological activity as that portion of the overall molecule to which they correspond.

By "mutant" of Matin is meant a polypeptide that includes any change in the amino acid sequence relative to the amino acid sequence of the equivalent reference Matin polypeptide. Such changes can arise either spontaneously or by manipulations by man, by chemical energy (*e.g*., X-ray), or by other forms of chemical mutagenesis, or by genetic engineering, or as a result of mating or other forms of exchange of genetic information. Mutations include, *e.g*., base changes, deletions, insertions, inversions, translocations, or duplications. Mutant forms of Matin may display either increased or decreased anti-angiogenic activity relative to the equivalent reference Matin polynucleotide, and such mutants may or may not also comprise additional amino acids derived from the process of cloning, *e.g*., amino acid residues or amino acid sequences corresponding to full or partial linker sequences. Mutants/fragments of the anti-angiogenic proteins of the present invention can also be generated by PCR cloning, or by *Pseudomonas* elastase digestion, as described by Mariyama, M. *et al*. (1992, *J. Biol. Chem.* 267:1253-1258).

By "analog" of Matin is meant a non-natural molecule substantially similar to either the entire Matin molecule or a fragment or allelic variant thereof, and having substantially the same or superior biological activity. Such analogs are intended to include derivatives (*e*.*g*., chemical derivatives, as defined above) of the biologically active Matin, as well as its fragments, mutants, homologs, and allelic variants, which derivatives exhibit a qualitatively similar agonist or antagonist effect to that of the unmodified Matin polypeptide, fragment, mutant, homolog, or allelic variant.

By "allele" of Matin is meant a polypeptide sequence containing a naturally-occurring sequence variation relative to the polypeptide sequence of the reference Matin polypeptide. By "allele" of a polynucleotide encoding the Matin polypeptide is meant a polynucleotide containing a sequence variation relative to the reference polynucleotide sequence encoding the reference Matin polypeptide, where the allele of the polynucleotide encoding the Matin polypeptide encodes an allelic form of the Matin polypeptide.

It is possible that a given polypeptide may be either a fragment, a mutant, an analog, or allelic variant of Matin, or it may be two or more of those things, *e*.*g*., a polypeptide may be both an analog and a mutant of the Matin polypeptide. For example, a shortened version of the Matin molecule (*e.g*., a fragment of Matin) may be created in the laboratory. If that fragment is then mutated through means known in the art, a molecule is created that is both a fragment and a mutant of Matin. In another example, a mutant may be created, which is later discovered to exist as an allelic form of Matin in some mammalian individuals. Such a mutant Matin molecule would therefore be both a mutant and an allelic variant. Such combinations of fragments, mutants, allelic variants, and analogs are intended to be encompassed in the present invention.

Encompassed by the present invention are proteins that have substantially the same amino acid sequence as Matin, or polynucleotides that have substantially the same nucleic acid sequence as the polynucleotides encoding Matin. "Substantially the same sequence" means a nucleic acid or polypeptide that exhibits at least about 70 % sequence identity with a reference sequence, *e.g*., another nucleic acid or polypeptide, typically at least about 80% sequence identity with the reference sequence, preferably at least about 90% sequence identity, more preferably at least about 95% identity, and most preferably at least about 97% sequence identity with the reference sequence. The length of comparison for sequences will generally be at least 75 nucleotide bases or 25 amino acids, more preferably at least 150 nucleotide bases or 50 amino acids, and most preferably 243-264 nucleotide bases or 81-88 amino acids. "Polypeptide" as used herein indicates a molecular chain of amino acids and does not refer to a specific length of the product. Thus, peptides, oligopeptides and proteins are included within the definition of polypeptide. This term is also intended to include polypeptide that have been subjected to post-expression modifications such as, for example, glycosylations, acetylations, phosphorylations and the like.

"Sequence identity," as used herein, refers to the subunit sequence similarity between two polymeric molecules, *e.g*., two polynucleotides or two polypeptides. When a subunit position in both of the two molecules is occupied by the same monomeric subunit, *e*.*g*., if a position in each of two peptides is occupied by serine, then they are identical at that position. The identity between two sequences is a direct function of the number of matching or identical positions, *e*.*g*., if half (*e*.*g*., 5 positions in a polymer 10 subunits in length) of the positions in two peptide or compound sequences are identical, then the two sequences are 50% identical; if 90% of the positions, *e.g*., 9 of 10 are matched, the two sequences share 90% sequence identity. By way of example, the amino acid sequences R₂R₅R₇R₁₀R₆R₃ and R₉R₈R₁R₁₀R₆R₃ have 3 of 6 positions in common, and therefore share 50% sequence identity, while the sequences R₂R₅R₇R₁₀R₆R₃ and R₈R₁R₁₀R₆R₃ have 3 of 5 positions in common, and therefore share 60% sequence identity. The identity between two sequences is a direct function of the number of matching or identical positions. Thus, if a portion of the reference sequence is deleted in a particular peptide, that deleted section is not counted for purposes of calculating sequence identity, *e.g.,* R₂R₅R₇R₁₀R₆R₃ and R₂R₅R₇R₁₀R₃ have 5 out of 6 positions in common, and therefore share 83.3% sequence identity.

Identity is often measured using sequence analysis software *e.g*., BLASTN or BLASTP (available at http://www.ncbi.nlm.nih.gov/BLAST/). The default parameters for comparing two sequences (*e.g.,* "Blast"-ing two sequences against each other, http://www.ncbi.nlm.nih.gov/gorf/bl2.html) by BLASTN (for nucleotide sequences) are reward for match = 1, penalty for mismatch = -2, open gap = 5, extension gap = 2. When using BLASTP for protein sequences, the default parameters are reward for match = 0, penalty for mismatch = 0, open gap = 11, and extension gap =1.

When two sequences share "sequence homology," it is meant that the two sequences differ from each other only by conservative substitutions. For polypeptide sequences, such conservative substitutions consist of substitution of one amino acid at a given position in the sequence for another amino acid of the same class (*e*.*g*., amino acids that share characteristics of hydrophobicity, charge, pK or other conformational or chemical properties, *e.g.,* valine for leucine, arginine for lysine), or by one or more non-conservative amino acid substitutions, deletions, or insertions, located at positions of the sequence that do not alter the conformation or folding of the polypeptide to the extent that the biological activity of the polypeptide is destroyed. Examples of "conservative substitutions" include substitution of one non-polar (hydrophobic) residue such as isoleucine, valine, leucine or methionine for one another; the substitution of one polar (hydrophilic) residue for another such as between arginine and lysine, between glutamine and asparagine, between threonine and serine; the substitution of one basic residue such as lysine, arginine or histidine for one another; or the substitution of one acidic residue, such as aspartic acid or glutamic acid for one another; or the use of a chemically derivatized residue in place of a non-derivatized residue; provided that the polypeptide displays the requisite biological activity. Two sequences which share sequence homology may called "sequence homologs."

The invention contemplates mutants of the proteins and peptides disclosed herein, where the mutation(s) do not substantially alter the activity of the protein or peptide, that is the mutations are effectively "silent" mutations.

Homology, for polypeptides, is typically measured using sequence analysis software (*e*.*g*., Sequence Analysis Software Package of the Genetics Computer Group, University of Wisconsin Biotechnology Center, 1710 University Avenue, Madison, WI 53705). Protein analysis software matches similar sequences by assigning degrees of homology to various substitutions, deletions, and other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

Also encompassed by the present invention are chemical derivatives of Matin. "Chemical derivative" refers to a subject polypeptide having one or more residues chemically derivatized by reaction of a functional side group. Such derivatized residues include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-imbenzylhistidine. Also included as chemical derivatives are those peptides which contain one or more naturally occurring amino acid derivatives of the twenty standard amino acids. For examples: 4-hydroxyproline may be substituted for proline; 5-hydroxylysine may be substitute for lysine; 3-methylhistidine may be substituted for histidine; homoserine may be substituted for serine; and ornithine may be substituted for lysine.

The present invention also includes fusion proteins and chimeric proteins comprising the anti-angiogenic proteins, their fragments, mutants, homologs, analogs, and allelic variants. A fusion or chimeric protein can be produced as a result of recombinant expression and the cloning process, *e*.*g*., the protein may be produced comprising additional amino acids or amino acid sequences corresponding to full or partial linker sequences. A fusion or chimeric protein can consist of a multimer of a single protein, *e.g*., repeats of the anti-angiogenic proteins, or the fusion and chimeric proteins can be made up of several proteins, *e.g*., several of the anti-angiogenic proteins. The fusion or chimeric protein can comprise a combination of two or more known anti-angiogenic proteins (*e.g.,* angiostatin and endostatin, or biologically active fragments of angiostatin and endostatin), or an anti-angiogenic protein in combination with a targeting agent (*e.g.,* endostatin with epidermal growth factor (EGF) or RGD peptides), or an anti-angiogenic protein in combination with an immunoglobulin molecule (*e.g*., endostatin and IgG, specifically with the Fc portion removed). The fusion and chimeric proteins can also include the anti-angiogenic proteins, their fragments, mutants, homologs, analogs, and allelic variants, and other anti-angiogenic proteins, *e*.*g*., endostatin, or angiostatin. Other anti-angiogenic proteins can include Arresten, Canstatin or Tumstatin (PCT/US99/13737), Vascostatin, restin and apomigren (PCT/US98/26058) and fragments of endostatin (PCT/US98/26057). The term "fusion protein" or "chimeric protein" as used herein can also encompass additional components for *e.g.,* delivering a chemotherapeutic agent, wherein a polynucleotide encoding the chemotherapeutic agent is linked to the polynucleotide encoding the anti-angiogenic protein. Fusion or chimeric proteins can also encompass multimers of an anti-angiogenic protein, *e*.*g*., a dimer or trimer. Such fusion or chimeric proteins can be linked together via post-translational modification (*e.g*., chemically linked), or the entire fusion protein may be made recombinantly.

Multimeric proteins comprising Matin, its fragments, mutants, homologs, analogs and allelic variants are also intended to be encompassed by the present invention. By "multimer" is meant a protein comprising two or more copies of a subunit protein. The subunit protein may be one of the proteins of the present invention, *e.g*., Matin repeated two or more times, or a fragment, mutant, homolog, analog or allelic variant, *e.g*., a Matin mutant or fragment, repeated two or more times. Such a multimer may also be a fusion or chimeric protein, *e.g.*, a repeated tumstatin mutant may be combined with polylinker sequence, and/or one or more anti-angiogenic peptides, which may be present in a single copy, or may also be tandemly repeated, *e*.*g*., a protein may comprise two or more multimers within the overall protein.

The invention also encompasses a composition comprising one or more isolated polynucleotide(s) encoding Matin, as well as vectors and host cells containing such a polynucleotide, and processes for producing Matin and its fragments, mutants, homologs, analogs and allelic variants. The term "vector" as used herein means a carrier into which pieces of nucleic acid may be inserted or cloned, which carrier functions to transfer the pieces of nucleic acid into a host cell. Such a vector may also bring about the replication and/or expression of the transferred nucleic acid pieces. Examples of vectors include nucleic acid molecules derived, *e.g*., from a plasmid, bacteriophage, or mammalian, plant or insect virus, or non-viral vectors such as ligand-nucleic acid conjugates, liposomes, or lipid-nucleic acid complexes. It may be desirable that the transferred nucleic molecule is operatively linked to an expression control sequence to form an expression vector capable of expressing the transferred nucleic acid. Such transfer of nucleic acids is generally called "transformation," and refers to the insertion of an exogenous polynucleotide into a host cell, irrespective of the method used for the insertion. For example, direct uptake, transduction or f-mating are included. The exogenous polynucleotide may be maintained as a non-integrated vector, for example, a plasmid, or alternatively, may be integrated into the host genome. "Operably linked" refers to a situation wherein the components described are in a relationship permitting them to function in their intended manner, *e.g*., a control sequence "operably linked" to a coding sequence is ligated in such a manner that expression of the coding sequence is achieved under conditions compatible with the control sequence. A "coding sequence" is a polynucleotide sequence which is transcribed into mRNA and translated into a polypeptide when placed under the control of (*e.g*., operably linked to) appropriate regulatory sequences. The boundaries of the coding sequence are determined by a translation start codon at the 5'-terminus and a translation stop codon at the 3'-terminus. Such boundaries can be naturally-occurring, or can be introduced into or added the polynucleotide sequence by methods known in the art. A coding sequence can include, but is not limited to, mRNA, cDNA, and recombinant polynucleotide sequences.

The vector into which the cloned polynucleotide is cloned may be chosen because it functions in a prokaryotic, or alternatively, it is chosen because it functions in a eukaryotic organism. Two examples of vectors which allow for both the cloning of a polynucleotide encoding the Matin protein, and the expression of that protein from the polynucleotide, are the pET22b and pET28(a) vectors (Novagen, Madison, Wisconsin, USA) and a modified pPICZαA vector (In Vitrogen, San Diego, California, USA), which allow expression of the protein in bacteria and yeast, respectively (see for example, WO 99/29878 and U.S.S.N. 09/589,483).

Once a polynucleotide has been cloned into a suitable vector, it can be transformed into an appropriate host cell. By "host cell" is meant a cell which has been or can be used as the recipient of transferred nucleic acid by means of a vector. Host cells can prokaryotic or eukaryotic, mammalian, plant, or insect, and can exist as single cells, or as a collection, *e*.*g*., as a culture, or in a tissue culture, or in a tissue or an organism. Host cells can also be derived from normal or diseased tissue from a multicellular organism, *e*.*g*., a mammal. Host cell, as used herein, is intended to include not only the original cell which was transformed with a nucleic acid, but also descendants of such a cell, which still contain the nucleic acid.

In one embodiment, the isolated polynucleotide encoding the anti-angiogenic protein additionally comprises a polynucleotide linker encoding a peptide. Such linkers are known to those of skill in the art and, for example the linker can comprise at least one additional codon encoding at least one additional amino acid. Typically the linker comprises one to about twenty or thirty amino acids. The polynucleotide linker is translated, as is the polynucleotide encoding the anti-angiogenic protein, resulting in the expression of an anti-angiogenic protein with at least one additional amino acid residue at the amino or carboxyl terminus of the anti-angiogenic protein. Importantly, the additional amino acid, or amino acids, do not compromise the activity of the anti-angiogenic protein.

After inserting the selected polynucleotide into the vector, the vector is transformed into an appropriate prokaryotic strain and the strain is cultured (*e.g*., maintained) under suitable culture conditions for the production of the biologically active anti-angiogenic protein, thereby producing a biologically active anti-angiogenic protein, or mutant, derivative, fragment or fusion protein thereof. In one embodiment, the invention comprises cloning of a polynucleotide encoding an anti-angiogenic protein into the vectors pET22b, pET17b or pET28a, which are then transformed into bacteria. The bacterial host strain then expresses the anti-angiogenic protein. Typically the anti-angiogenic proteins are produced in quantities of about 10-20 milligrams, or more, per liter of culture fluid.

In another embodiment of the present invention, the eukaryotic vector comprises a modified yeast vector. One method is to use a pPICzα plasmid wherein the plasmid contains a multiple cloning site. The multiple cloning site has inserted into the multiple cloning site a His.Tag motif. Additionally the vector can be modified to add a *Nde*I site, or other suitable restriction sites. Such sites are well known to those of skill in the art. Anti-angiogenic proteins produced by this embodiment comprise a histidine tag motif (His.tag) comprising one, or more histidines, typically about 5-20 histidines. The tag must not interfere with the anti-angiogenic properties of the protein.

One method of producing Matin, for example, is to amplify the polynucleotide of SEQ ID NO:1 and clone it into an expression vector, *e.g*., pET22b, pET28(a), pPICZαA, or some other expression vector, transform the vector containing the polynucleotide into a host cell capable of expressing the polypeptide encoded by the polynucleotide, culturing the transformed host cell under culture conditions suitable for expressing the protein, and then extracting and purifying the protein from the culture. Exemplary methods of producing anti-angiogenic proteins in general, are provided in the Examples below and in U.S.S.N. 09/335, 224, "Anti-Angiogenic Proteins and Methods of Use Thereof," by Raghuram Kalluri, filed June 17, 1999. The Matin protein may also be expressed as a product of transgenic animals, *e.g*., as a component of the milk of transgenic cows, goats, sheep or pigs, as is described in U.S. Pat. No. 5,962,648, or as a product of a transgenic plant, *e.g*., combined or linked with starch molecules in maize, or as is described in U.S. Pat. No. 5,639,947 or 5,990,385.

Matin may also be produced by conventional, known methods of chemical synthesis. Methods for constructing the proteins of the present invention by synthetic means are known to those skilled in the art. The synthetically-constructed Matin protein sequence, by virtue of sharing primary, secondary or tertiary structural and/or conformational characteristics with *e.g*., recombinantly-produced Matin, may possess biological properties in common therewith, including biological activity. Thus, the synthetically-constructed Matin protein sequence may be employed as biologically active or immunological substitutes for *e.g*., recombinantly-produced, purified Matin protein in screening of therapeutic compounds and in immunological processes for the development of antibodies.

The Matin protein is useful in inhibiting angiogenesis, as determined in standard assays, and provided in the Examples below.

Polynucleotides encoding Matin can be cloned out of isolated DNA or a cDNA library. Nucleic acids polypeptides, referred to herein as "isolated" are nucleic acids or polypeptides substantially free (*i.e.,* separated away from) the material of the biological source from which they were obtained (*e.g*., as exists in a mixture of nucleic acids or in cells), which may have undergone further processing. "Isolated" nucleic acids or polypeptides include nucleic acids or polypeptides obtained by methods described herein, similar methods, or other suitable methods, including essentially pure nucleic acids or polypeptides, nucleic acids or polypeptides produced by chemical synthesis, by combinations of chemical or biological methods, and recombinantly produced nucleic acids or polypeptides which are isolated. An isolated polypeptide therefore means one which is relatively free of other proteins, carbohydrates, lipids, and other cellular components with which it is normally associated. An isolated nucleic acid is not immediately contiguous with (*i.e*., covalently linked to) both of the nucleic acids with which it is immediately contiguous in the naturally-occurring genome of the organism from which the nucleic acid is derived. The term, therefore, includes, for example, a nucleic acid which is incorporated into a vector (*e.g*., an autonomously replicating virus or plasmid), or a nucleic acid which exists as a separate molecule independent of other nucleic acids such as a nucleic acid fragment produced by chemical means or restriction endonuclease treatment.

The polynucleotides and proteins of the present invention can also be used to design probes to isolate other anti-angiogenic proteins. Exceptional methods are provided in U.S. Pat. No. 5,837,490, by Jacobs *et al*.. The design of the oligonucleotide probe should preferably follow these parameters: (a) it should be designed to an area of the sequence which has the fewest ambiguous bases ("N's"), if any, and (b) it should be designed to have a Tₘ of approx. 80°C (assuming 2°C for each A or T and 4 degrees for each G or C).

The oligonucleotide should preferably be labeled with g-³²P-ATP (specific activity 6000 Ci/mmole) and T4 polynucleotide kinase using commonly employed techniques for labeling oligonucleotides. Other labeling techniques can also be used. Unincorporated label should preferably be removed by gel filtration chromatography or other established methods. The amount of radioactivity incorporated into the probe should be quantitated by measurement in a scintillation counter. Preferably, specific activity of the resulting probe should be approximately 4 x 10⁶ dpm/pmole. The bacterial culture containing the pool of full-length clones should preferably be thawed and 100 µl of the stock used to inoculate a sterile culture flask containing 25 ml of sterile L-broth containing ampicillin at I00 µg/ml. The culture should preferably be grown to saturation at 37°C, and the saturated culture should preferably be diluted in fresh L-broth. Aliquots of these dilutions should preferably be plated to determine the dilution and volume which will yield approximately 5000 distinct and well-separated colonies on solid bacteriological media containing L-broth containing ampicillin at 100 µg/ml and agar at 1.5% in a 150 mm petri dish when grown overnight at 37°C. Other known methods of obtaining distinct, well-separated colonies can also be employed.

Standard colony hybridization procedures should then be used to transfer the colonies to nitrocellulose filters and lyse, denature and bake them. Highly stringent condition are those that are at least as stringent as, for example, 1x SSC at 65°C, or 1x SSC and 50% formamide at 42°C. Moderate stringency conditions are those that are at least as stringent as 4x SSC at 65°C, or 4x SSC and 50% formamide at 42°C. Reduced stringency conditions are those that are at least as stringent as 4x SSC at 50°C, or 6x SSC and 50% formamide at 40°C.

The filter is then preferably incubated at 65°C for 1 hour with gentle agitation in 6x SSC (20x stock is 175.3 g NaCl/liter, 88.2 g Na citrate/liter, adjusted to pH 7.0 with NaOH) containing 0.5% SDS, 100 µg/ml of yeast RNA, and 10 mM EDTA (approximately 10 mL per 150 min filter). Preferably, the probe is then added to the hybridization mix at a concentration greater than or equal to 1 x 10⁶ dpm/mL. The filter is then preferably incubated at 65°C with gentle agitation overnight. The filter is then preferably washed in 500 mL of 2x SSC/0.5% SDS at room temperature without agitation, preferably followed by 500 mL of 2x SSC/0.1% SDS at room temperature with gentle shaking for 15 minutes. A third wash with 0.1x SSC/0.5% SDS at 65°C for 30 minutes to 1 hour is optional. The filter is then preferably dried and subjected to autoradiography for sufficient time to visualize the positives on the X-ray film. Other known hybridization methods can also be employed. The positive colonies are then picked, grown in culture, and plasmid DNA isolated using standard procedures. The clones can then be verified by restriction analysis, hybridization analysis, or DNA sequencing.

Stringency conditions for hybridization refers to conditions of temperature and buffer composition which permit hybridization of a first nucleic acid sequence to a second nucleic acid sequence, wherein the conditions determine the degree of identity between those sequences which hybridize to each other. Therefore, "high stringency conditions" are those conditions wherein only nucleic acid sequences which are very similar to each other will hybridize. The sequences may be less similar to each other if they hybridize under moderate stringency conditions. Still less similarity is needed for two sequences to hybridize under low stringency conditions. By varying the hybridization conditions from a stringency level at which no hybridization occurs, to a level at which hybridization is first observed, conditions can be determined at which a given sequence will hybridize to those sequences that are most similar to it. The precise conditions determining the stringency of a particular hybridization include not only the ionic strength, temperature, and the concentration of destabilizing agents such as formamide, but also on factors such as the length of the nucleic acid sequences, their base composition, the percent of mismatched base pairs between the two sequences, and the frequency of occurrence of subsets of the sequences (*e*.*g*., small stretches of repeats) within other non-identical sequences. Washing is the step in which conditions are set so as to determine a minimum level of similarity between the sequences hybridizing with each other. Generally, from the lowest temperature at which only homologous hybridization occurs, a 1% mismatch between two sequences results in a 1°C decrease in the melting temperature (Tₘ) for any chosen SSC concentration. Generally, a doubling of the concentration of SSC results in an increase in the Tₘ of about 17°C. Using these guidelines, the washing temperature can be determined empirically, depending on the level of mismatch sought. Hybridization and wash conditions are explained in *Current Protocols in Molecular Biology* (Ausubel, *F.M. et al*., eds., John Wiley & Sons, Inc., 1995, with supplemental updates) on pages 2.10.1 to 2.10.16, and 6.3.1 to 6.3.6.

High stringency conditions can employ hybridization at either (1) 1x SSC (10x SSC = 3 M NaCl, 0.3 M Na₃-citrate·2H₂O (88 g/liter), pH to 7.0 with 1 M HCl), 1% SDS (sodium dodecyl sulfate), 0.1 - 2 mg/ml denatured salmon sperm DNA at 65°C, (2) 1x SSC, 50% formamide, 1% SDS, 0.1 - 2 mg/ml denatured salmon sperm DNA at 42°C, (3) 1% bovine serum albumen (fraction V), 1 mM Na₂·EDTA, 0.5 M NaHPO₄ (pH 7.2) (1 M NaHPO₄ =134 g Na₂HPO₄·7H₂O, 4 ml 85% H₃PO₄ per liter), 7% SDS, 0.1 - 2 mg/ml denatured salmon sperm DNA at 65°C, (4) 50% formamide, 5x SSC, 0.02 M Tris-HCl (pH 7.6), 1x Denhardt's solution (100x =10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5x SSC, 5x Denhardt's solution, 1% SDS, 100 µg/ml denatured salmon sperm DNA at 65°C, or (6) 5x SSC, 5x Denhardt's solution, 50% formamide, 1% SDS, 100 µg/ml denatured salmon sperm DNA at 42°C, with high stringency washes of either (1) 0.3 - O.lx SSC, 0.1 % SDS at 65°C, or (2) 1 mM Na₂EDTA, 40 mM NaHPO₄ (pH 7.2), 1% SDS at 65°C. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations (*e.g*., Na⁺), and "L" is the length of the hybrid in base pairs.

Moderate stringency conditions can employ hybridization at either (1) 4x SSC, (10x SSC = 3 M NaCl, 0.3 M Na₃-citrate·2H₂O (88 g/liter), pH to 7.0 with 1 M HCl), 1% SDS (sodium dodecyl sulfate), 0.1 - 2 mg/ml denatured salmon sperm DNA at 65°C, (2) 4x SSC, 50% formamide, 1% SDS, 0.1 - 2 mg/ml denatured salmon sperm DNA at 42°C, (3) 1% bovine serum albumen (fraction V), 1 mM Na₂·EDTA, 0.5 M NaHPO₄ (pH 7.2) (1 M NaHPO₄ = 134 g Na₂HPO₄·7H₂O, 4 ml 85% H₃PO₄ per liter), 7% SDS, 0.1 - 2 mg/ml denatured salmon sperm DNA at 65°C, (4) 50% formamide, 5x SSC, 0.02 M Tris-HCl (pH 7.6), 1x Denhardt's solution (100x = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5x SSC, 5x Denhardt's solution, 1% SDS, 100 µg/ml denatured salmon sperm DNA at 65°C, or (6) 5x SSC, 5x Denhardt's solution, 50% formamide, 1% SDS, 100 µg/ml denatured salmon sperm DNA at 42°C, with moderate stringency washes of 1x SSC, 0.1% SDS at 65°C. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations (*e*.*g*., Na⁺), and "L" is the length of the hybrid in base pairs.

Low stringency conditions can employ hybridization at either (1) 4x SSC, (10x SSC = 3 M NaCl, 0.3 M Na₃-citrate·2H₂O (88 g/liter), pH to 7.0 with 1 M HCl), 1% SDS (sodium dodecyl sulfate), 0.1- 2 mg/ml denatured salmon sperm DNA at 50°C, (2) 6x SSC, 50% formamide, 1% SDS, 0.1 - 2 mg/ml denatured salmon sperm DNA at 40°C, (3) 1% bovine serum albumen (fraction V), 1 mM Na₂·EDTA, 0.5 M NaHPO₄ (pH 7.2) (1 M NaBPO₄ = 134 g Na₂HPO₄·7H₂O, 4 ml 85% H₃PO₄ per liter), 7% SDS, 0.1 - 2 mg/ml denatured salmon sperm DNA at 50°C, (4) 50% formamide, 5x SSC, 0.02 M Tris-HCl (pH 7.6), 1x Denhardt's solution (100x = 10 g Ficoll 400,10 g polyvinylpymolidone, 10 g bovine serum albumin (fraction V), water to 500 ml), 10% dextran sulfate, 1% SDS, 0.1 - 2 mg/ml denatured salmon sperm DNA at 40°C, (5) 5x SSC, 5x Denhardt's solution, 1% SDS, 100 µg/ml denatured salmon sperm DNA at 50°C, or (6) 5x SSC, 5x Denhardt's solution, 50% formamide, 1% SDS, 100 µg/ml denatured salmon sperm DNA at 40°C, with low stringency washes of either 2x SSC, 0.1 % SDS at 50°C, or (2) 0.5% bovine serum albumin (fraction V), 1 mM Na₂EDTA, 40 mM NaHPO₄ (pH 7.2), 5% SDS. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations (*e*.*g*., Na⁺), and "L" is the length of the hybrid in base pairs.

Methods of inhibiting angiogenesis in mammalian tissue can use Matin or its biologically-active fragments, analogs, homologs, derivatives or mutants. Methods of treating an angiogenesis-mediated disease can use an effective amount of one or more of the anti-angiogenic proteins, or one or more biologically active fragment thereof, or combinations of fragments that possess anti-angiogenic activity, or agonists and antagonists. An effective amount of anti-angiogenic protein is an amount sufficient to inhibit the angiogenesis which results in the disease or condition, thus completely, or partially, alleviating the disease or condition. Alleviation of the angiogenesis-mediated disease can be determined by observing an alleviation of symptoms of the disease, *e.g*., a reduction in the size of a tumor, or arrested tumor growth. As used herein, the term "effective amount" also means the total amount of each active component of the composition or method that is sufficient to show a meaningful patient benefit, *i*.*e*., treatment, healing, prevention or amelioration of the relevant medical condition, or an increase in rate of treatment, healing, prevention or amelioration of such conditions. When applied to a combination, the term refers to combined amounts of the active ingredients that result in the therapeutic effect, whether administered in combination, serially or simultaneously. Angiogenesis-mediated diseases include, but are not limited to, cancers, solid tumors, blood-born tumors (*e*.*g*., leukemias), tumor metastasis, benign tumors (*e.g.,* hemangiomas, acoustic neuromas, neurofibromas, organ fibrosis, trachomas, and pyogenic granulomas), rheumatoid arthritis, psoriasis, ocular angiogenic diseases (*e.g*., diabetic retinopathy, retinopathy of prematurity, macular degeneration, corneal graft rejection, neovascular glaucoma, retrolental fibroplasia, rubeosis), Osler-Webber Syndrome, myocardial angiogenesis, plaque neovascularization, telangiectasia, hemophiliac joints, angiofibroma, and wound granulation. The anti-angiogenic proteins are useful in the treatment of diseases of excessive or abnormal stimulation of endothelial cells. These diseases include, but are not limited to, intestinal adhesions, Crohn's disease, atherosclerosis, scleroderma, fibrosis and hypertrophic scars (*i*.*e*., keloids). The anti-angiogenic proteins can be used as a birth control agent by preventing vascularization required for embryo implantation. The anti-angiogenic proteins are useful in the treatment of diseases that have angiogenesis as a pathologic consequence such as cat scratch disease (*Rochele minalia quintosa*) and ulcers (*Heliobacter pylori*). The anti-angiogenic proteins can also be used to prevent dialysis graft vascular access stenosis, and obesity, *e.g.*, by inhibiting capillary formation in adipose tissue, thereby preventing its expansion. The anti-angiogenic proteins can also be used to treat localized (*e.g*., nonmetastisized) diseases. "Cancer" means neoplastic growth, hyperplastic or proliferative growth or a pathological state of abnormal cellular development and includes solid tumors, non-solid tumors, and any abnormal cellular proliferation, such as that seen in leukemia. As used herein, "cancer" also means angiogenesis-dependent cancers and tumors, *i.e*., tumors that require for their growth (expansion in volume and/or mass) an increase in the number and density of the blood vessels supplying them with blood. "Regression" refers to the reduction of tumor mass and size as determined using methods well-known to those of skill in the art.

Alternatively, where an increase in angiogenesis is desired, *e*.*g*., in wound healing, or in post-infarct heart tissue, antibodies or antisera to the anti-angiogenic proteins can be used to block localized, native anti-angiogenic proteins and processes, and thereby increase formation of new blood vessels so as to inhibit atrophy of tissue.

The anti-angiogenic proteins may be used in combination with themselves, or other compositions and procedures for the treatment of diseases, *e.g*., Matin and Vascostatin can be combined in a pharmaceutical composition, one or more of their fragments can be combined in a composition, or a tumor may be treated conventionally with surgery, radiation, chemotherapy, or immunotherapy, combined with the anti-angiogenic proteins and then the anti-angiogenic proteins may be subsequently administered to the patient to extend the dormancy of micrometastases and to stabilize and inhibit the growth of any residual primary tumor. The anti-angiogenic proteins, or fragments, antisera, receptor agonists, or receptor antagonists thereof, or combinations thereof can also be combined with other anti-angiogenic compounds, or proteins, fragments, antisera, receptor agonists, receptor antagonists of other anti-angiogenic proteins (*e.g*., angiostatin, endostatin). Additionally, the anti-angiogenic proteins, or their fragments, antisera, receptor agonists, receptor antagonists, or combinations thereof, are combined with pharmaceutically acceptable excipients, and optionally sustained-release matrix, such as biodegradable polymers, to form therapeutic compositions. The compositions of the present invention may also contain other anti-angiogenic proteins or chemical compounds, such as endostatin or angiostatin, and mutants, fragments, and analogs thereof. The compositions may further contain other agents which either enhance the activity of the protein or compliment its activity or use in treatment, such as chemotherapeutic or radioactive agents. Such additional factors and/or agents may be included in the composition to produce a synergistic effect with protein of the invention, or to minimize side effects. Additionally, administration of the composition of the present invention may be administered concurrently with other therapies, *e.g*., administered in conjunction with a chemotherapy or radiation therapy regimen.

Angiogenesis can be inhibited in mammalian (*e*.*g*., human) tissues by contacting the tissue with a composition comprising the proteins of the invention. By "contacting" is meant not only topical application, but also those modes of delivery that introduce the composition into the tissues, or into the cells of the tissues.

Use of timed release or sustained release delivery systems are also included in the invention. Such systems are highly desirable in situations where surgery is difficult or impossible, *e.g*., patients debilitated by age or the disease course itself, or where the risk-benefit analysis dictates control over cure.

A sustained-release matrix, as used herein, is a matrix made of materials, usually polymers, which are degradable by enzymatic or acid/base hydrolysis or by dissolution. Once inserted into the body, the matrix is acted upon by enzymes and body fluids. The sustained-release matrix desirably is chosen from biocompatible materials such as liposomes, polylactides (polylactic acid), polyglycolide (polymer of glycolic acid), polylactide co-glycolide (co-polymers of lactic acid and glycolic acid) polyanhydrides, poly(ortho)esters, polyproteins, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrolidone and silicone. A preferred biodegradable matrix is a matrix of one of either polylactide, polyglycolide, or polylactide co-glycolide (co-polymers of lactic acid and glycolic acid).

The angiogenesis-modulating composition of the present invention may be a solid, liquid or aerosol and may be administered by any known route of administration. Examples of solid compositions include pills, creams, and implantable dosage units. The pills may be administered orally, the therapeutic creams may be administered topically. The implantable dosage unit may be administered locally, for example at a tumor site, or which may be implanted for systemic release of the angiogenesis-modulating composition, for example subcutaneously. Examples of liquid composition include formulations adapted for injection subcutaneously, intravenously, intraarterially, and formulations for topical and intraocular administration. Examples of aerosol formulation include inhaler formulation for administration to the lungs.

The proteins and protein fragments with the anti-angiogenic activity described above can be provided as isolated and substantially-purified proteins and protein fragments in pharmaceutically acceptable formulations using formulation methods known to those of ordinary skill in the art. These formulations can be administered by standard routes. In general, the combinations may be administered by the topical, transdermal, intraperitoneal, intracranial, intracerebroventricular, intracerebral, intravaginal, intrauterine, oral, rectal or parenteral (*e*.*g*., intravenous, intraspinal, subcutaneous or intramuscular) route. In addition, the anti-angiogenic proteins may be incorporated into biodegradable polymers allowing for sustained release of the compound, the polymers being implanted in the vicinity of where drug delivery is desired, for example, at the site of a tumor or implanted so that the anti-angiogenic proteins are slowly released systemically. Osmotic minipumps may also be used to provide controlled delivery of high concentrations of the anti-angiogenic proteins through cannulae to the site of interest, such as directly into a metastatic growth or into the vascular supply to that tumor. The biodegradable polymers and their use are described, for example, in detail in Brem *et al.* (1991, *J*. *Neurosurg.* 74:441-6).

The compositions containing a polypeptide of this invention can be administered intravenously, as by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; *i.e*., carrier or vehicle.

Modes of administration of the compositions of the present inventions include intravenous, intramuscular, intraperitoneal, intrasternal, subcutaneous and intraarticular injection and infusion. Pharmaceutical compositions for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyois (*e.g*., glycerol, propylene glycol, polyethylene glycol and the like), carboxymethylcellulose and suitable mixtures thereof, vegetable oils (*e*.*g*., olive oil) and injectable organic esters such as ethyl oleate. Proper fluidity may be maintained, for example, by the use of coating materials such as lecithin, by the maintenance of the required particle size in the case of dispersions and by the use of surfactants. These compositions may also contain adjuvants such as preservatives, wetting agents, emulsifying agents and dispersing agents. Prevention of the action of microorganisms may be ensured by the inclusion of various antibacterial and antifungal agents such as paraben, chlorobutanol, phenol sorbic acid and the like. It may also be desirable to include isotonic agents such as sugars, sodium chloride and the like. Prolonged absorption of the injectable pharmaceutical form may be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polmer employed, the rate of drug release can be controlled. Depot injectable formulations are also prepared by entrapping the drug in liposomes or microemulsions which are compatible with body tissues. The injectable formulations may be sterilized, for example, by filtration through a bacterial-retaining filter or by incorporating sterilizing agents in the form of sterile solid compositions which can be dissolved or dispersed in sterile water or other sterile injectable media just prior to use.

The therapeutic compositions of the present invention can include pharmaceutically acceptable salts of the components therein, *e*.*g*., which may be derived from inorganic or organic acids. By "pharmaceutically acceptable salt" is meant those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge, et al. describe pharmaceutically acceptable salts in detail in *J. Pharmaceutical Sciences* (1977) 66:1 *et seq*.. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like. The salts may be prepared *in situ* during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsufonate, digluconate, glycerophosphate, hemisulfate, heptonoate, hexanoate, fumarate, hydrochloride, hydrobromide, hydroiodide, 2-hydroxymethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl, and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

As used herein, the terms "pharmaceutically acceptable," "physiologically tolerable" and grammatical variations thereof as they refer to compositions, carriers, diluents and reagents, are used interchangeably and represent that the materials are capable of administration to or upon a mammal with a minimum of undesirable physiological effects such as nausea, dizziness, gastric upset and the like. The preparation of a pharmacological composition that contains active ingredients dissolved or dispersed therein is well understood in the art and need not be limited based on formulation. Typically such compositions are prepared as injectables either as liquid solutions or suspensions, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients include, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The anti-angiogenic proteins of the present invention can also be included in a composition comprising a prodrug. As used herein, the term "prodrug" refers to compounds which are rapidly transformed *in vivo* to yield the parent compound, for example, by enzymatic hydrolysis in blood. A thorough discussion is provided in T. Higuchi and V. Stella, *Prodrugs as Novel Delivery Systems*, Vol. 14 of the ACS Symposium Series and in Edward B. Roche, ed., *Bioreversible Carriers in Drug Design,* American Pharmaceutical Association and Permagon Press, 1987. As used herein, the term "pharmaceutically acceptable prodrug" refers to (1) those prodrugs of the compounds of the present invention which are, within the scope of sound medical judgement, suitable for use in contact with the tissues of humans and animals without undue toxicity, irritation, allergic response and the like, commensurate with a suitable benefit-to-risk ratio and effective for their intended use and (2) zwitterionic forms, where possible, of the parent compound.

The dosage of the anti-angiogenic proteins of the present invention will depend on the disease state or condition being treated and other clinical factors such as weight and condition of the human or animal and the route of administration of the compound. Depending upon the half-life of the anti-angiogenic proteins in the particular animal or human, the anti-angiogenic proteins can be administered between several times per day to once a week. It is to be understood that the present invention has application for both human and veterinary use. The methods of the present invention contemplate single as well as multiple administrations, given either simultaneously or over an extended period of time. In addition, the anti-angiogenic proteins can be administered in conjunction with other forms of therapy, *e*.*g*., chemotherapy, radiotherapy, or immunotherapy.

The anti-angiogenic protein formulations include those suitable for oral, rectal, ophthalmic (including intravitreal or intracameral), nasal, topical (including buccal and sublingual), intrauterine, vaginal or parenteral (including subcutaneous, intraperitoneal, intramuscular, intravenous, intradermal, intracranial, intratracheal, and epidural) administration. The anti-angiogenic protein formulations may conveniently be presented in unit dosage form and may be prepared by conventional pharmaceutical techniques. Such techniques include the step of bringing into association the active ingredient and the pharmaceutical carrier(s) or excipient(s). In general, the formulations are prepared by uniformly and intimately bringing into association the active ingredient with liquid carriers or finely divided solid carriers or both, and then, if necessary, shaping the product.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the kind previously described.

When an effective amount of protein of the present invention is administered orally, the anti-angiogenic proteins of the present invention will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% protein of the present invention, and preferably from about 25 to 90% protein of the present invention. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of protein of the present invention, and preferably from about 1 to 50% protein of the present invention.

When an effective amount of protein of the present invention is administered by intravenous, cutaneous or subcutaneous injection, protein of the present invention will be in the form of a pyrogen-free, parenterally acceptable aqueous solution. The preparation of such parenterally acceptable protein solutions, having due regard to pH, isotonicity, stability, and the like, is within the skill in the art. A preferred pharmaceutical composition for intravenous, cutaneous, or subcutaneous injection should contain, in addition to protein of the present invention, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art.

The amount of protein of the present invention in the pharmaceutical composition of the present invention will depend upon the nature and severity of the condition being treated, and on the nature of prior treatments which the patient has undergone. Ultimately, the attending physician will decide the amount of protein of the present invention with which to treat each individual patient. Initially, the attending physician will administer low doses of protein of the present invention and observe the patient's response. Larger doses of protein of the present invention may be administered until the optimal therapeutic effect is obtained for the patient, and at that point the dosage is not increased further.

The duration of intravenous therapy using the pharmaceutical composition of the present invention will vary, depending on the severity of the disease being treated and the condition and potential idiosyncratic response of each individual patient. It is contemplated that the duration of each application of the protein of the present invention will be in the range of 12 to 24 hours of continuous intravenous administration. Ultimately the attending physician will decide on the appropriate duration of intravenous therapy using the pharmaceutical composition of the present invention.

Preferred unit dosage formulations are those containing a daily dose or unit, daily sub-dose, or an appropriate fraction thereof, of the administered ingredient. It should be understood that in addition to the ingredients, particularly mentioned above, the formulations of the present invention may include other agents conventional in the art having regard to the type of formulation in question. Optionally, cytotoxic agents may be incorporated or otherwise combined with the anti-angiogenic proteins, or biologically functional protein fragements thereof, to provide dual therapy to the patient.

The therapeutic compositions are also presently valuable for veterinary applications. Particularly domestic animals and thoroughbred horses, in addition to humans, are desired patients for such treatment with proteins of the present invention.

Cytotoxic agents such as ricin, can be linked to the anti-angiogenic proteins, and fragments thereof, thereby providing a tool for destruction of cells that bind the anti-angiogenic proteins. These cells may be found in many locations, including but not limited to, micrometastases and primary tumors. Proteins linked to cytotoxic agents are infused in a manner designed to maximize delivery to the desired location. For example, non-linked high affinity fragments are delivered through a cannula into vessels supplying the target site or directly into the target Such agents are also delivered in a controlled manner through osmotic pumps coupled to infusion cannulae. A combination of antagonists to the anti-angiogenic proteins may be co-applied with stimulators of angiogenesis to increase vascularization of tissue. This therapeutic regimen provides an effective means of destroying metastatic cancer.

Additional treatment methods include administration of the anti-angiogenic proteins, fragments, analogs, antisera, or receptor agonists and antagonists thereof, linked to cytotoxic agents. It is to be understood that the anti-angiogenic proteins can be human or animal in origin. The anti-angiogenic proteins can also be produced synthetically by chemical reaction or by recombinant techniques in conjunction with expression systems. The anti-angiogenic proteins can also be produced by enzymatically cleaving isolated laminin to generate proteins having anti-angiogenic activity. The anti-angiogenic proteins may also be produced by compounds that mimic the action of endogenous enzymes that cleave laminin to the anti-angiogenic proteins. Production of the anti-angiogenic proteins may also be modulated by compounds that affect the activity of cleavage enzymes.

The present invention also encompasses gene therapy whereby a polynucleotide encoding the anti-angiogenic proteins, integrins, integrin subunits, or a mutant, fragment, or fusion protein thereof, is introduced and regulated in a patient. Various methods of transferring or delivering DNA to cells for expression of the gene product protein, otherwise referred to as gene therapy, are disclosed in *Gene Transfer into Mammalian Somatic Cells in vivo,* N. Yang (1992) *Crit. Rev. Biotechn.* 12(4):335-56. Gene therapy encompasses incorporation of DNA sequences into somatic cells or germ line cells for use in either ex vivo or in vivo therapy. Gene therapy functions to replace genes, augment normal or abnormal gene function, and to combat infectious diseases and other pathologies.

Strategies for treating these medical problems with gene therapy include therapeutic strategies such as identifying the defective gene and then adding a functional gene to either replace the function of the defective gene or to augment a slightly functional gene; or prophylactic strategies, such as adding a gene for the product protein that will treat the condition or that will make the tissue or organ more susceptible to a treatment regimen. As an example of a prophylactic strategy, a gene such as that encoding one or more of the anti-angiogenic proteins may be placed in a patient and thus prevent occurrence of angiogenesis; or a gene that makes tumor cells more susceptible to radiation could be inserted and then radiation of the tumor would cause increased killing of the tumor cells.

Many protocols for transfer of the DNA or regulatory sequences of the anti-angiogenic proteins are envisioned in this invention. Transfection of promoter sequences, other than one normally found specifically associated with the anti-angiogenic proteins, or other sequences which would increase production of the anti-angiogenic proteins are also envisioned as methods of gene therapy. An example of this technology is found in Transkaryotic Therapies, Inc., of Cambridge, Mass., using homologous recombination to insert a "genetic switch" that turns on an erythropoietin gene in cells. See *Genetic Engineering News,* Apr. 15, 1994. Such "genetic switches" could be used to activate the anti-angiogenic proteins (or their receptors) in cells not normally expressing those proteins (or receptors).

Gene transfer methods for gene therapy fall into three broad categories: physical (*e.g*., electroporation, direct gene transfer and particle bombardment), chemical (*e*.*g*., lipid-based carriers, or other non-viral vectors) and biological (*e*.*g*., virus-derived vector and receptor uptake). For example, non-viral vectors may be used which include liposomes coated with DNA. Such liposome/DNA complexes may be directly injected intravenously into the patient. It is believed that the liposome/DNA complexes are concentrated in the liver where they deliver the DNA to macrophages and Kupffer cells. These cells are long lived and thus provide long term expression of the delivered DNA. Additionally, vectors or the "naked" DNA of the gene may be directly injected into the desired organ, tissue or tumor for targeted delivery of the therapeutic DNA.

Gene therapy methodologies can also be described by delivery site. Fundamental ways to deliver genes include *ex vivo* gene transfer, *in vivo* gene transfer, and *in vitro* gene transfer. In *ex vivo* gene transfer, cells are taken from the patient and grown in cell culture. The DNA is transfected into the cells, the transfected cells are expanded in number and then reimplanted in the patient. In *in vitro* gene transfer, the transformed cells are cells growing in culture, such as tissue culture cells, and not particular cells from a particular patient. These "laboratory cells" are transfected, the transfected cells are selected and expanded for either implantation into a patient or for other uses.

*In vivo* gene transfer involves introducing the DNA into the cells of the patient when the cells are within the patient. Methods include using virally mediated gene transfer using a noninfectious virus to deliver the gene in the patient or injecting naked DNA into a site in the patient and the DNA is taken up by a percentage of cells in which the gene product protein is expressed. Additionally, the other methods described herein, such as use of a "gene gun," may be used for in vitro insertion of the DNA or regulatory sequences controlling production of the anti-angiogenic proteins.

Chemical methods of gene therapy may involve a lipid based compound, not necessarily a liposome, to transfer the DNA across the cell membrane. Lipofectins or cytofectins, lipid-based positive ions that bind to negatively charged DNA, make a complex that can cross the cell membrane and provide the DNA into the interior of the cell. Another chemical method uses receptor-based endocytosis, which involves binding a specific ligand to a cell surface receptor and enveloping and transporting it across the cell membrane. The ligand binds to the DNA and the whole complex is transported into the cell. The ligand gene complex is injected into the blood stream and then target cells that have the receptor will specifically bind the ligand and transport the ligand-DNA complex into the cell.

Many gene therapy methodologies employ viral vectors to insert genes into cells. For example, altered retrovirus vectors have been used in *ex vivo* methods to introduce genes into peripheral and tumor-infiltrating lymphocytes, hepatocytes, epidermal cells, myocytes, or other somatic cells. These altered cells are then introduced into the patient to provide the gene product from the inserted DNA.

Viral vectors have also been used to insert genes into cells using in vivo protocols. To direct the tissue-specific expression of foreign genes, cis-acting regulatory elements or promoters that are known to be tissue-specific can be used. Alternatively, this can be achieved using *in situ* delivery of DNA or viral vectors to specific anatomical sites *in vivo.* For example, gene transfer to blood vessels *in vivo* was achieved by implanting *in vitro* transduced endothelial cells in chosen sites on arterial walls. The virus infected surrounding cells which also expressed the gene product. A viral vector can be delivered directly to the *in vivo* site, by a catheter for example, thus allowing only certain areas to be infected by the virus, and providing long-term, site specific gene expression. *In vivo* gene transfer using retrovirus vectors has also been demonstrated in mammary tissue and hepatic tissue by injection of the altered virus into blood vessels leading to the organs.

Viral vectors that have been used for gene therapy protocols include but are not limited to, retroviruses, other RNA viruses such as poliovirus or Sindbis virus, adenovirus, adeno-associated virus, herpes viruses, SV 40, vaccinia and other DNA viruses. Replication-defective murine retroviral vectors are the most widely utilized gene transfer vectors. Murine leukemia retroviruses are composed of a single strand RNA complexed with a nuclear core protein and polymerase (pol) enzymes, encased by a protein core (gag) and surrounded by a glycoprotein envelope (env) that determines host range. The genomic structure of retroviruses include the *gag, pol,* and *env* genes enclosed at by the 5' and 3' long terminal repeats (LTR). Retroviral vector systems exploit the fact that a minimal vector containing the 5' and 3' LTRs and the packaging signal are sufficient to allow vector packaging, infection and integration into target cells providing that the viral structural proteins are supplied in trans in the packaging cell line. Fundamental advantages of retroviral vectors for gene transfer include efficient infection and gene expression in most cell types, precise single copy vector integration into target cell chromosomal DNA, and ease of manipulation of the retroviral genome.

The adenovirus is composed of linear, double stranded DNA complexed with core proteins and surrounded with capsid proteins. Advances in molecular virology have led to the ability to exploit the biology of these organisms to create vectors capable of transducing novel genetic sequences into target cells *in vivo.* Adenoviral-based vectors will express gene product proteins at high levels. Adenoviral vectors have high efficiencies of infectivity, even with low titers of virus. Additionally, the virus is fully infective as a cell free virion so injection of producer cell lines is not necessary. Another potential advantage to adenoviral vectors is the ability to achieve long term expression of heterologous genes *in vivo*.

Mechanical methods of DNA delivery include fusogenic lipid vesicles such as liposomes or other vesicles for membrane fusion, lipid particles of DNA incorporating cationic lipid such as lipofectin, polylysine-mediated transfer of DNA, direct injection of DNA, such as microinjection of DNA into germ or somatic cells, pneumatically delivered DNA-coated particles, such as the gold particles used in a "gene gun," and inorganic chemical approaches such as calcium phosphate transfection. Particle-mediated gene transfer methods were first used in transforming plant tissue. With a particle bombardment device, or "gene gun," a motive force is generated to accelerate DNA-coated high density particles (such as gold or tungsten) to a high velocity that allows penetration of the target organs, tissues or cells. Particle bombardment can be used in *in vitro* systems, or with *ex vivo* or *in vivo* techniques to introduce DNA into cells, tissues or organs. Another method, ligand-mediated gene therapy, involves complexing the DNA with specific ligands to form ligand-DNA conjugates, to direct the DNA to a specific cell or tissue.

It has been found that injecting plasmid DNA into muscle cells yields high percentage of the cells which are transfected and have sustained expression of marker genes. The DNA of the plasmid may or may not integrate into the genome of the cells. Non-integration of the transfected DNA would allow the transfection and expression of gene product proteins in terminally differentiated, non-proliferative tissues for a prolonged period of time without fear of mutational insertions, deletions, or alterations in the cellular or mitochondrial genome. Long-term, but not necessarily permanent, transfer of therapeutic genes into specific cells may provide treatments for genetic diseases or for prophylactic use. The DNA could be reinjected periodically to maintain the gene product level without mutations occurring in the genomes of the recipient cells. Non-integration of exogenous DNAs may allow for the presence of several different exogenous DNA constructs within one cell with all of the constructs expressing various gene products.

Electroporation for gene transfer uses an electrical current to make cells or tissues susceptible to electroporation-mediated mediated gene transfer. A brief electric impulse with a given field strength is used to increase the permeability of a membrane in such a way that DNA molecules can penetrate into the cells. This technique can be used in *in vitro* systems, or with *ex vivo* or *in vivo* techniques to introduce DNA into cells, tissues or organs.

Carrier mediated gene transfer *in vivo* can be used to transfect foreign DNA into cells. The carrier-DNA complex can be conveniently introduced into body fluids or the bloodstream and then site-specifically directed to the target organ or tissue in the body. Both liposomes and polycations, such as polylysine, lipofectins or cytofectins, can be used. Liposomes can be developed which are cell specific or organ specific and thus the foreign DNA carried by the liposome will be taken up by target cells. Injection of immunoliposomes that are targeted to a specific receptor on certain cells can be used as a convenient method of inserting the DNA into the cells bearing the receptor. Another carrier system that has been used is the asialoglycoportein/polylysine conjugate system for carrying DNA to hepatocytes for *in vivo* gene transfer.

The transfected DNA may also be complexed with other kinds of carriers so that the DNA is carried to the recipient cell and then resides in the cytoplasm or in the nucleoplasm. DNA can be coupled to carrier nuclear proteins in specifically engineered vesicle complexes and carried directly into the nucleus.

Gene regulation of the anti-angiogenic proteins may be accomplished by administering compounds that bind to the gene encoding one of the anti-angiogenic proteins, or control regions associated with the gene, or its corresponding RNA transcript to modify the rate of transcription or translation. Additionally, cells transfected with a DNA sequence encoding the anti-angiogenic proteins may be administered to a patient to provide an *in vivo* source of those proteins. For example, cells may be transfected with a vector containing a nucleic acid sequence encoding the anti-angiogenic proteins. The transfected cells may be cells derived from the patient's normal tissue, the patient's diseased tissue, or may be non-patient cells.

For example, tumor cells removed from a patient can be transfected with a vector capable of expressing the proteins of the present invention, and re-introduced into the patient. The transfected tumor cells produce levels of the protein in the patient that inhibit the growth of the tumor. Patients may be human or non-human animals. Cells may also be transfected by non-vector, or physical or chemical methods known in the art such as electroporation, ionoporation, or via a "gene gun." Additionally, the DNA may be directly injected, without the aid of a carrier, into a patient. In particular, the DNA may be injected into skin, muscle or blood.

The gene therapy protocol for transfecting the anti-angiogenic proteins into a patient may either be through integration of the anti-angiogenic protein DNA into the genome of the cells, into minichromosomes or as a separate replicating or non-replicating DNA construct in the cytoplasm or nucleoplasm of the cell. Expression of the anti-angiogenic proteins may continue for a long-period of time or may be reinjected periodically to maintain a desired level of the protein(s) in the cell, the tissue or organ or a determined blood level.

Angiogenesis-inhibiting proteins of the present invention can be synthesized in a standard microchemical facility and purity checked with HPLC and mass spectrophotometry. Methods of protein synthesis, HPLC purification and mass spectrophotometry are commonly known to those skilled in these arts. The anti-angiogenic proteins are also produced in recombinant *E*. *coli* or yeast expression systems, and purified with column chromatography.

Different protein fragmsnis of the intact anti-angiogenic proteins can be synthesized for use in several applications including, but not limited to the following; as antigens for the development of specific antisera, as agonists and antagonists active at binding sites of the anti-angiogenic proteins, as proteins to be linked to, or used in combination with, cytotoxic agents for targeted killing of cells that bind the anti-angiogenic proteins.

The synthetic protein fragments of the anti-aagiogenic proteins have a variety of uses. The protein that binds to the receptor(s) of the anti-angiogenic proteins with high specificity and avidity is radiolabeled and employed for visualization and quantitation of binding sites using autoradiographic and membrane binding techniques. This application provides important diagnostic and research tools. Knowledge of the binding properties of the receptor(s) facilitates investigation of the transduction mechanisms linked to the recxptor(s).

The anti-angiogenic proteins and proteins derived from them can be coupled to other molecules using standard methods. The amino and carboxyl termini of the anti-angiogenic proteins both contain tyrosine and lysine residues and are isotopically and nonisotopically labeled with many techniques, for example radiolabeling using conventional techniques (tyrosine residues-chloramine T, iodogen, lactoperoxidase; lysine residues-Bolton-Hunter reagent). These coupling techniques are well known to those skilled in the art. Alternatively, tyrosine or lysine is added to fragments that do not have these residues to facilitate labeling of reactive amino and hydroxyl groups on the protein. The coupling technique is chosen on the basis of the functional groups available on the amino acids including, but not limited to amino, sulfhydral, carboxyl, amide, phenol, and irnidazole. Various reagents used to effect these couplings include among others, glutaraldehyde, diazotized benzidine, carbodiimide, and p-benzoquinone.

The anti-angiogenic proteins are chemically coupled to isotopes, enzymes, carrier proteins, cytotoxic agents, fluorescent molecules, chemiluminescent, bioluminescent and other compounds for a variety of applications. The efficiency of the coupling reaction is determined using different techniques appropriate for the specific reaction. For example, radiolabeling of a protein of the present invention with ¹²⁵I is accomplished using chloramine T and Na¹²⁵I of high specific activity. The reaction is terminated with sodium metabisulfite and the mixture is desalted on disposable columns. The labeled protein is eluted from the column and fractions are collected. Aliquots are removed from each fraction and radioactivity measured in a gamma counter. In this manner, the unreacted Na¹²⁵I is separated from the labeled protein. The protein fractions with the highest specific radioactivity are stored for subsequent use such as analysis of the ability to bind to antisera of the anti-angiogenic proteins.

In addition, labeling the anti-angiogenic proteins with short lived isotopes enables visualization of receptor binding sites *in vivo* using positron emission tomography or other modern radiographic techniques to locate tumors with the proteins' binding sites.

Systematic substitution of amino acids within these synthesized proteins yields high affinity protein agonists and antagonists to the receptor(s) of the anti-angiogenic proteins that enhance or diminish binding to the receptor(s). Such agonists are used to suppress the growth of micmmetastases, thereby limiting the spread of cancer. Antagonists to the anti-angiogenic proteins are applied in situations of inadequate vascularization, to block the inhibitory effects of the anti-angiogenic proteins and promote angiogenesis. For example, this treatment may have therapeutic effects to promote wound healing in diabetics.

The invention is further illustrated by the following examples, which are not meant to be construed in any way as imposing limitations upon the scope thereof.

### EXAMPLES

### Example 1: Recombinant Production of Matin in E. coli.

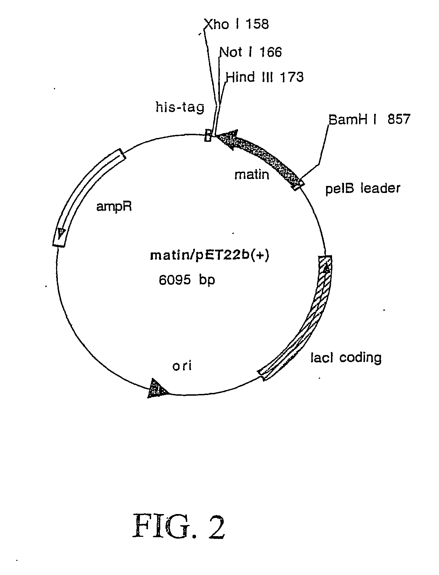

The nucleotide (SEQ ID NO:1) and amino acid (SEQ ID NO:2) sequences for the α1 chain of laminin (GenBank Acc. No. NM_008480) are shown in Fig. 1. The sequence encoding Matin (globular domain 1, or the G1 domain, extending approximately from nucleotide 6442 to nucleotide 7062) was amplified by PCR from the plasmid FBssrAi using the forward primer 5'-CGG-GAT-CCT-AGA-GAC-TGC-ATC-CGC-GCC-TAT-3' (SEQ ID NO:3), and the reverse primer was 5'-CCC-AAG-CTT-TAC-TAT-CTG-CGT-CAC-GGT-GGG-3' (SEQ ID NO:4) (underlined portions of the primer represent laminin sequence). The resulting cDNA fragment was digested with *Bam*HI and *Hin*dIII and ligated into predigested pET22b(+) (Novagen, Madison, Wisconsin, USA). The construct is shown in Fig. 2. The ligation placed Matin in-frame with the pelB leader sequence, allowing for periplasmic localization and expression of sohible protein. The 3' end of the sequence was ligated in-frame with the polyhistidine tag sequence.

Plasmid constructs encoding Matin were first transformed into *E. coli* HMS 174 (Novagen, Madison, Wisconsin, USA) and then transformed into BL21 for expression (Novagen, Madison, Wisconsin, USA). Overnight bacterial culture was used to inoculate a 500 ml culture in LB medium (Fisher Scientific, Pittsburgh, Pennsylvania, USA). This culture was grown for approximately 4 hours until the cells reached an OD₆₀₀ of 0.6. Protein expression was then induced by addition of IPTG to a final concentration of 1 mM. After a 2-hour induction, cells were harvested by centrifugation at 5,000 x g and lysed by resnspension in 6 M guanidine, 0.1 M NaH₂PO₄, 0.01 M Tris-HCl, pH 8.0. Resuspended cells were sonicated briefly, and centrifuged at 12,000 x g for 30 minutes. The supernatant fraction was passed over a 5 ml Ni-NTA agarose column (Qiagen, Hilden, Germany) 4-6 times at a speed of 2 ml per minute. Non-specifically bound protein was removed by washing with both 10 mM and 25 mM imidazole in 8 M urea, 0.1 M NaH₂PO₄, 0.01 M Tris-HCl, pH 8.0. Matin protein was eluted from the column with increasing concentrations of imidazole (50 mM, 125 mM, and 250 mM) in 8 M urea, 0.1 M NaH₂PO₄, 0.01 M Tris-HCl, pH 8.0. The eluted protein was dialyzed twice against PBS at 4°C. A portion of the total protein precipitated during dialysis. Dialyzed protein was collected and centrifuged at approximately 3,500 x g and separated into insoluble (pellet) and soluble (supematant) fractions.

*E. coli*-ex-pressed Matin was isolated predominantly as a soluble protein and SDS-PAGE analysis revealed a monomeric band at about 30 kDa. The eluted fractions containing this band were used in the following experiments. Protein concentration in each fraction was determined by the BCA assay (Pierce Chemical Co., Rockford, Illinois, USA) and quantitative SDS-PAGE analysis using scanning densitometry.

### Example 2: Matin Inhibits Endothelial Cell Proliferation.

The anti-proliferative effect of Matin on C-PAE cells was examined by the methylene blue staining assay using *E*. *coli* produced soluble protein.

*Cell lines and culture*. PC-3 (human prostate adenocarcinoma cell line) and C-PAE (bovine pulmonary arterial endothelial cell line) cells were obtained from American Type Culture Collection. The C-PAE cell lines were maintained in DMEM (Life Technologies/Gibco BRL, Gaithersburg, Maryland, USA) supplemented with 10% fetal calf serum (FCS), 100 units/ml of penicillin, and 100 mg/ml of streptomycin.

*Proliferation assay.* C-PAE cells were grown to confluence in DMEM with 10% FCS and kept contact-inhibited for 48 hours. C-PAE cells were used between the second and fourth passages. PC-3 cells were used as non-endothelial controls in this experiment. Cells were harvested by trypsinization (Life Technologies/Gibco BRL, Gaithersberg, Maryland, USA) at 37°C for 5 minutes. A suspension of 12,500 cells in DMEM with 0.1 % FCS was added to each well of a 24-well plate coated with 10 µg/ml fibronectin. The cells were incubated for 24 hours at 37°C with 5% CO₂ and 95% humidity. Medium was removed and replaced with DMEM containing 20% FCS. Unstimulated control cells were incubated with 0.1 % FCS.

Using the methylene-blue staining method, 7000 cells were plated into each well of a 96-well plate, and treated as described above. Cells were then counted using the method of Oliver *et al*. (Oliver, M.H. *et al*., 1989, *J. Cell. Sci.* 92:513-518). After 48 hours of treatment, all wells were washed with 100 µl of PBS, and the cells fixed with 10% formalin in neutral-buffered saline (Sigma Chemical Co., St. Louis, Missouri, USA). The cells were then stained with 1% methylene blue (Sigma) in 0.01M borate buffer, pH 8.5. Wells were washed with 0.01M borate buffer, and the methylene blue extracted from the cells with 0.1N HCl/ethanol, and the absorbance measured in a microplate reader (Bio-Rad, Hercules, California, USA) at 655 nm. Polymyxin B (Sigma) at a final concentration of 5 µg/ml was used to inactivate endotoxin (Liu, S. *et al*., 1997, *Clin. Biochem.* 30:455-463).

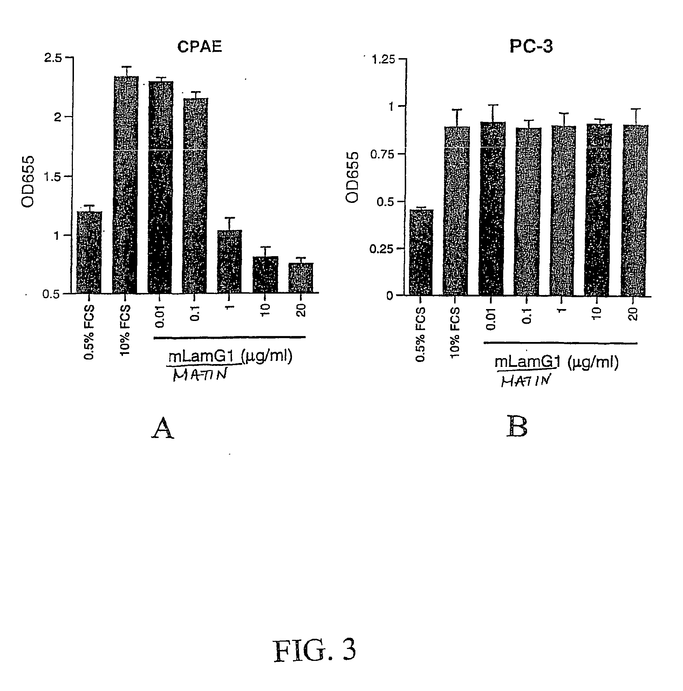

The results are shown in Figs. 3A and 3B, which are a pair of histograms showing the effect of increasing amounts of Matin on the uptake of dye by C-PAE cells relative to PC-3 cells. Absorbance at OD₆₅₅ is shown on the y-axis. "0.5% FCS" represents the 0.5% FCS-treated (unstimulated) control, and "10% FCS" is the 10% FCS-treated (stimulated) control. The remaining bars represent treatments with increasing concentrations of Matin. Matin inhibited FCS-stimulated proliferation of C-PAE cells in a dose-dependent manner. The difference between the mean value of the cell number in the Matin treatment versus the control was significant in the 0.1 - 10.0 µg/ml range, with p<0.05. When PC-3 cells were treated with Matin, no inhibitory effect was observed. In C-PAE cells, dye uptake dropped off to the level seen in unstimulated cells at a Matin treatment level of about 0.1 µg/ml. Each bars represents the mean of the relative absorbance units at 655 nm ± the standard error of the mean for triplicate wells. This endothelial cell specificity indicates that Matin is likely an effective anti-angiogenic agent.

### Example 3: Matin Induces Endothelial Cell Apoptosis.

*Annexin V-FITC assay.* In the early stage of apoptosis, translocation of the membrane phospholipid PS from the inner surface of plasma membrane to outside is observed (van Engeland, M. *et al*., 1998, *Cytometry* 31:1-9; Zhang, G*. et al.,* 1997, *Biotechniques* 23:525-531; Koopman, G. *et al*. 1994, *Blood* 84:1415-1420). Externalized PS can be detected by staining with a FITC conjugate of Annexin V that has a naturally high binding affinity to PS (van Engeland, *supra*)*.* Apoptosis of endothelial cells upon treatment with Matin was therefore evaluated using annexin V-FITC labeling.

C-PAE cells (0.5 x 10⁶ per well) were seeded onto a 6-well plate in 10% FCS supplemented DMEM. The next day, fresh medium containing 10% FCS was added with either 80 ng/ml of TNF-α (positive control) or Matin ranging from 0.02 to 20 µg/ml. Control cells received an equal volume of PBS. After 18 hours of treatment, medium containing floating cells was collected, and attached cells were trypsinized and centrifuged together with floating cells at 3,000 x g. The cells were then washed in PBS and resuspended in binding buffer (10 mM HEPES/NaOH, pH 7.4, 140 mM NaCl, 2.5 mM CaCl₂). Annexin V-FITC (Clontech, Palo Alto, California, USA) was added to a final concentration of 150 ng/ml, and the cells were incubated in the dark for 10 minutes. The cells were washed again in PBS and resuspended in binding buffer. Annexin V-FITC labeled cells were counted using a FACStar Plus flow cytometer (Becton-Dickinson, Waltham, Massachusetts, USA). For each treatment, 15,000 cells were counted and stored in listmode. This data was then analyzed using Cell Quest software (Becton-Dickinson, Waltham, Massachusetts, USA).

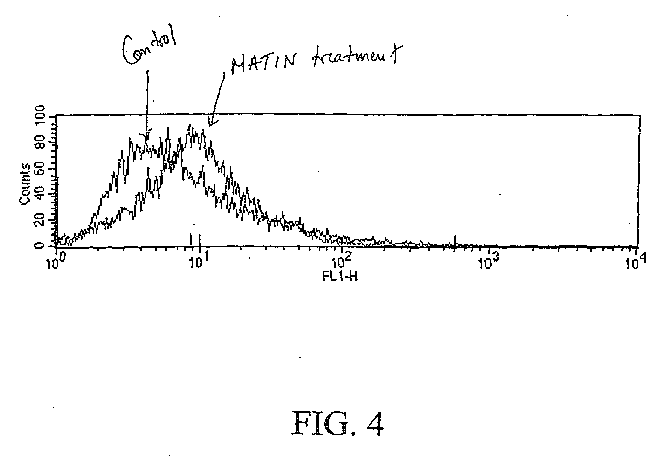

The results are shown in Fig. 4, which is a plot showing annexin fluorescence activity. After 18 hours of treatment with Matin at 20 µg/ml, a distinct shift of peak annexin fluorescence was observed. The shift in fluorescence intensity was similar for Matin at 20 µg/ml and the positive control TNF-α (80 ng/ml). Matin at 2 µg/ml also showed a mild shift in annexin fluorescence intensity, but concentrations below 0.2 µg/ml did not demonstrate any annexin V positivity. This shift of peak intensity was not observed when nonendothelial cells (PC-3) were used.

*Caspase-3 assay.* Caspase-3 (CPP32) is an intracellular protease activated at the early stage of apoptosis, and initiates cellular breakdown by degrading structural and DNA repair proteins (Casciola-Rosen, L. *et al*., 1996, *J. Exp. Med.* 183:1957-1964; Salvesen, G.S. *et al*., 1997, *Cell* 91:443-446). The protease activity of Caspase-3 was measured spectrophotometrically by detection of the chromophore (p-nitroanilide) cleaved from the labeled substrate (DEVD-pNA).

C-PAE cells or PC-3 cells (0.5 x 10⁶ per well) were plated onto a 6-well plate precoated with fibronectin (10 µg/ml) in DMEM supplemented with 10% FCS, and incubated overnight. The next day, the medium was replaced with DMEM containing 2% FCS and then incubated overnight at 37°C. Then cells were then stimulated with bFGF (3ng/ml) in DMEM supplemented with 2% FCS, and also containing either TNF-α (80 ng/ml, positive control) or Matin (10 µg/ml), and incubated for 24 hours. Controls received PBS buffer. After 24 hours, the supernatant cells were collected, and attached cells were trypsinized and combined with the supernatant cells. Cells were counted and resuspended in cell lysis buffer (Clontech, Palo Alto, California, USA) at a concentration of 4 x 10⁷ cells/ml. The rest of the protocol followed the manufacturer's instructions (Clontech, Palo Alto, California, USA). A specific inhibitor of Caspase-3, DEVD-fmk (Asp-Glu-Val-Asp-fluoromethyl ketone) was used to confirm the specificity of the assay. The absorbance was measured in a microplate reader (Bio-Rad, Hercules, California, USA) at 405 nm.

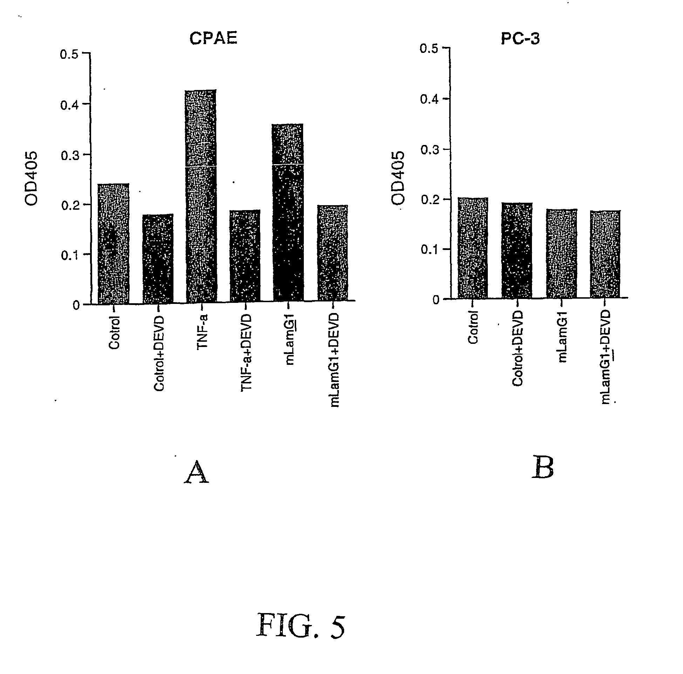

The results are shown in Figs. 5A and 5B, which are a pair of histograms showing the amount of Caspase-3 activity as a function of absorbance at OD₄₀₅ (y-axis) for C-PAE cells (Fig. 5A) and PC-3 cells (Fig. 5B) under various treatments (x-axis).

C-PAE cells treated with20 µg/ml Matin exhibited a 1.6-fold increase in Caspase-3 activity, whereas the positive control TNF-α gave a comparable (1.7-fold) increase compared with control. A specific inhibitor of Caspase-3, DEVD-fink, decreased the protease activity to baseline indicating that the increase in the measured activity was specific for Caspase-3. In nonendothelial PC-3 cells, there was no difference in Caspase-3 activity between control and Matin-treated cells.

*MTT Assay*. The pro-apoptotic activity of Matin was examined in C-PAE cells. Cell viability was assessed by MTT (3-(4, 5-dimethylthiazol-2-yl)-2, 5-diphenyl tetrasolium bromide) assay (Sugiyama, H. *et al*., 1998, *Kidney Int.* 54:1188-1196). This assay is a quantitative colorimetric analysis for cell survival based on the ability of living cells to cleave the tetrasolium ring in active mitochondria. C-PAE cells (7,000 cells per well) were plated to a 96-well plate in 10% FCS containing DMEM. The next day, either TNF-α (positive control, 80 ng/ml), or varying concentrations of Matin were added to the wells and incubated for 24 hours. MTT solution (5 mg/ml; CHEMICON International, Temecula, California, USA) was then added to the wells at a rate of 10 µl/well and incubated at 37°C for 4 hours. Acid-isopropanol was added and mixed thoroughly. The absorbance was measured in a microplate reader (Bio-Rad, Hercules, California, USA) at 590 nm.

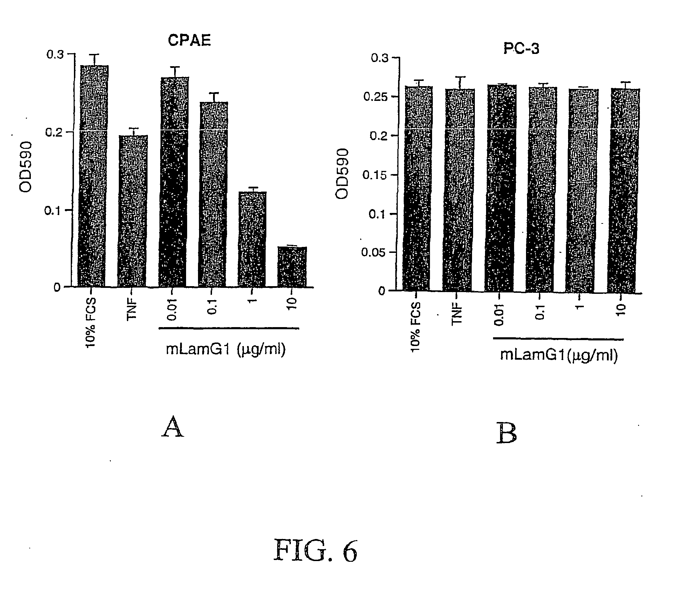

The results are shown in Figs. 6A and 6B, which are a pair of histograms showing cell viability (as a function of OD₅₉₀, y-axis) at increasing concentrations of Matin (x-axis). Each point represents the mean +/- the standard error of the mean for triplicate wells.

Matin decreased cell viability in a dose-dependent manner. At 10 µg/ml, Matin decreased the cell viability by about 80% compared to controls. No inhibitory effect was observed in Matin-treated PC-3 cells.

### Example 4: Matin Inhibits Tumor Growth in vivo.

Five million PC-3 cells were harvested and injected subcutaneously on the back of 7- to 9-week-old male athymic nude mice. The tumors were measured using Vernier calipers and the volume was calculated using the standard formula width² x length x 0.52. The tumors were allowed to grow to about 100 mm³, and animals were then divided into groups of 5 or 6 mice. Matin or nephrin was intraperitoneally injected daily (20 mg/kg) for 10 days in sterile PBS to their respective experimental group. The control group received vehicle injection (either BSA or PBS). Tumor volume was calculated every 2 or 3 days over 10 days. Nephiin is a non-collagen-derived protein which was used as a control. The nephrin was expressed in pET22b, as for Matin.

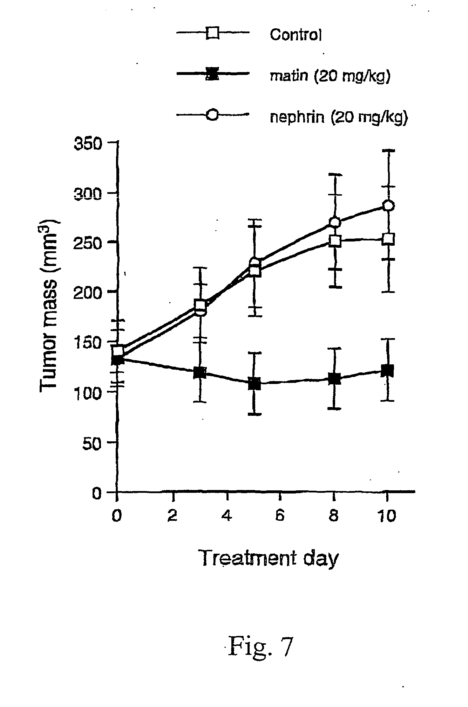

The results are shown in Fig. 7, which is a graph showing tumor size in mm³ (y-axis) against days of treatment (x-axis) for the PBS control (□), 20 mg/kg Matin (■) and 20 mg/kg nephrin (○). Matin, produced in *E. coli*, significantly inhibited the growth of PC-3 human prostate tumors (Fig. 7).

## Claims

1. A protein that consists of the amino acids 2132 to 2338 or of amino acids 2132 to 3084 of SEQ ID No:2, or a fragment thereof comprising at least 25 continuous amino acids of said protein, or a variant of said protein having at least 70% sequence identity therewith; wherein said protein, variant or fragment has anti-angiogenic activity, with the proviso that said variant does not have the amino acid sequence shown in SEQ ID No 113 of WO 00/56754.

2. A protein of Claim 1 that is the G1 domain of the α1 chain of mouse laminin.

3. A variant according to Claim 1 having at least 80% sequence identity with said protein.

4. A variant according to Claim 1 having at least 90% sequence identity with said protein.

5. A protein, fragment, or variant according to any of Claims 1 to 4 that is a monomer.

6. A multimer of a protein, fragment, or variant according to any of Claims 1 to 4.

7. A chimeric protein comprising a protein, fragment or variant thereof according to any of Claims 1 to 4; with the proviso that it is not a fusion protein of glutathione-S-transferase and the G1, G2, G3, G4 or G5 domain of the human laminin α3 chain.

8. A chimeric protein according to Claim 7, further comprising at least one protein molecule selected from the group consisting of: vascostatin or fragments thereof, arrestin or fragments thereof, canstatin or fragments thereof, tumstatin or fragments thereof, endostatin or fragments thereof, angiostatin or fragments thereof, restin or fragments thereof, apomigren or fragments thereof, or other anti-angiogenic proteins or fragments thereof.

9. A pharmaceutical or contraceptive composition comprising a protein, fragment, variant, monomer, multimer or chimeric protein as described in any of Claims 1 to 8.

10. The composition of Claim 9, further comprising a pharmaceutically compatible carrier.

11. The composition of Claim 9 or 10, further comprising at least one protein molecule selected from the group consisting of: vascostatin or fragments thereof, arrestin or fragments thereof, canstatin or fragments thereof, tumstatin or fragments thereof, endostatin or fragments thereof, angiostatin or fragments thereof restin or fragments thereof, apomigren or fragments thereof, or other anti-angiogenic proteins, or fragments thereof.

12. A protein, fragment, variant, monomer, multimer, chimeric protein, or composition as described in any of Claims 1 to 11 for use in medicine.

13. The use of protein, fragment, variant, monomer, multimer, chimeric protein, or composition as described in any of Claims 1 to 11, but ignoring the provisos in Claims 1 and 7, in the preparation of a medicament for treating a disorder involving angiogenesis.

14. The use according to Claim 13, wherein the disorder is cancer.

15. The use according to Claim 13, wherein the disorder involves tumor growth.

16. The use of a protein, fragment, variant, monomer, multimer, chimeric protein or composition according to any of Claims 1 to 11, but ignoring the provisos in Claims 1 and 7, in the preparation of a medicament for treating a disorder involving endothelial cell proliferation.

17. The use of a protein, fragment, variant, monomer, multimer, chimeric protein or composition according to any of Claims 1 to 11, but ignoring the provisos in Claims 1 and 7, in the preparation of a medicament for treating a disorder selected from the group comprising angiogenesis-dependent cancers, benign tumors, rheumatoid arthritis, diabetic retinopathy, fibrosis, psoriasis, ocular angiogenesis diseases, Osler-Webber Syndrome, myocardial angiogenesis, plaque neovascularization, telangiectasia, hemopheliac joints, angiofibroma, wound granulation, intestinal adhesions, atherosclerosis, scleroderma, hypertrophic scars, cat scratch disease, *Helicobacter pylori* ulcers, dialysis graft vascular access stenosis and obesity.

18. The combination of a protein, fragment, variant, monomer, multimer, chimeric protein, on composition according to any of claims 1 to 11 and a radiotherapeutic, chemotherapeutic or immunotherapeutic agent; for simultaneous, separate or sequential use is anti-cancer therapy.

19. A polynucleotide encoding the protein, fragment, variant or chimeric protein according to any of Claims 1 to 8.

20. The polynucleotide of Claim 19, wherein the polynucleotide is operably linked to an expression control sequence.

21. A host cell, e.g. a cell selected from the group comprising bacterial, yeast, mammalian, insect or plant cells, transformed with the polynucleotide of Claim 19 or Claim 20, wherein said host cell is not part of a human embryo.

22. A polynucleotide encoding the protein, fragment, variant or chimeric protein as described in any of Claims 1 to 8, or said polynucleotide operably linked to an expression control sequence, or a host cell according to Claim 21; for use in medicine.

23. A process for producing a protein, fragment, variant, or chimeric protein encoded by the polynucleotide of Claim 19 or Claim 20, wherein the process comprises:
(a) growing a culture of a host cell of Claim 21
and
(b) purifying the protein, fragment, variant, or chimeric protein from the culture.

24. The use of a polynucleotide encoding the protein, fragment, variant or chimeric protein as described in any of Claims 1 to 8, but ignoring the provisos in Claims 1 and 7, or of said polynucleotide operably linked to an expression control sequence, in the preparation of a medicament for treating a disorder as described in any of Claims 13 to 17.

25. The use according to Claim 24, wherein the medicament is a mammalian cell that is not part of a human embryo and the preparation involves treating the cell *in vitro* to insert the polynucleotide therein.

26. The use of Claim 25, wherein the cell allows transient expression.

27. The use of Claim 25 or 26, wherein the cell is chosen from the group consisting of blood cells, TIL cells, bone marrow cells, vascular cells, tumor cells, liver cells, muscle cells and fibroblast cells.

28. The use according to any of Claims 25 to 27, wherein the polynucleotide is inserted into the cell by a viral vector.

29. The use according to Claim 24, wherein the medicament comprises the polynucleotide and a carrier.

30. A protein, fragment, variant, monomer, or chimeric protein according to any of Claims 1 to 8, a polynucleotide according to Claim 19 or 20, or a host cell according to Claim 21; wherein said protein, fragment, variant, monomer, chimeric protein, polynucleotide or host cell is in isolated form.

## Patentansprüche

1. Protein, bestehend aus den Aminosäuren 2132 bis 2338 oder den Aminosäuren 2132 bis 3084 der SEQ ID NO: 2, oder ein Fragment des Proteins, das wenigstens 25 aufeinander folgende Aminosäuren des Proteins umfasst, oder eine Variante des Proteins mit wenigstens 70 % Sequenzidentität damit, wobei das Protein, die Variante oder das Fragment antiangiogene Aktivität aufweist, mit der Maßgabe, daß die Variante nicht die in der SEQ ID NO: 113 der WO 00/56754 gezeigte Aminosäuresequenz aufweist.

2. Protein nach Anspruch 1, bei dem es sich um die G1-Domäne der α1-Kette von Maus-Laminin handelt.

3. Variante gemäß Anspruch 1 mit wenigstens 80 % Sequenzidentität mit dem Protein.

4. Variante gemäß Anspruch 1 mit wenigstens 90 % Sequenzidentität mit dem Protein.

5. Protein, Fragment oder Variante gemäß einem der Ansprüche 1 bis 4, wobei es sich um ein Monomer handelt.

6. Multimer eines Proteins, eines Fragments oder einer Variante gemäß einem der Ansprüche 1 bis 4.

7. Chimäres Protein, das ein Protein, ein Fragment oder eine Variante davon gemäß einem der Ansprüche 1 bis 4 umfasst, mit der Maßgabe, daß es sich dabei nicht um ein Fusionsprotein von Glutathion-S-Transferase und der G1-, G2-, G3-, G4- oder G5-Domäne der α3-Kette von menschlichem Laminin handelt.

8. Chimäres Protein gemäß Anspruch 7, das zusätzlich wenigstens ein Proteinmolekül umfasst, das aus der Gruppe, bestehend aus: Vascostatin oder Fragmenten davon, Arrestin oder Fragmenten davon, Canstatin oder Fragmenten davon, Tumstatin oder Fragmenten davon, Endostatin oder Fragmenten davon, Angiostatin oder Fragmenten davon, Restin oder Fragmenten davon, Apomigren oder Fragmenten davon oder anderen anti-angiogenen Proteinen oder Fragmenten davon, ausgewählt ist.

9. Pharmazeutische oder empfängnisverhütende Zusammensetzung, die ein Protein, ein Fragment, eine Variante, ein monomeres, multimeres oder chimäres Protein mit der in einem der Ansprüche 1 bis 8 angegebenen Bedeutung umfasst.

10. Zusammensetzung nach Anspruch 9, die zusätzlich ein pharmazeutisch verträgliches Trägermittel umfasst.

11. Zusammensetzung nach Anspruch 9 oder 10, die zusätzlich wenigstens ein Proteinmolekül umfasst, das aus der Gruppe, bestehend aus: Vascostatin oder Fragmenten davon, Arrestin oder Fragmenten davon, Canstatin oder Fragmenten davon, Tumstatin oder Fragmenten davon, Endostatin oder Fragmenten davon, Angiostatin oder Fragmenten davon, Restin oder Fragmenten davon, Apomigren oder Fragmenten davon oder anderen anti-angiogenen Proteinen oder Fragmenten davon, ausgewählt ist.

12. Protein, Fragment, Variante, monomeres, multimeres, chimäres Protein oder Zusammensetzung mit der in einem der Ansprüche 1 bis 11 angegebenen Bedeutung zur Verwendung in der Medizin.

13. Verwendung eines Proteins, eines Fragments, einer Variante, eines monomeren, multimeren oder chimären Proteins oder einer Zusammensetzung mit der in einem der Ansprüche 1 bis 11 angegebenen Bedeutung, jedoch unter Nichtbeachten der Maßgaben in Ansprüchen 1 und 7, bei der Herstellung eines Medikaments zur Behandlung einer Erkrankung, die Angiogenese involviert.

14. Verwendung gemäß Anspruch 13, wobei es sich bei der Erkrankung um Krebs handelt.

15. Verwendung gemäß Anspruch 13, wobei die Erkrankung Tumorwachstum involviert.

16. Verwendung eines Proteins, eines Fragments, einer Variante, eines monomeren, multimeren, chimären Proteins oder einer Zusammensetzung gemäß einem der Ansprüche 1 bis 11, jedoch unter Nichtbeachten der Maßgaben in Ansprüchen 1 und 7, bei der Herstellung eines Medikaments zur Behandlung einer Erkrankung, die endotheliale Zellproliferation involviert.

17. Verwendung eines Proteins, eines Fragments, einer Variante, eines monomeren, multimeren, chimären Proteins oder einer Zusammensetzung gemäß einem der Ansprüche 1 bis 11, jedoch unter Nichtbeachten der Maßgaben in Ansprüchen 1 und 7, bei der Herstellung eines Medikaments zur Behandlung einer Erkrankung, die aus der Gruppe, umfassend Angiogenese-abhängige Krebsarten, gutartige Tumoren, rheumatoide Arthritis, diabetische Retinopathie, Fibrose, Psoriasis, okulare Angiogeneseerkrankungen, Morbus Osler, Herzmuskelangiogenese, Plaqueneovaskularisierung, Telangiektasie, Blutergelenke, Angiofibrom, Wundgranulation, Darmverklebungen, Atherosklerose, Skleroderm, hypertrophe Narben, Katzenkratzerkrankung, *Helicobacter pylori*-Geschwüre, vaskuläre Dialysepfropfzugangsstenose und Fettleibigkeit, ausgewählt ist.

18. Kombination aus einem Protein, einem Fragment, einer Variante, einem monomeren, multimeren, chimären Protein oder einer Zusammensetzung gemäß einem der Ansprüche 1 bis 11 und einem radiotherapeutischen, chemotherapeutischen oder immuntherapeutischen Mittel zur gleichzeitigen, getrennten oder aufeinander folgenden Verwendung in einer Anti-Krebstherapie.

19. Polynukleotid, das das Protein, das Fragment, die Variante oder das chimäre Protein gemäß einem der Ansprüche 1 bis 8 kodiert.

20. Polynukleotid nach Anspruch 19, wobei das Polynukleotid operativ mit einer Expressionskontrollsequenz verknüpft ist.

21. Wirtszelle, z. B. eine Zelle, die aus der Gruppe, umfassend Bakterien-, Hefe-, Säugetier-, Insekten- oder Pflanzenzellen ausgewählt ist und die mit dem Polynukleotid nach Anspruch 19 oder 20 transformiert ist, wobei die Wirtszelle nicht Teil eines menschlichen Embryos ist.

22. Polynukleotid, das das Protein, das Fragment, die Variante oder das chimäre Protein mit der in einem der Ansprüche 1 bis 8 angegebenen Bedeutung kodiert, oder das operativ mit einer Expressionskontrollsequenz verknüpfte Polynukleotid oder eine Wirtszelle gemäß Anspruch 21 zur Verwendung in der Medizin.

23. Verfahren zur Herstellung eines Proteins, eines Fragments, einer Variante oder eines monomeren, multimeren oder chimären Proteins, das bzw. die durch das Polynukleotid nach Anspruch 19 oder 20 kodiert wird, wobei das Verfahren das Folgende umfasst:
(a) das Anziehen einer Kultur aus einer Wirtszelle nach Anspruch 21 und
(b) das Aufreinigen des Proteins, des Fragments, der Variante oder des chimären Proteins aus der Kultur.

24. Verwendung eines Polynukleotids, das das Protein, das Fragment, die Variante oder das chimäre Protein mit der in einem der Ansprüche 1 bis 8 angegebenen Bedeutung, jedoch unter Nichtbeachten der Maßgaben in Ansprüchen 1 und 7, kodiert, oder des operativ mit einer Expressionskontrollsequenz verknüpften Polynukleotids bei der Herstellung einer therapeutischen Säugetierzelle zur Behandlung einer wie in einem der Ansprüche 13 bis 17 beschriebenen Erkrankung, wobei die Herstellung die Behandlung einer Säugetierzelle *in vitro* zur Einführung des Polynukleotids darin involviert und wobei die Zelle nicht Teil eines menschlichen Embryos ist.

25. Verwendung nach Anspruch 24, wobei die Zelle die transiente Expression erlaubt.

26. Verwendung nach Anspruch 24 oder 25, wobei die Zelle aus der Gruppe, bestehend aus: Blutzellen, TIL-Zellen, Knochenmarkszellen, vaskulären Zellen, Tumorzellen, Leberzellen, Muskelzellen und Fibroblastenzellen, gewählt wird.

27. Verwendung nach einem der Ansprüche 24 bis 26, wobei das Polynukleotid durch einen viralen Vektor in die Zelle eingeführt wird.

28. Protein, Fragment, Variante, monomeres, multimeres oder chimäres Protein gemäß einem der Ansprüche 1 bis 8, Polynukleotid gemäß Anspruch 19 oder Anspruch 20, Wirtszelle gemäß Anspruch 21, wobei das Protein, das Fragment, die Variante, das monomere, multimere oder chimäre Protein, das Polynukleotid oder die Wirtszelle in isolierter Form vorliegt.

## Revendications

1. Protéine constituée des aminoacides 2132 à 2338 ou des aminoacides 2132 à 3084 de SEQ ID NO:2, ou fragment de celle-ci comprenant au moins 25 aminoacides continus de ladite protéine, ou variant de ladite protéine ayant au moins 70 % d'identité de séquence avec celle-ci ; où ladite protéine, ledit variant ou ledit fragment ont une activité anti-angiogène, à condition que ledit variant n'ait pas la séquence d'aminoacides illustrée par SEQ ID NO:113 dans le document WO 00/56754.

2. Protéine selon la revendication 1 qui est le domaine G1 de la chaîne α1 de la laminine de souris.

3. Variant selon la revendication 1 ayant au moins 80 % d'identité de séquence avec ladite protéine.

4. Variant selon la revendication 1 ayant au moins 90 % d'identité de séquence avec ladite protéine.

5. Protéine, fragment ou variant selon l'une quelconque des revendications 1 à 4 qui est un monomère.

6. Multimère d'une protéine, d'un fragment ou d'un variant selon l'une quelconque des revendications 1 à 4.

7. Protéine chimère comprenant une protéine, un fragment ou un variant de celle-ci selon l'une quelconque des revendications 1 à 4; à condition qu'il ne s'agisse pas d'une protéine de fusion de la glutathione-S-transférase et du domaine G1, G2, G3, G4 ou G5 de la chaîne α3 de la laminine humaine.

8. Protéine chimère selon la revendication 7 comprenant en outre au moins une molécule de protéine choisie dans le groupe consistant en : la vascostatine ou des fragments de celle-ci, l'arrestine ou des fragments de celle-ci, la canstatine ou des fragments de celle-ci, la tumstatine ou des fragments de celle-ci, l'endostatine ou des fragments de celle-ci, l'angiostatine ou des fragments de celle-ci, la restine ou des fragments de celle-ci, l'apomigrène ou des fragments de celle-ci, ou d'autres protéines anti-angiogènes ou des fragments de celles-ci.

9. Composition pharmaceutique ou contraceptive comprenant une protéine, un fragment, un variant, un monomère, un multimère ou une protéine chimère selon l'une quelconque des revendications 1 à 8.

10. Composition selon la revendication 9, comprenant en outre un support pharmaceutiquement compatible.

11. Composition selon la revendication 9 ou 10 comprenant en outre au moins une molécule de protéine choisie dans le groupe consistant en : la vascostatine ou des fragments de celle-ci, l'arrestine ou des fragments de celle-ci, la canstatine ou des fragments de celle-ci, la tumstatine ou des fragments de celle-ci, l'endostatine ou des fragments de celle-ci, l'angiostatine ou des fragments de celle-ci, la restine ou des fragments de celle-ci, l'apomigrène ou des fragments de celle-ci, ou d'autres protéines anti-angiogènes, ou des fragments de celles-ci.

12. Protéine, fragment, variant, monomère, multimère, protéine chimère ou composition selon l'une quelconque des revendications 1 à 11 destinés à être utilisés en médecine.

13. Utilisation d'une protéine, d'un fragment, d'un variant, d'un monomère, d'un multimère, d'une protéine chimère ou d'une composition selon l'une quelconque des revendications 1 à 11, mais en faisant abstraction des conditions mentionnées dans les revendications 1 et 7, dans la préparation d'un médicament pour traiter une affection impliquant l'angiogenèse.

14. Utilisation selon la revendication 13, où l'affection est un cancer.

15. Utilisation selon la revendication 13, où l'affection implique la croissance tumorale.

16. Utilisation d'une protéine, d'un fragment, d'un variant, d'un monomère, d'un multimère, d'une protéine chimère ou d'une composition selon l'une quelconque des revendications 1 à 11, mais en faisant abstraction des conditions mentionnées dans les revendications 1 et 7, dans la préparation d'un médicament pour traiter une affection impliquant la prolifération de cellules endothéliales.

17. Utilisation d'une protéine, d'un fragment, d'un variant, d'un monomère, d'un multimère, d'une protéine chimère ou d'une composition selon l'une quelconque des revendications 1 à 11, mais en faisant abstraction des conditions mentionnées dans les revendications 1 et 7, dans la préparation d'un médicament pour traiter une affection choisie dans le groupe comprenant les cancers dépendants de l'angiogenèse, les tumeurs bénignes, la polyarthrite rhumatoïde, la rétinopathie diabétique, la fibrose, le psoriasis, les maladies d'angiogenèse oculaires, le syndrome de Osler-Webber, l'angiogenèse myocardique, la néovascularisation en plaques, les télangiectasies, l'arthropathie hémophilique, l'angiofibrome, la granulation des plaies, les adhésions intestinales, l'athérosclérose, la sclérodermie, les cicatrices hypertrophiques, la maladie des griffes de chat, les ulcères à *Helicobacter pylori*, la sténose d'accès vasculaire de greffe et de dialyse et l'obésité.

18. Combinaison d'une protéine, d'un fragment, d'un variant, d'un monomère, d'un multimère, d'une protéine chimère ou d'une composition selon l'une quelconque des revendications 1 à 11 et d'un agent radiothérapeutique, chimiothérapeutique ou immunothérapeutique, pour une utilisation simultanée, séparée ou successive dans la thérapie anticancéreuse.

19. Polynucléotide codant pour la protéine, le fragment, le variant ou la protéine chimère selon l'une quelconque des revendications 1 à 8.

20. Polynucléotide selon la revendication 19, où le polynucléotide est lié de manière fonctionnelle à une séquence de contrôle de l'expression.

21. Cellule hôte, par exemple une cellule choisie dans le groupe comprenant les cellules bactériennes, de levure, de mammifères, d'insectes ou de plantes, transformée avec le polynucléotide selon la revendication 19 ou la revendication 20, où ladite cellule hôte ne fait pas partie d'un embryon humain.

22. Polynucléotide codant pour la protéine, le fragment, le variant ou la protéine chimère selon l'une quelconque des revendications 1 à 8, ou ledit polynucléotide lié de manière fonctionnelle à une séquence de contrôle de l'expression ou cellule hôte selon la revendication 21, destinés à être utilisés en médecine.

23. Procédé pour produire une protéine, un fragment, un variant ou une protéine chimère codés par le polynucléotide selon la revendication 19 ou la revendication 20, où le procédé comprend :
(a) la croissance d'une culture d'une cellule hôte selon la revendication 21 ; et
(b) la purification de la protéine, du fragment, du variant ou de la protéine chimère à partir de la culture.

24. Utilisation d'un polynucléotide codant pour la protéine, le fragment, le variant ou la protéine chimère selon l'une quelconque des revendications 1 à 8, mais en faisant abstraction des conditions mentionnées dans les revendications 1 et 7, ou dudit polynucléotide lié de manière fonctionnelle à une séquence de contrôle de l'expression, dans la préparation d'un médicament pour traiter une affection telle que décrite dans l'une quelconque des revendications 13 à 17.

25. Utilisation selon la revendication 24, où le médicament est une cellule de mammifère qui ne fait pas partie d'un embryon humain et la préparation comprend le traitement de la cellule *in vitro* pour y insérer le polynucléotide.

26. Utilisation selon la revendication 25, où la cellule permet une expression transitoire.

27. Utilisation selon la revendication 25 ou 26, où la cellule est choisie dans le groupe consistant en: les cellules sanguines, les cellules TIL, les cellules de moelle osseuse, les cellules vasculaires, les cellules tumorales, les cellules hépatiques, les cellules musculaires et les cellules de fibroblastes.

28. Utilisation selon l'une quelconque des revendications 25 à 27, où le polynucléotide est inséré dans la cellule par un vecteur viral.

29. Utilisation selon la revendication 24, où le médicament comprend le polynucléotide et un support.

30. Protéine, fragment, variant, monomère ou protéine chimère selon l'une quelconque des revendications 1 à 8, polynucléotide selon la revendication 19 ou 20, ou cellule hôte selon la revendication 21, où ladite protéine, ledit fragment, ledit variant, ledit monomère, ladite protéine chimère, ledit polynucléotide ou ladite cellule hôte sont sous forme isolée.
